# EUROPEAN PATENT SPECIFICATION

(11) **EP 0 951 295 B1**
(45) Date of publication and mention of the grant of the patent: **09.06.2010**
(21) Application number: 97928698.6
(22) Date of filing: 30.05.1997
(51) Int. Cl.: A61K 38/48, A61K 39/00, A61P 7/00, A61P 35/00, A61K 31/12

(54) **COMPOSITIONS FOR USE IN INHIBITING OF ALPHA-V-BETA3 MEDIATED ANGIOGENESIS**
ZUSAMMENSTELLUNGEN ZUR VERWENDUNG ALS ALPHA-V-BETA3 VERMITTELTER ANGIOGENESIS HEMMERS
COMPOSITIONS POUR L'UTILISATION AFIN D'INHIBER L'ANGIOGENESE MEDIEE PAR ALPHA-V- BETA3

(30) Priority: 31.05.1996 US 18733 P; 31.05.1996 US 15869 P
(43) Date of publication of application: 27.10.1999
(73) Proprietor: THE SCRIPPS RESEARCH INSTITUTE, La Jolla, CA 92037 (US)
(72) Inventor: BROOKS, Peter, San Diego, CA 92122 (US); CHERESH, David, A., Cardiff, CA 92007 (US)
(74) Representative: Fisher, Adrian John
(86) International application number: PCT/US1997/009158
(87) International publication number: WO 1997/045137

(56) References cited:
- EP-A- 0 770 622
- WO-A-95/28426
- WO-A-97/06791
- WO-A-97/14716
- H P HAMMES ET AL.: "Subcutaneous injection of a cyclic peptide antagonist of vitronectin receptor-type integrins inhibits retinal neovascularization" NATURE MEDICINE., vol. 2, no. 5, May 1996 (1996-05), pages 529-533, XP002138522 CO US
- P C BROOKS ET AL.: "Integrin alphaV beta3 antagonists promote tumor regression by inducing apoptosis of angiogenic blood vessels" CELL, vol. 79, 30 December 1994 (1994-12-30), pages 1157-1164, XP002138523 NA US
- M FRIEDLANDER ET AL.: "Definition of two antagonists pathways by distinct alphaV integrins" SCIENCE., vol. 270, 1 December 1995 (1995-12-01), pages 1500-1502, XP000867841 AAAS. LANCASTER, PA., US
- P C BROOKS ET AL.: "Requirement of vascular integrin alphaV beta3 for angiogenesis" SCIENCE., vol. 264, 22 April 1994 (1994-04-22), pages 569-571, XP002138524 AAAS. LANCASTER, PA., US
- CHEMICAL ABSTRACTS, vol. 125, no. 1, 1 July 1996 (1996-07-01) Columbus, Ohio, US; abstract no. 7248, R A CLARK ET AL.: "Transient functional expression of alpha.v.beta.3 on vascular cells during wound repair" XP002138525 & AM. J. PATHOL., vol. 148, no. 5, May 1996 (1996-05), pages 1407-1421,
- EUR. J. CANCER, 1996, Vol. 32A, No. 1, TIMAR et al., "The Antimetabolite Tiazofurin(TR) Inhibits Glycoconjugate Biosynthesis and Invasiveness of Tumor Cells", pages 152-159.
- CIRCULATION, November 1994, Vol. 90, No. 5, MATSUNO et al., "Inhibition of Integrin Function by a Cyclic RGD-Containing Peptide Prevents Neointima Formation", pages 2203-2206.
- HOOD JD ET AL.: "Differential alpha-v integrin-mediated Ras-ERK signaling during two pathways of angiogenesis" JOURNAL OF CELL BIOLOGY, vol. 162, no. 5, 1 September 2003 (2003-09-01), pages 933-943,

## Description

### Technical Field

The present invention relates generally to the field of medicine, and relates specifically to compositions for use in inhibiting angiogenesis of tissues using antagonists of the vitronectin receptor αᵥβ₃.

### Background

Integrins are a class of cellular receptors known to bind extracellular matrix proteins, and therefore mediate cell-cell and cell-extracellular matrix interactions, referred generally to as cell adhesion events. However, although many integrins and the ligands that bind an integrin are described in the literature, the biological function of many of the integrins remains elusive. The integrin receptors constitute a family of proteins with shared structural characteristics of noncovalent heterodimeric glycoprotein complexes formed of α and β subunits.

The vitronectin receptor, named for its original characteristic of preferential binding to vitronectin, is now known to refer to three different integrins, designated αᵥβ₁, αᵥβ₃ and αᵥβ₅. Horton, Int. J. Exp. Pathol., 71:741-759 (1990). αᵥβ₁ binds fibronectin and vitronectin. αᵥβ₃ binds a large variety of ligands, including fibrin, fibrinogen, laminin, thrombospondin, vitronectin, von Willebrand's factor, osteospontin and bone sialoprotein I. αᵥβ₅ binds vitronectin. The specific cell adhesion roles these three integrins play in the many cellular interactions in tissues are still under investigation, but it is clear that there are different integrins with different biological functions.

One important recognition site in the ligand for many integrins is the arginine-glycine-aspartic acid (RGD) tripeptide sequence. RGD is found in all of the ligands identified above for the vitronectin receptor integrins. This RGD recognition site can be mimicked by polypeptides ("peptides") that contain the RGD sequence, and such RGD peptides are known inhibitors of integrin function. It is important to note, however, that depending upon the sequence and structure of the RGD peptide, the specificity of the inhibition can be altered to target specific integrins.

For discussions of the RGD recognition site, see Pierschbacher et al., Nature, 309:30-33 (1984), and Pierschbacher et al., Proc. Natl. Acad. Sci., USA, 81:5985-5988 (1984). Various RGD polypeptides of varying integrin specificity have also been described by Grant et al., Cell, 58:933-943 (1989), Cheresh et al., Cell, 58:945-953 (1989), Aumailley et al., FEBS Letts., 291:50-54 (1991), and Pfaff et al., J. Biol. Chem., 269:20233-20238 (1994), and in United States Patent Nos. 4,517,686, 4,578,079, 4,589,881, 4,614,517, 4,661,111, 4,792,525, 4,683,291, 4,879,237, 4,988,621, 5,041,380 and 5,061,693.

Angiogenesis is a process of tissue vascularization that involves the growth of new developing blood vessels into a tissue, and is also referred to as neo-vascularization. The process is mediated by the infiltration of endothelial cells and smooth muscle cells. The process is believed to proceed in any one of three ways: the vessels can sprout from pre-existing vessels, de-novo development of vessels can arise from precursor cells (vasculogenesis), or existing small vessels can enlarge in diameter. Blood et al., Bioch. Biophys. Acta, 1032:89-118 (1990). Vascular endothelial cells are known to contain at least five RGD-dependent integrins, including the vitronectin receptor (αᵥβ₃ or αᵥβ₅), the collagen Types I and IV receptor (α₁β₁), the laminin receptor (α₂β₁), the fibronectin/laminin/collagen receptor (α₃β₁) and the fibronectin receptor (α₅β₁). Davis et al., J. Cell. Biochem., 51:206-218 (1993). The smooth muscle cell is known to contain at least six RGD-dependent integrins, including α₅β₁, αᵥβ₃ and αᵥβ₅.

Angiogenesis is an important process in neonatal growth, but is also important in wound healing and in the pathogenesis of a large variety of clinical diseases including tissue inflammation, arthritis, tumor growth, diabetic retinopathy, macular degeneration by neovascularization of retina and the like conditions. These clinical manifestations associated with angiogenesis are referred to as angiogenic diseases. Folkman et al., Science, 235:442-447 (1987). Angiogenesis is generally absent in adult or mature tissues, although it does occur in wound healing and in the corpeus leuteum growth cycle. See, for example, Moses et al., Science, 248:1408-1410 (1990).

It has been proposed that inhibition of angiogenesis would be a useful therapy for restricting tumor growth. Inhibition of angiogenesis has been proposed by (1) inhibition of release of "angiogenic molecules" such as bFGF (basic fibroblast growth factor), (2) neutralization of angiogenic molecules, such as by use of anti-βbFGF antibodies, and (3) inhibition of endothelial cell response to angiogenic stimuli. This latter strategy has received attention, and Folkman et al., Cancer Biology, 3:89-96 (1992), have described several endothelial cell response inhibitors, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like that might be used to inhibit angiogenesis. For additional proposed inhibitors of angiogenesis, see Blood et al., Bioch. Biophys. Acta., 1032:89-118 (1990), Moses et al., Science, 248:1408-1410 (1990), Ingber et al., Lab. Invest., 59:44-51 (1988), and United States Patent Nos. 5,092,885, 5,112,946, 5,192,744, and 5,202,352. None of the inhibitors of angiogenesis described in the foregoing references are targeted at inhibition of αᵥβ₃.

RGD-containing peptides that inhibit vitronectin receptor αᵥβ₃ have also been described. Aumailley et al., FEBS Letts., 291:50-54 (1991), Choi et al., J. Vasc. Surg., 19:125-134 (1994), Smith et al., J. Biol. Chem., 265:12267-12271 (1990), and Pfaff et al., J. Biol. Chem., 269:20233-20238 (1994). However, the role of the integrin αᵥβ₃ in angiogenesis has never been suggested nor identified until the present invention.

For example, Hammes et al., Nature Med., 2:529-53 (1996) confirmed the findings of the present invention. Specifically, the paper shows that cyclic peptides including cyclic RGDfV, the structure and function of the latter of which has been previously described in the priority applications on which the present application is based, inhibited retinal neovascularization in a mouse model of hypoxia-induced retinal neovascularization. In a separate study that also supports the present invention as well as the priority applications, Luna et al., Lab. Invest., 75:563-573 (1996) described two particular cyclic methylated RGD-containing peptides that were partially effective at inhibiting retinal neovascularization in the mouse model of oxygen-induced ischemic retinopathy. In contrast, the peptides of the present invention exhibit almost complete inhibition of neovascularization in the model systems described herein.

Inhibition of cell adhesion in vitro using monoclonal antibodies immunospecific for various integrin α or β subunits have implicated αᵥβ₃ in cell adhesion of a variety of cell types including microvascular endothelial cells. Davis et al., J. Cell. Biol., 51:206-218 (1993). In addition, Nicosia et al., Am. J. Pathol., 138:829-833 (1991), described the use of the RGD peptide GRGDS to in vitro inhibit the formation of "microvessels" from rat aorta cultured in collagen gel. However, the inhibition of formation of "microvessels" in vitro in collagen gel cultures is not a model for inhibition of angiogenesis in a tissue because it is not shown that the microvessel structures are the same as capillary sprouts or that the formation of the microvessel in collagen gel culture is the same as neovascular growth into an intact tissue, such as arthritic tissue, tumor tissue or disease tissue where inhibition of angiogenesis is desirable.

For angiogenesis to occur, endothelial cells must first degrade and cross the blood vessel basement membrane in a similar manner used by tumor cells during invasion and metastasis formation.

The inventors have previously reported that angiogenesis depends on the interaction between vascular integrins and extracellular matrix proteins. Brooks et al., Science, 264:569-571 (1994). Furthermore, it was reported that programmed cell death (apoptosis) of angiogenic vascular cells is initiated by the interaction, which would be inhibitied by certain antagonists of the vascular integrin αᵥβ₃. Brooks et al., Cell, 79:1157-1164 (1994). More recently, the inventors have reported that the binding of matrix metalloproteinase-2 (MMP-2) to vitronectin receptor (αᵥβ₅) can be inhibited using αᵥβ₅ antagonists, and thereby inhibit the enzymatic function of the proteinase. Brooks et al., Cell, 85:683-693 (1996).

Other than the studies reported here, Applicants are unaware of any other demonstration that angiogenesis could be inhibited in a tissue using inhibitors of cell adhesion. In particular, it has never been previously demonstrated by others that αᵥβ₃ function is required for angiogenesis in a tissue or that αᵥβ₃ antagonists can inhibit angiogenesis in a tissue.

### Brief Description of the Invention

The present invention disclosure demonstrates that angiogenesis in tissues requires integrin αᵥβ₃, and that inhibitors of αᵥβ₃ can inhibit angiogenesis. The disclosure also demonstrates that antagonists of other integrins, such as α_{IIb}β₃, or αᵥβ₁, do not inhibit angiogenesis, presumably because these other integrins are not essential for angiogenesis to occur.

The invention therefore describes compounds for use in inhibiting αᵥβ₃ mediated angiogenesis in a tissue.

The tissue to be treated can be any tissue in which inhibition of angiogenesis is desirable, such as diseased tissue where neo-vascularization is occurring. Exemplary tissues include inflamed tissue, solid tumors, metastases, tissues undergoing restenosis, and the like tissues.

An αᵥβ₃ antagonist for use in the present invention is capable of binding to αᵥβ₃ and competitively inhibiting the ability of αᵥβ₃ to bind to a natural ligand. The αᵥβ₃ antagonist is an organic mimetic of αᵥβ₃ as defined in claim 1.

### Brief Description of the Drawings

In the drawings forming a portion of this disclosure:

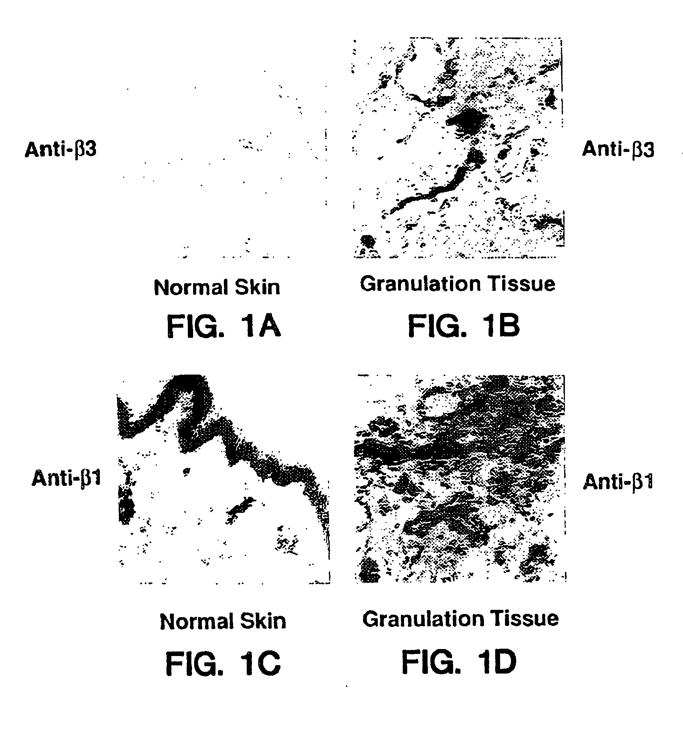

Figures 1A-1D illustrate the tissue distribution of the integrin subunits, β₃ and β₁, in normal skin and in skin undergoing wound healing designated as granulation tissue. Immunohistochemistry with antibodies to β₃ and β₁ was performed as described in Example 3A. Figures 1A and 1B respectively illustrate the immunoreactivity of anti-β₃ in normal skin and granulation tissue. Figures 1C and 1D respectively illustrate the immunoreactivity of anti-β₁ in normal skin and granulation tissue.

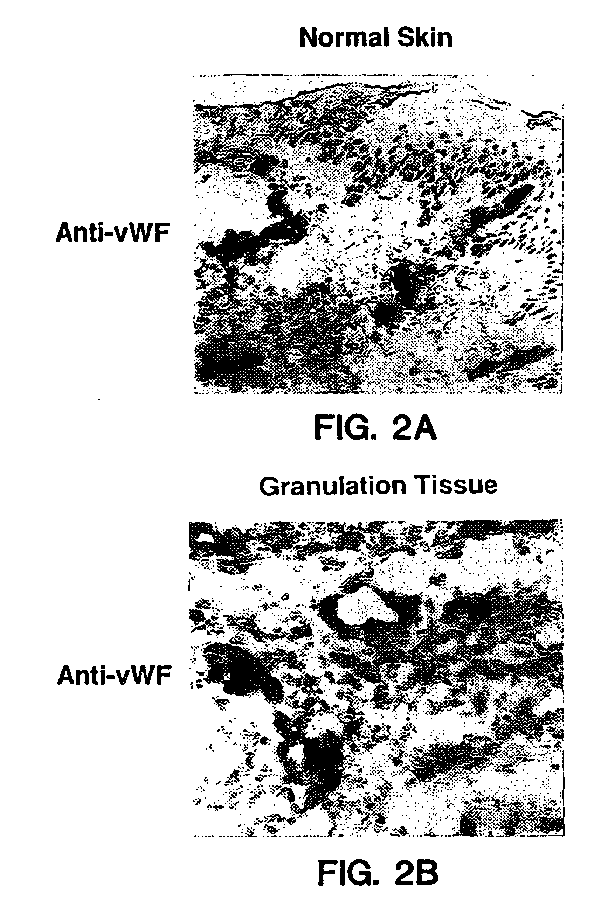

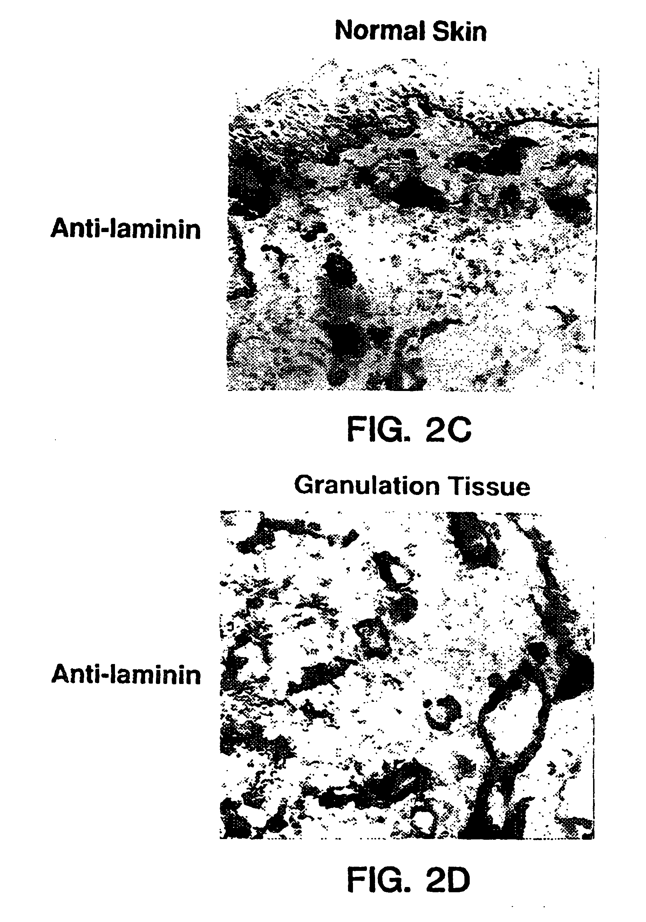

Figures 2A-2D illustrate the tissue distribution of the von Willebrand factor and laminin ligands that respectively bind the β₃ and β₁ integrin subunits in normal skin and in skin undergoing wound healing designated as granulation tissue. Immunohistochemistry with antibodies to von Willebrand factor (anti-vWF) and laminin (anti-laminin) was performed as described in Example 3B. Figures 2A and 2B respectively illustrate the immunoreactivity of anti-vWF in normal skin and granulation tissue. Figures 2C and 2D respectively illustrate the immunoreactivity of anti-laminin in normal skin and granulation tissue.

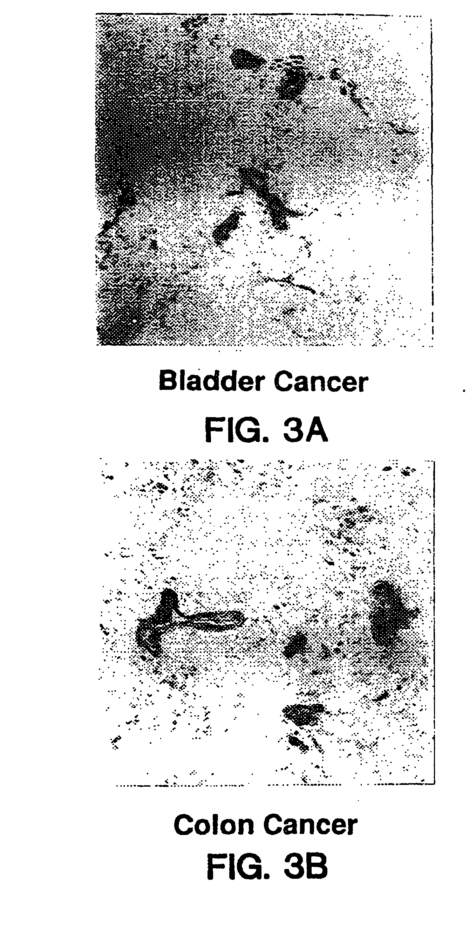

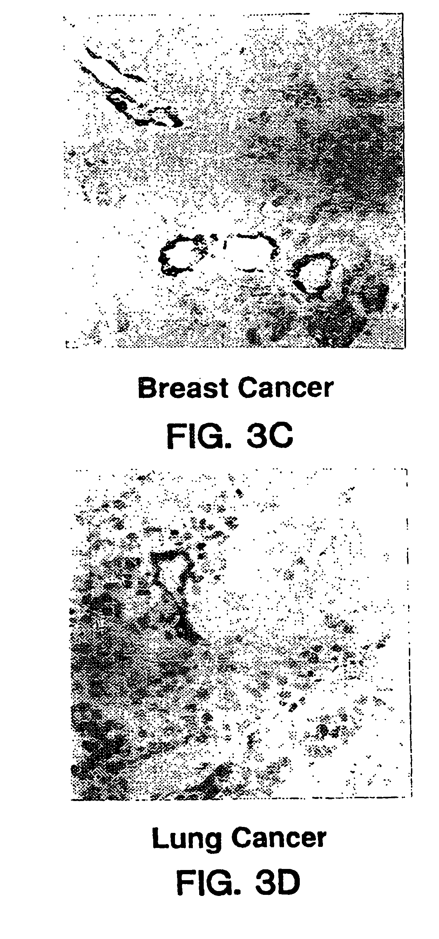

Figures 3A-3D illustrate the tissue distribution of the vitronectin integrin receptor, αᵥβ₃, in tissue biopsies of bladder cancer, colon cancer, breast cancer and lung cancer, respectively. Immunohistochemistry with the LM609 antibody against αᵥβ₃ was performed as described in Example 3C.

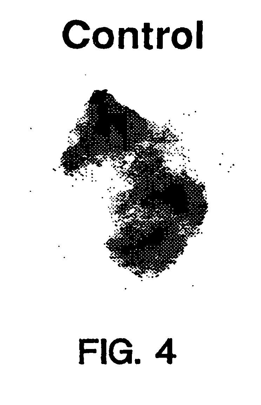

Figure 4 illustrates a typical photomicrograph of a CAM of this invention devoid of blood vessels in an untreated 10 day old chick embryo. The preparation is described in Example 5B.

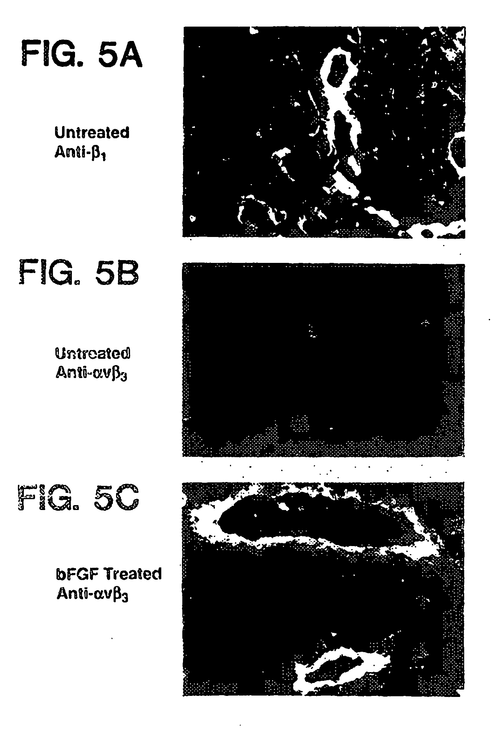

Figures 5A-5C illustrate the tissue distribution of the integrins β₁ and αᵥβ₃ in the CAM preparation of this invention. Figure 5A shows the distribution of the β₁ subunit in an untreated 10 day old CAM preparation as detected by immunofluorescence immunoreactivity with CSAT, an anti-β₁ antibody. Figure 5B shows the distribution of the αᵥβ₃ receptor in an untreated 10 day old CAM preparation as detected by immunofluorescence immunoreactivity with LM609, an anti-αᵥβ₃ antibody. Figure 5C shows the distribution of the αᵥβ₃ receptor in an bFGF treated 10 day old CAM preparation as detected by immunofluorescence immunoreactivity with LM609, an anti-αᵥβ₃ antibody. The treatments and results are described in Example 5C.

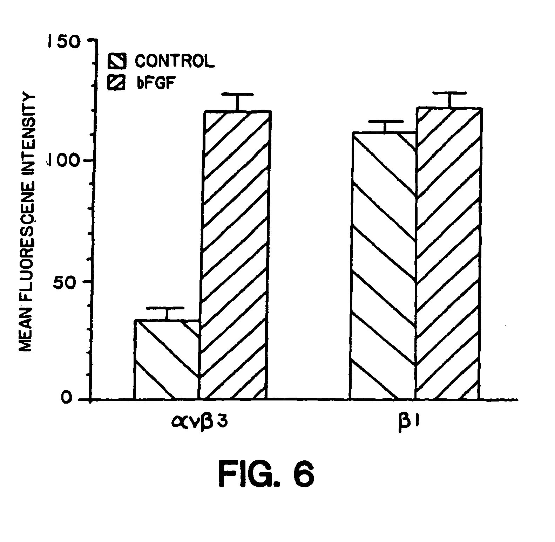

Figure 6 illustrates the quantification in a bar graph of the relative expression of αᵥβ₃ and β₁ in untreated and bFGF treated 10 day old CAMs as described in Example 6A. The mean fluorescence intensity is plotted on the Y-axis with the integrin profiles plotted on the X-axis.

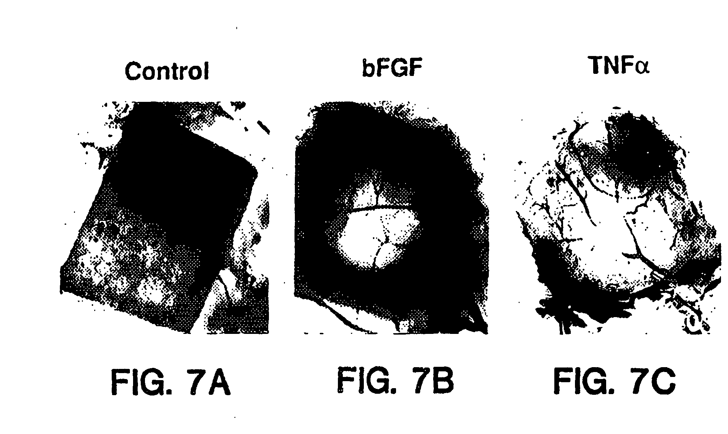

Figures 7A-7C illustrates the appearance of an untreated 10 day old CAM, a bFGF treated CAM, and a TNFα treated CAM, respectively, the procedures and results of which are described in Example 6A.

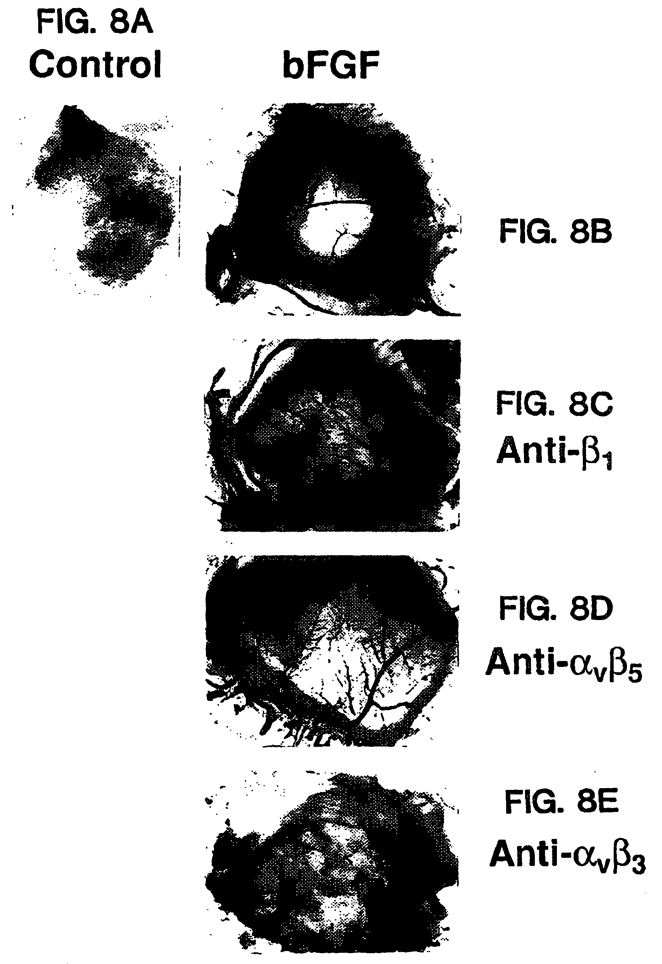

Figures 8A-8E illustrate the effect of topical antibody treatment on bFGF-induced angiogenesis in a day 10 CAM as described in Example 7A1). Figure 8A shows an untreated CAM preparation that is devoid of blood vessels. Figure 8B shows the infiltration of new vasculature into an area previously devoid of vasculature induced by bFGF treatment. Figures 8C, 8D and 8E respectively show the effects of antibodies against β₁ (anti-β₁; CSAT), αᵥβ₅ (anti-αᵥβ₅; P3G2) and αᵥβ₃ (anti-αᵥβ₃; LM609).

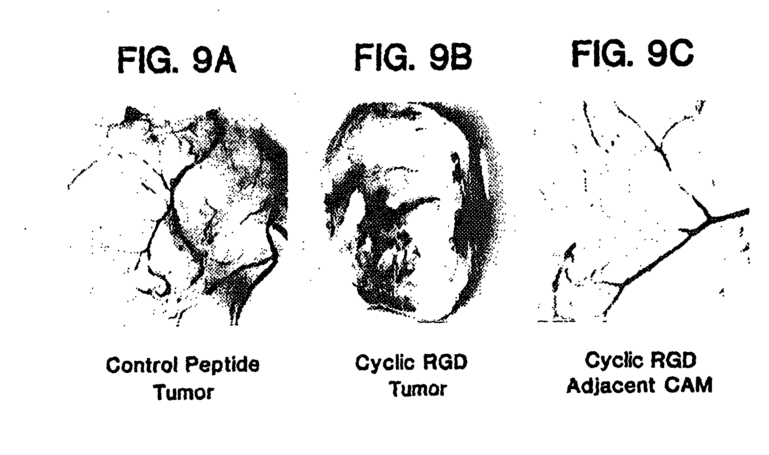

Figures 9A-9C illustrate the effect of intravenous injection of synthetic peptide 66203 on angiogenesis induced by tumors as described in Example 7E2). Figure 9A shows the lack of inhibitory effect of intravenous treatment with a control peptide (control peptide tumor) on angiogenesis resulting from tumor induction. The inhibition of such angiogenesis by intravenous injection of peptide 66203 (cyclic RGD tumor) is shown in Figure 9B. The lack of inhibitory effects or cytotoxicity on mature preexisting vessels following intravenous infusion of peptide 66203 in an area adjacent to the tumor-treated area is shown in Figure 9C (cyclic RGD adjacent CAM).

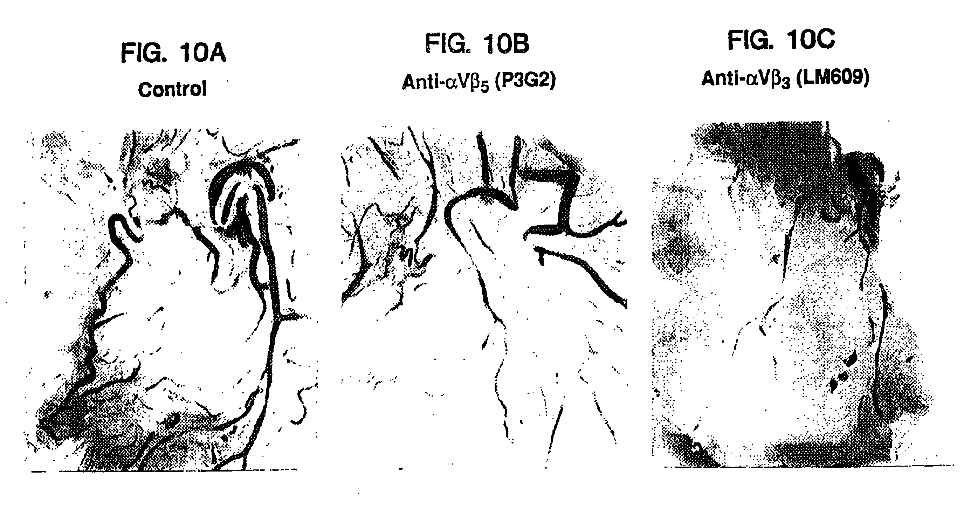

Figures 10A-10C illustrate the effect of intravenous application of monoclonal antibodies to growth factor induced angiogenesis as described in Example 7B1). Figure 10A shows bFGF-induced angiogenesis not exposed to antibody treatment (control). No inhibition of angiogenesis resulted when a similar preparation was treated with anti-αᵥβ₅ antibody P3G2 as shown in Figure 10B. Inhibition of angiogenesis resulted with treatment of anti-αᵥβ₃ antibody LM609 as shown in Figure 10C.

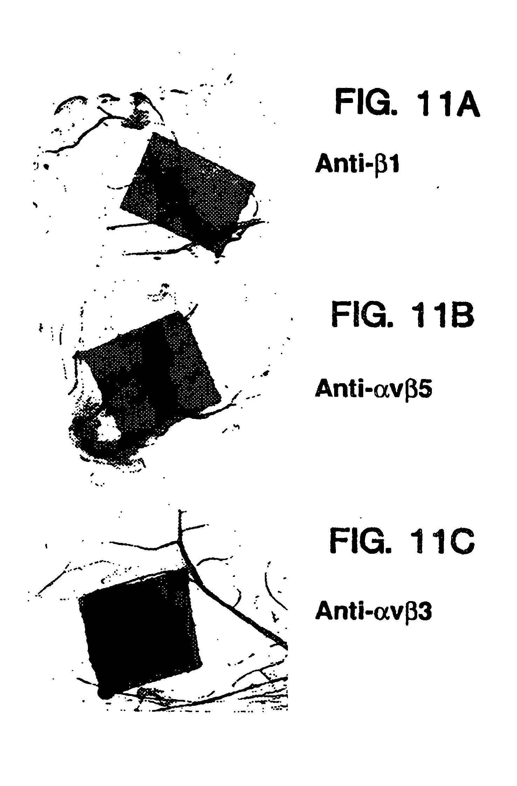

Figures 11A-11C illustrate the effect on embryonic angiogenesis following topical application of anti-integrin antibodies as described in Example 7C. Angiogenesis was not inhibited by treatment of a 6 day CAM with anti-β₁ and anti-αᵥβ₅ antibodies respectively shown in Figures 11A and 11B. In contrast, treatment with the anti-αᵥβ₃ antibody LM609 resulted in the inhibition of blood vessel formation as shown in Figure 11C.

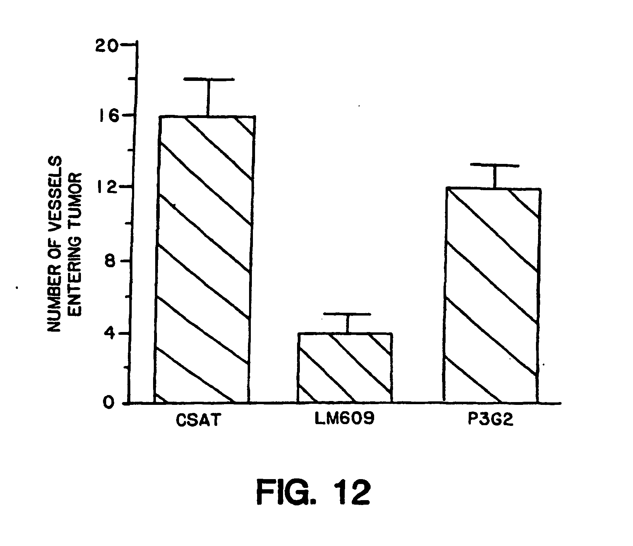

Figure 12 illustrates the quantification of the number of vessels entering a tumor in a CAM preparation as described in Example 7D1). The graph shows the number of vessels as plotted on the Y-axis resulting from topical application of either CSAT (anti-β₁), LM609 (anti-αᵥβ₃) or P3G2 (anti-αᵥβ₅).

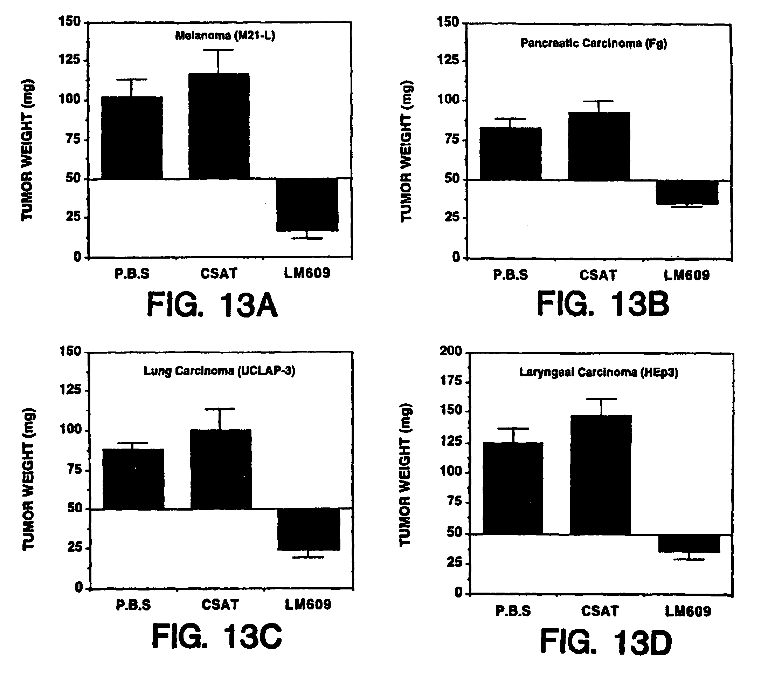

Figures 13A-13D illustrate a comparison between wet tumor weights 7 days following treatment and initial tumor weights as described in Example 9A1)a. Each bar represents the mean ± S.E. of 5-10 tumors per group. Tumors were derived from human melanoma (M21-L) (Figure 13A), pancreatic carcinoma (Fg) (Figure 13B), lung carcinoma (UCLAP-3) (Figure 13C), and laryngeal carcinoma (HEp3) (Figure 13D) CAM preparations and treated intravenously with PBS, CSAT (anti-β₁), or LM609 (anti-αᵥβ₃). The graphs show the tumor weight as plotted on the Y-axis resulting from intravenous application of either CSAT (anti-β₁), LM609 (anti-αᵥβ₃) or PBS as indicated on the X-axis.

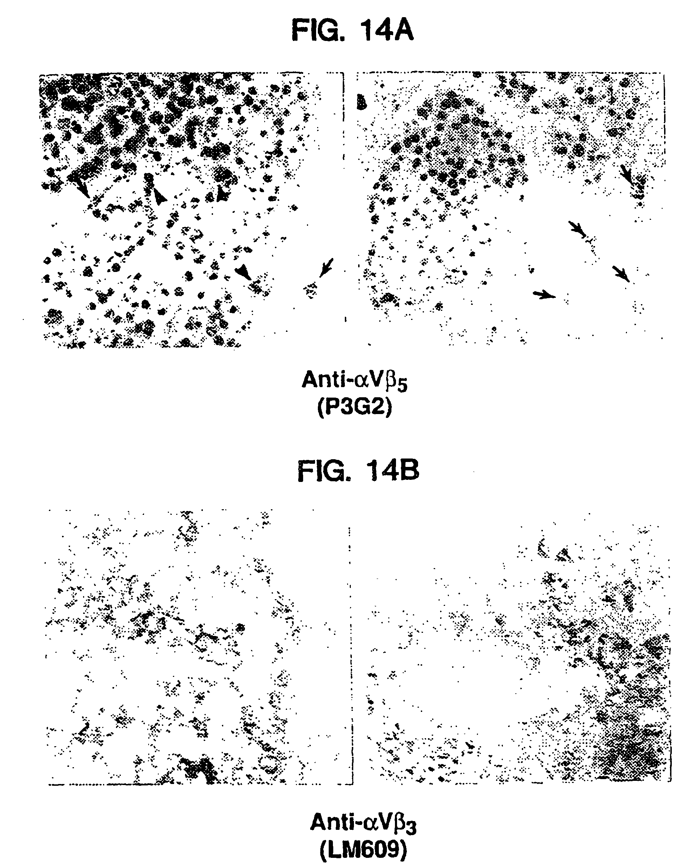

Figures 14A and 14B illustrate histological sections of tumors treated with the P3G2 (anti-αᵥβ₅) (Figure 14A) and LM609 (anti-αᵥβ₃) (Figure 14B), and stained with hematoxylin and eosin as described in Example 9Al)b. As shown in Figure 14A, tumors treated with control antibody (P3G2) showed numerous viable and actively dividing tumor cells as indicated by mitotic figures (arrowheads) as well as by multiple blood vessels (arrows) throughout the tumor stroma. In contrast, few if any viable tumor cells or blood vessels were detected in tumors treated with LM609 (anti-αᵥβ₃) in Figure 14B.

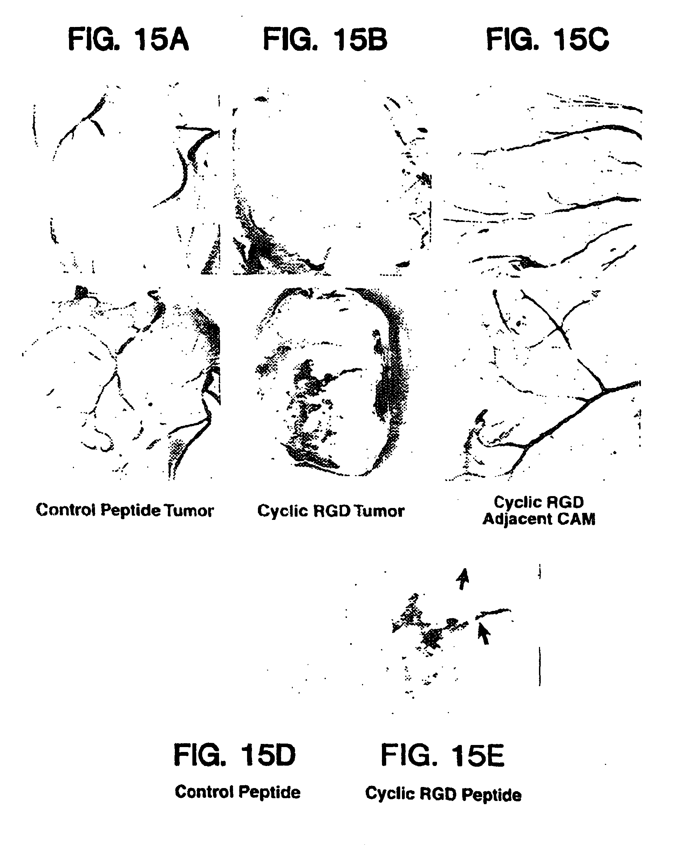

Figures 15A-15E correspond to M21L tumors treated with peptides as described in Example 9A2) and are as follows: Figure 15A, control cyclic RAD peptide (69601); Figure 15B, cyclic RGD peptide (66203); Figure 15C, adjacent CAM tissue taken from the same embryos treated with cyclic RGD peptide (66203) and high magnification (13x) of tumors treated with the control RAD (69601) in Figure 15D or cyclic RGD peptide (66203) in Figure 15E. Figure 15D depicts normal vessels from the RAD control peptide (69601) treated tumor. Figure 15E depicts examples of disrupted blood vessels from cyclic RGD peptide (66203) treated tumors (arrows).

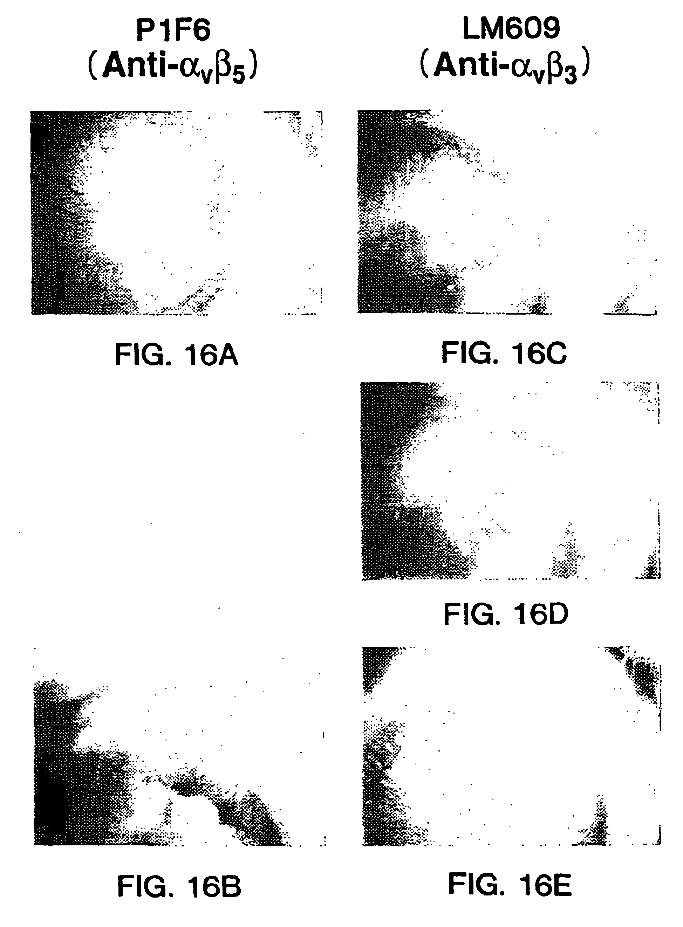

Figures 16A-16E represent inhibition of angiogenesis by antagonists of angiogenesis in the in vivo rabbit eye model assay as described in Example 10. Figure 16A and 16B depict angiogenesis of the rabbit eye in the presence of bFGF and mAb P1F6 (anti-αᵥβ₅). Figure 16C, 16D and 16E depict inhibition of angiogenesis of the rabbit eye in the presence of bFGF and mAb LM609 (anti-αᵥβ₃).

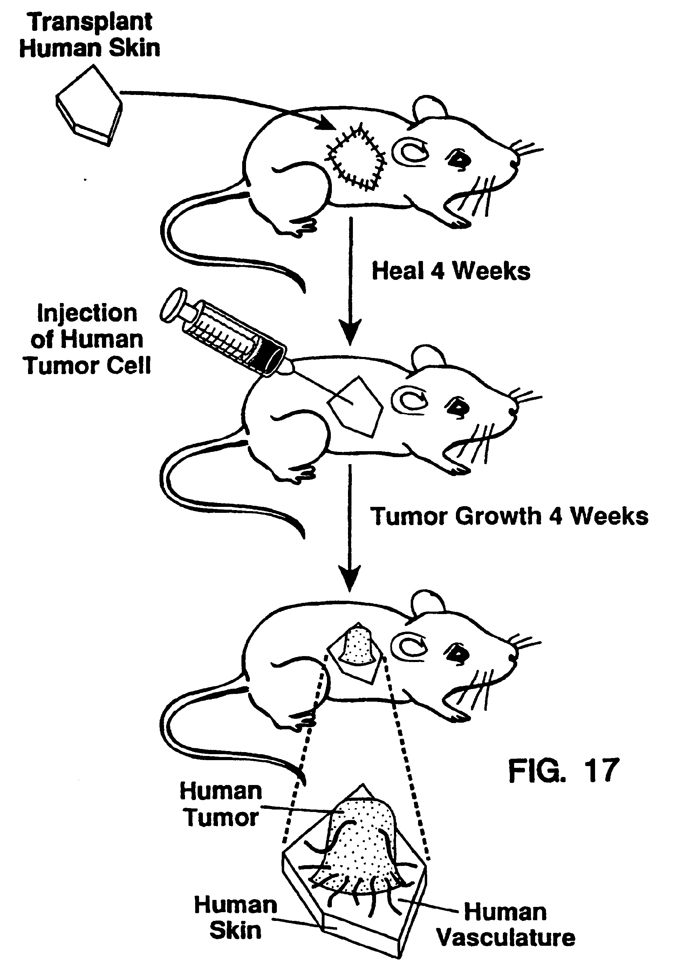

Figure 17 represents a flow chart of how the in vivo mouse:human chimeric mouse model was generated as described in Example 11. A portion of skin from a SCID mouse was replaced with human neonatal foreskin and allowed to heal for 4 weeks. After the graft had healed, the human foreskin was inoculated with human tumor cells. During the following 4 week period, a measurable tumor was established which comprised a human tumor with human vasculature growing from the human skin into the human tumor.

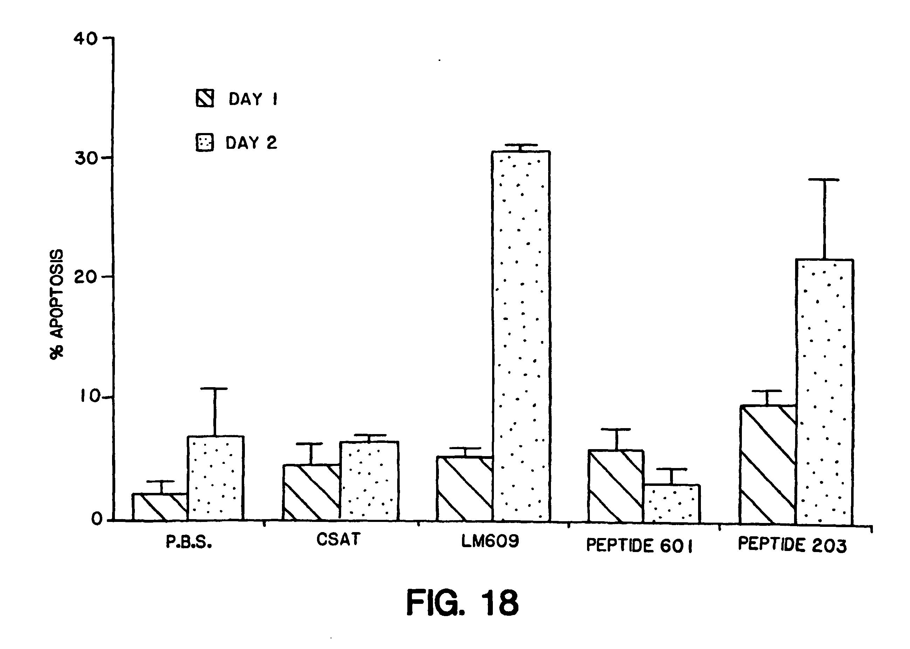

Figure 18 illustrates the percent of single cells derived from mAb-treated and peptide-treated CAMs and stained with Apop Tag as determined by FACS analysis and described in Example 12. The black and stippled bars represent cells from embryos treated 24 hours and 48 hours prior to the assay, respectively. Each bar represents the mean ± S.E. of three replicates. CAMs were treated mAb LM609 (anti-αᵥβ₃), or CSAT (anti-β₁), or PBS. CAMs were also treated with cyclic peptide 66203 (cyclo-RGDfV, indicated as Peptide 203) or control cyclic peptide 69601 (cyclo-RADfV, indicated as Peptide 601).

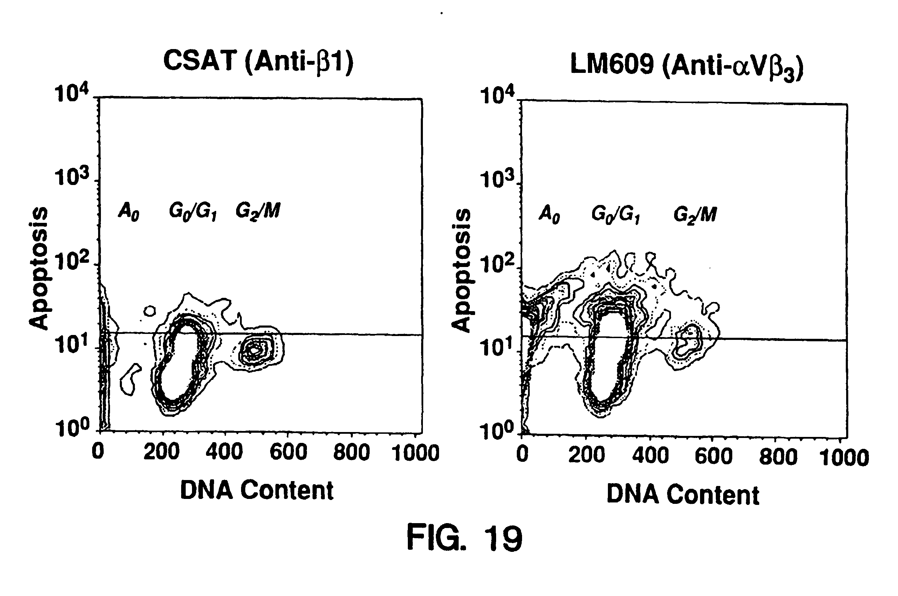

Figures 19A and 19B illustrate the combined results of single cell suspensions of CAMs from embryos treated with either CSAT (anti-β₁) (Figure 19A) or LM609 (ant-αᵥβ₃) (Figure 19B), stained with Apop Tag and propidium iodide, and analyzed by FACS as described in Example 12C. The Y axis represents Apop Tag staining in numbers of cells (Apoptosis), the X axis represents propidium iodide staining (DNA content). The horizontal line represents the negative gate for Apop Tag staining. The left and right panels indicates CAM cells from CSAT (anti-β₁) (Figure 19A) and LM609 (anti-αᵥβ₃) (Figure 19B) treated embryos, respectively. Cell cycle analysis was performed by analysis of approximately 8,000 events per condition.

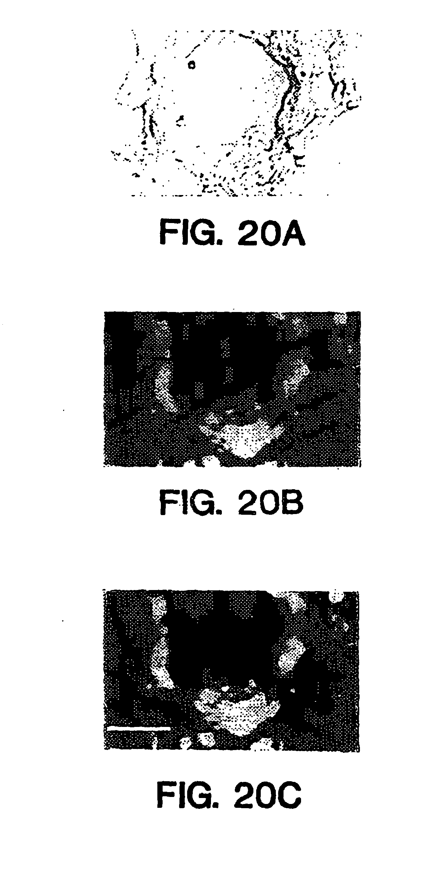

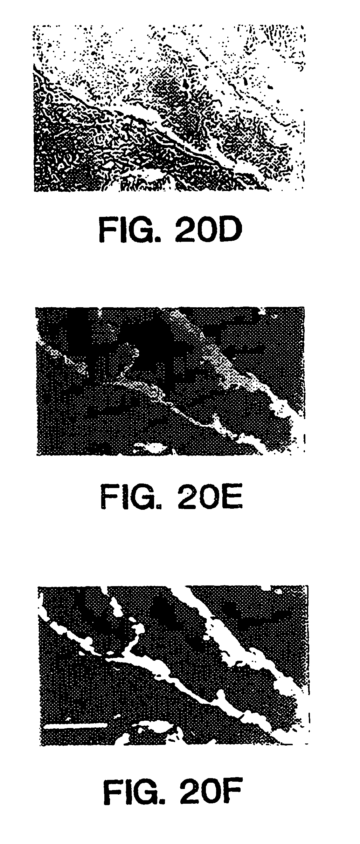

Figures 20A-20C represent CAM tissue from CSAT (anti-β₁) treated embryos and Figures 20D-20F represent CAM tissue from LM609 (anti-αᵥβ₃) treated embryos prepared as described in Example 12C. Figures 20A and 20D depict tissues stained with Apop Tag and visualized by fluorescence (FITC) superimposed on a D.I.C. image. Figures 20B and 20E depict the same tissues stained with mAb LM609 (anti-αᵥβ₃) and visualized by fluorescence (rhodamine). Figures 20C and 20F represent merged images of the same tissues stained with both Apop Tag and LM609 where yellow staining represents colocalization. The bar represents 15 and 50 µm in the left and right panels, respectively.

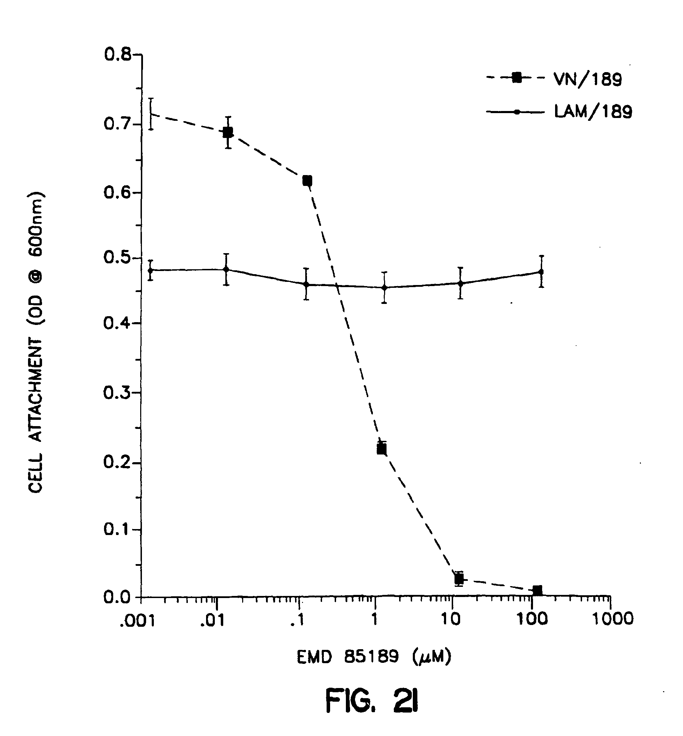

Figure 21 shows the result of a inhibition of cell attachment assay with peptide 85189 as described in Example 4A. The effects of the peptide antagonist was assessed over a dosage range of .001 to 100 uM as plotted on the X-axis. Cell attachment is plotted on the Y-axis measured at an optical density (O.D.) of 600 nm. Cell attachment was measured on vitronectin- (broken lines) versus laminin- (solid lines) coated surfaces.

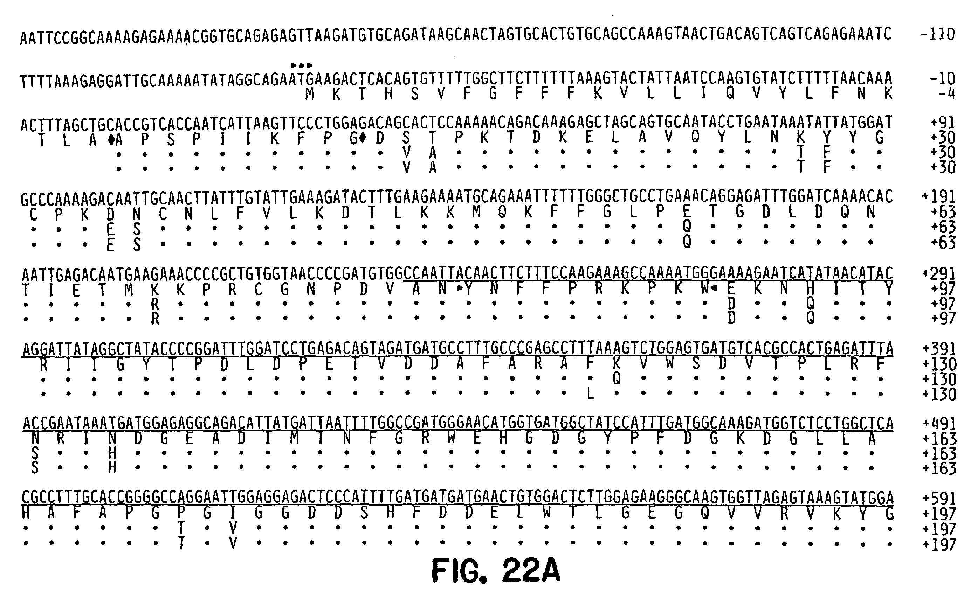

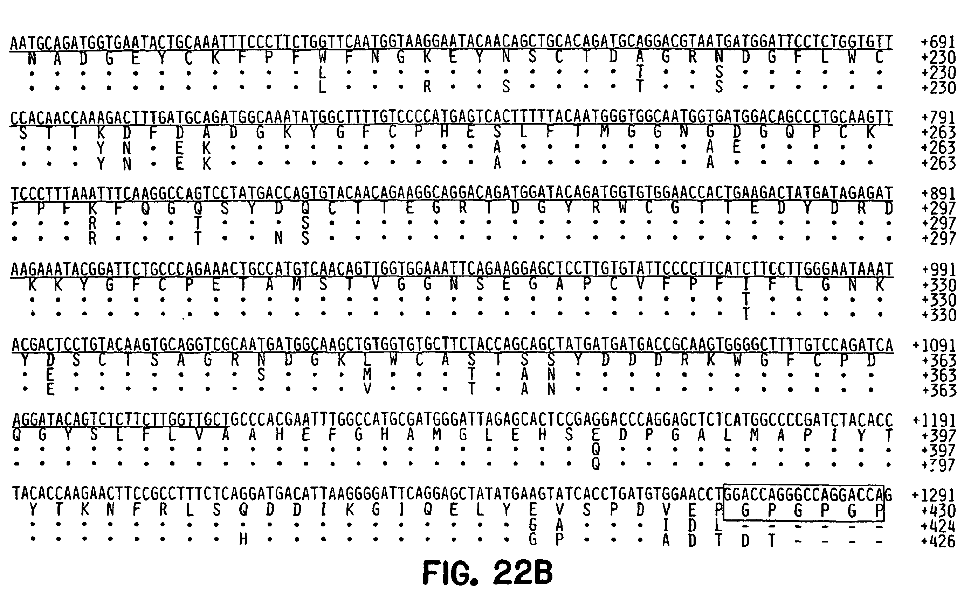

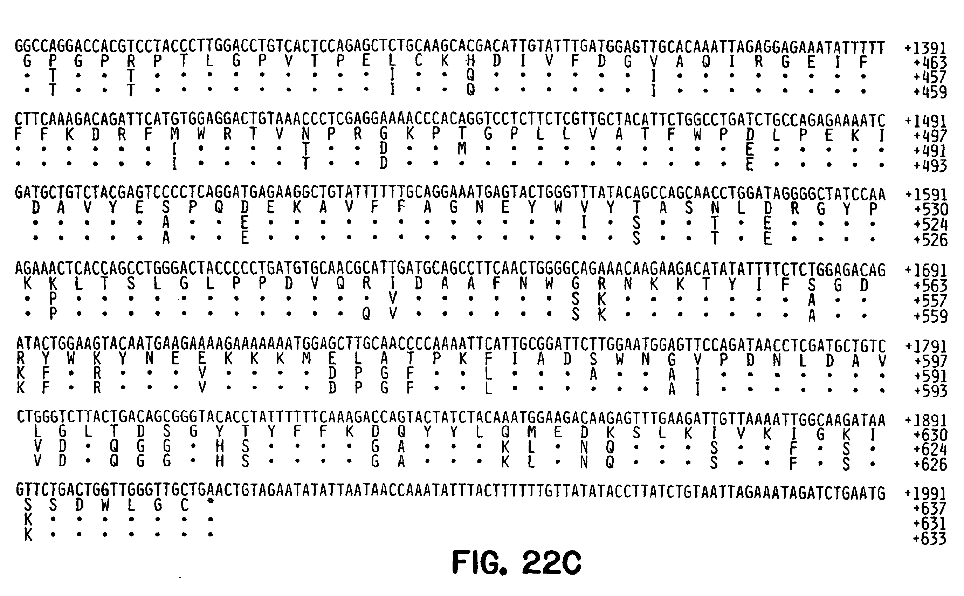

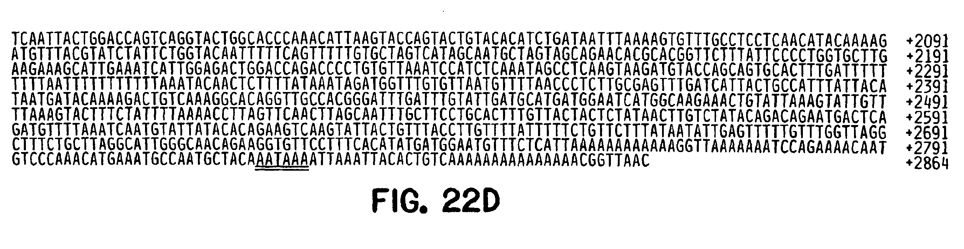

Figures 22A and 22B show the consecutive cDNA sequence of chicken MMP-2 along with the deduced amino acid sequence shown on the second line. The third and fourth lines respectively show the deduced amino acid sequence of human and mouse MMP-2 as described in Example 4A. The chicken cDNA sequence is listed in SEQ ID NO 29 along with the encoded amino acid sequence that is also presented separately as SEQ ID NO 30. The numbering of the first nucleotide of the 5' untranslated region and the region encoding the proenzyme sequence shown in Figure 22A as a negative number is actually presented as number 1 in Sequence Listing making the latter appear longer than the figure; however, the nucleotide sequence is the figure is identical in length and sequence to that as presented in the listing with the exception of the numbering. Accordingly, references to nucleotide position for chicken or human MMP-2 in the specification, such as in primers for use in amplifying MMP-2 fragments, are based on the nucleotide position as indicated in the figure and not as listed in the Sequence Listing.

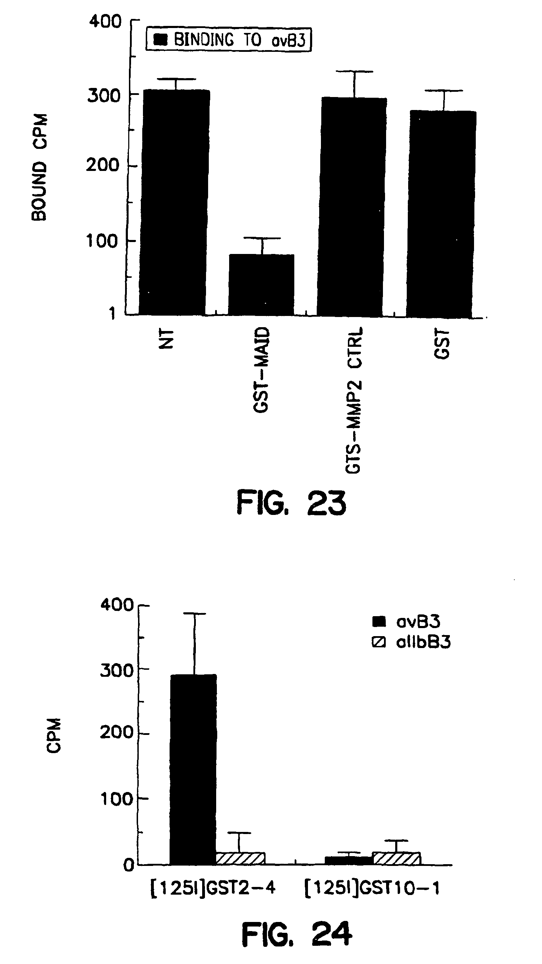

Figure 23 shows the results in bar-graph form of a solid-phase receptor binding assay of iodinated MMP-2 to bind to αᵥβ₃ with and without the presence of inhibitors as further described in Example 4B. The data is plotted as bound CPM on the Y-axis against the various potential inhibitors and controls.
Figure 24 shows the specificity of chicken-derived MMP-2 compositions for either the integrin receptors αᵥβ₃ and α_{11b}β₃ in the presence of MMP-2 inhibitors as further described in Example 4B. The data is presented as described in the legend in Figure 23.

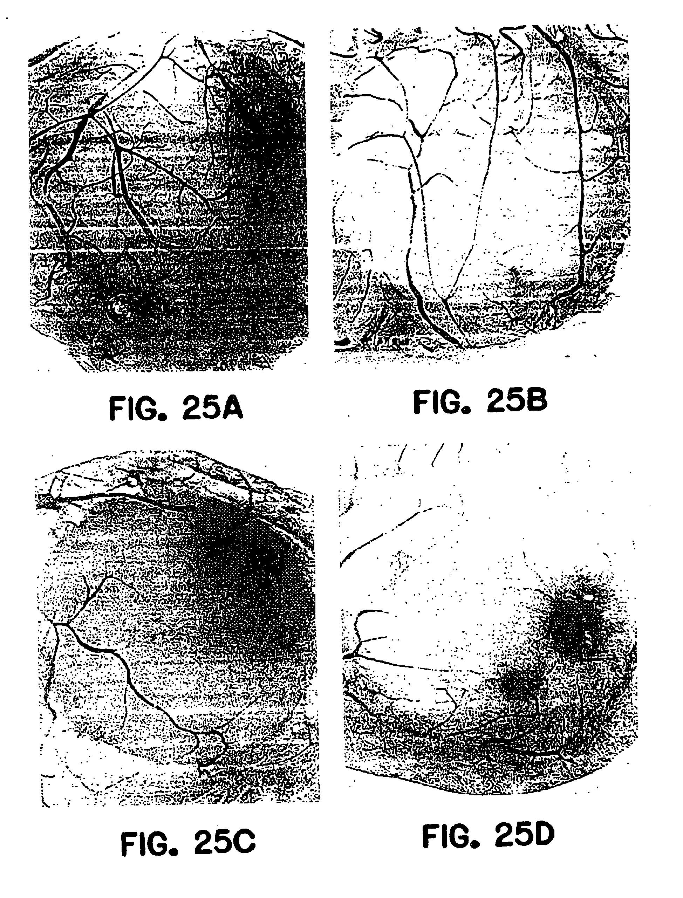

Figure 25 show the effect of chicken MMP-2(410-637) GST fusion protein on bFGF-induced angiogenesis as described in Example 7A3). Figures 25A-B and 25C-D respectively shown control (a non-MMP-2 fragment containing fusion protein) and MMP-2 fragment GST fusion protein effects.

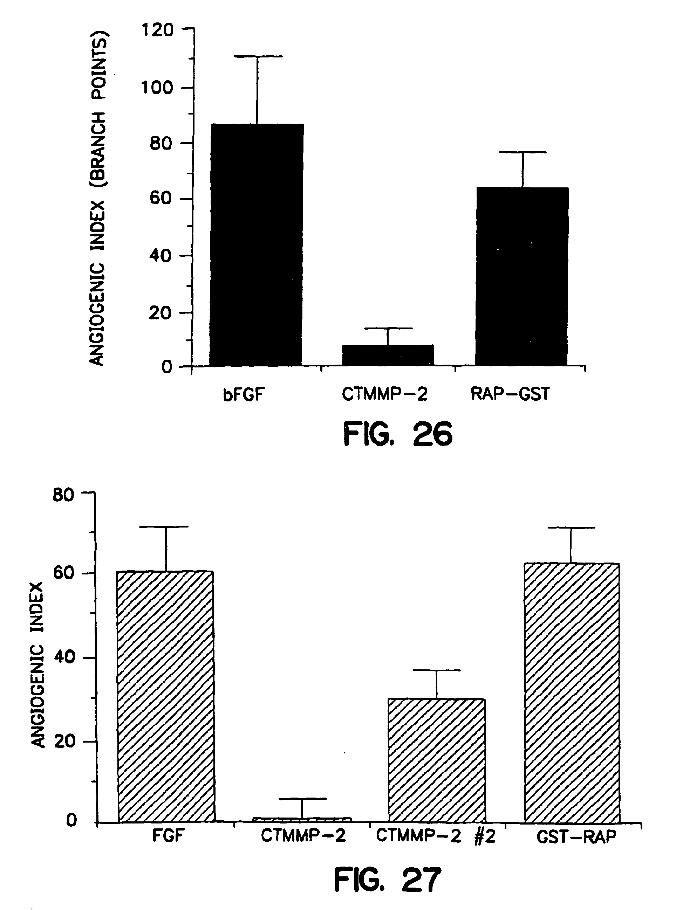

Figures 26 and 27 both illustrate in bar graph form the angiogenesis index (a measurement of branch points) of the effects of chicken MMP-2(410-637) GST fusion protein (labeled CTMMP-2) versus control (RAP-GST or GST-RAP) on bFGF-treated CAMs as described in Example 7A3). Angiogenic index is plotted on the Y-axis against the separate treatments on the X-axis.

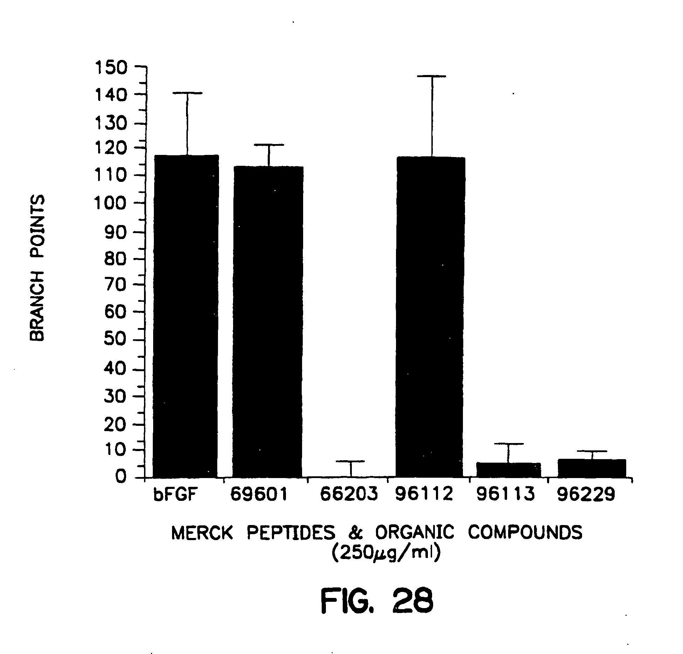

Figure 28 shows the effects of peptides and organic compounds on bFGF-induced angiogenesis as measured by the effect on branch points plotted on the Y-axis against the various treatments on the X-axis, including bFGF alone, and bFGF-treated CAMs with peptides 69601 or 66203 and organic componds 96112, 96113 and 96229, as described in Examples 7B and 14.

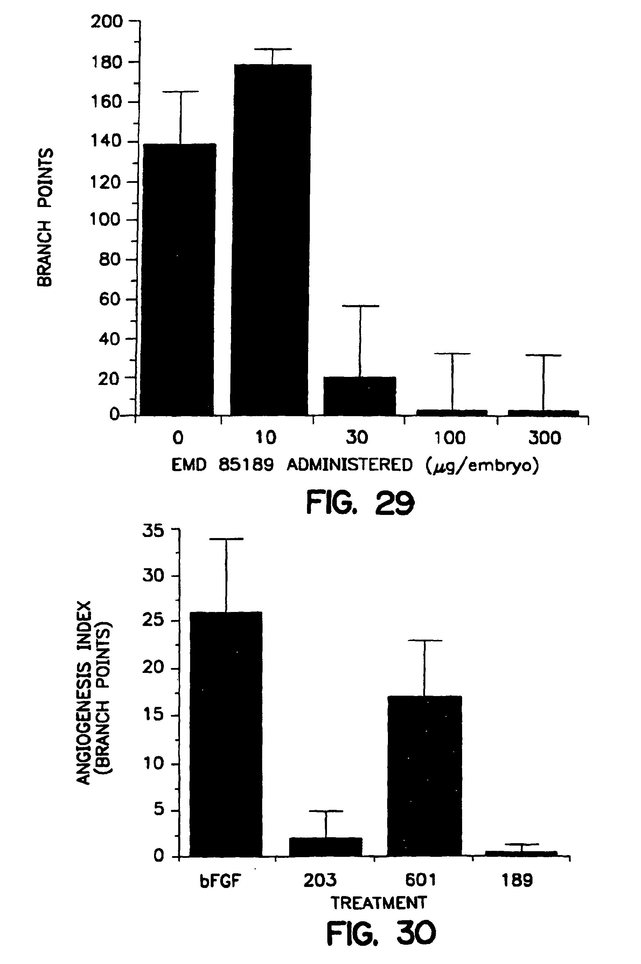

Figure 29 graphically shows the dose response of peptide 85189 on inhibiting bFGF-induced angiogenesis as further described in Example 7B2) where the number of branch points are plotted on the Y-axis against the amount of peptide administered to the embryo on the X-axis.
Figure 30 shows the inhibitory activity of peptides 66203 (labeled 203) and 85189 (labeled 189) in bFGF-induced angiogenesis in the CAM assay as described in Example 7B2). Controls included no peptide in bFGF-treated CAMS and peptide 69601 (labeled 601). The data is plotted as described in the legend for Figure 27.

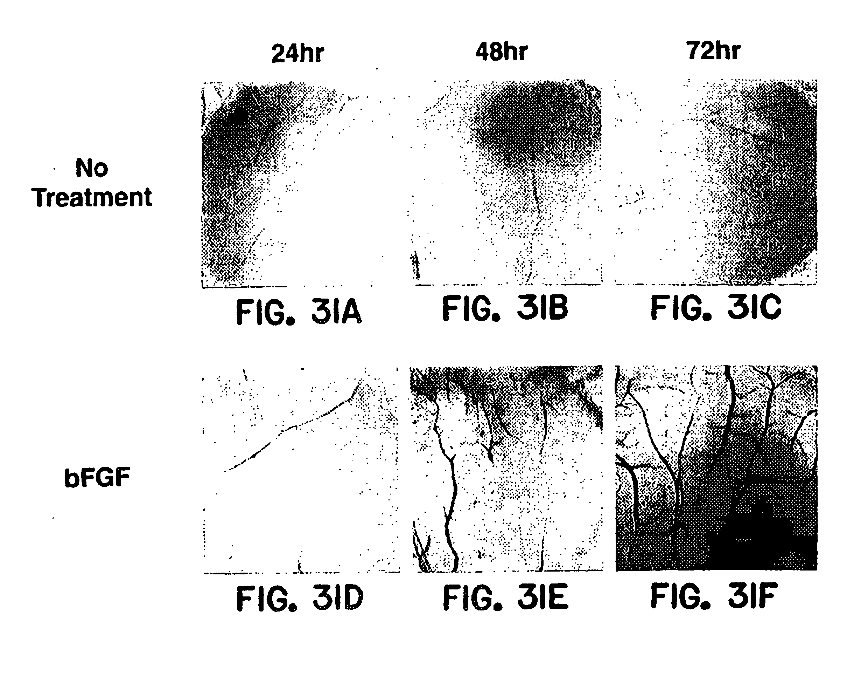

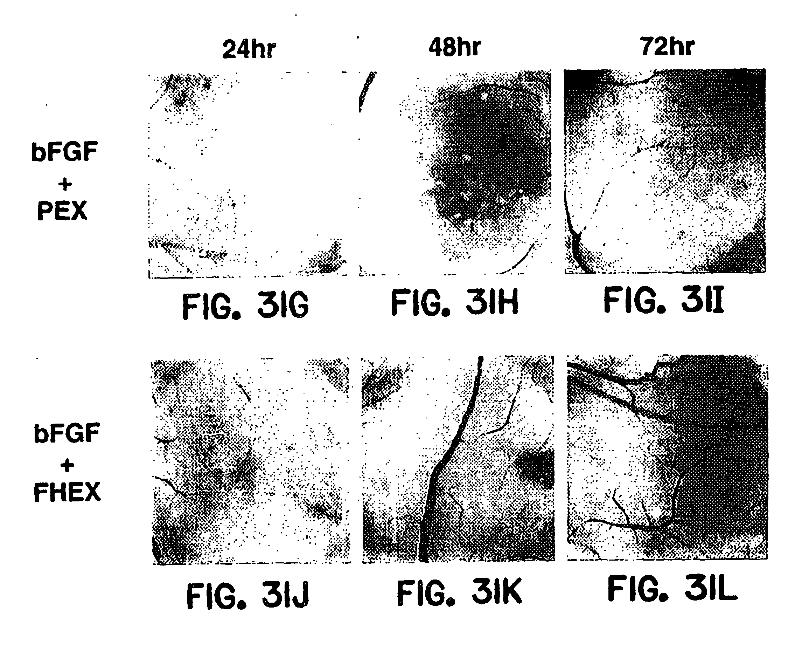

Figures 31A-L show the effect of various treatments against untreated CAM preparations over a time course beginning at 24 hours and ending at 72 hours as further described in Example 7B3). Photographs for the categories labeled untreated, bFGF, bFGF + MAID (bFGF treated followed with exposure to chicken MMP-2(2-4) GST fusion protein) and bFGF + control (bFGF treatment followed by chicken MMP-2(10-1) are respectively shown in Figures 31A-C, 31D-F, 31G-I, and 31J-L.

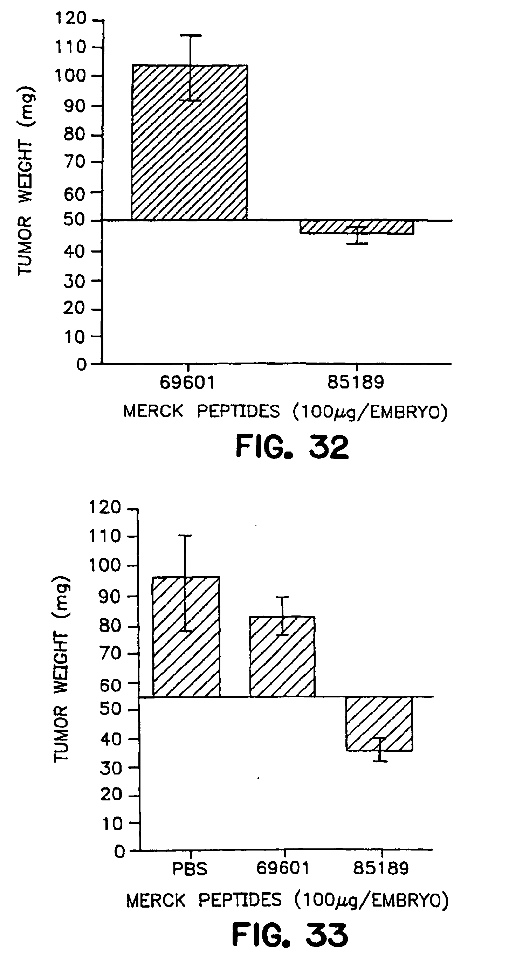

Figures 32, 33 and 34 respectively show the reduction in tumor weight for UCLAP-3, M21-L and FgM tumors following intravenous exposure to control peptide 69601 and antagonist 85189 as further described in Example 9A. The data is plotted with tumor weight on the Y-axis against the peptide treatments on the X-axis.

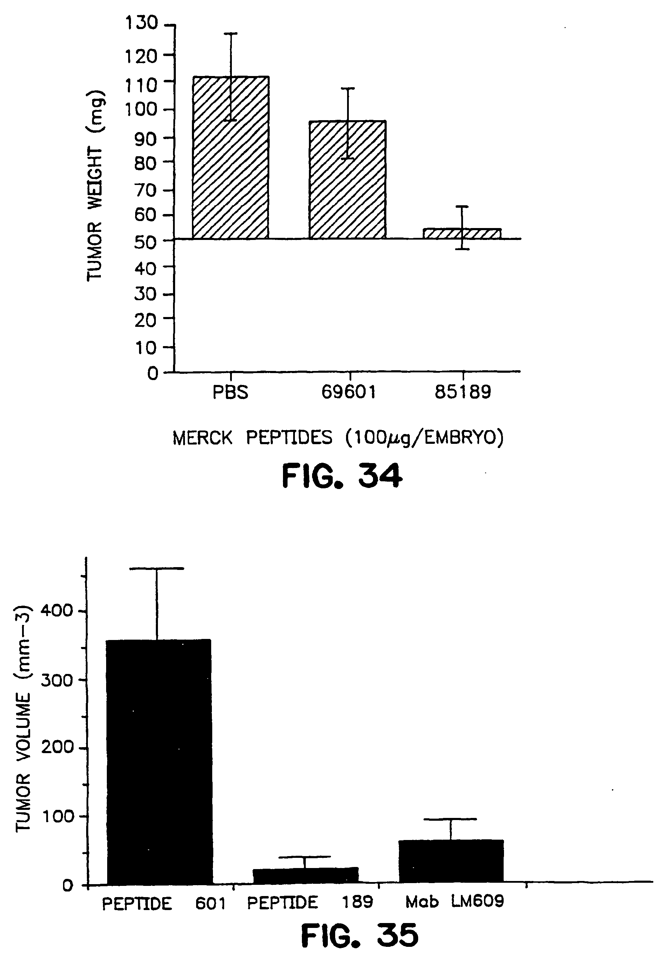

Figure 35 illustrates the effect of peptides and antibodies on melanoma tumor growth in the chimeric mouse:human model as further described in Examples 11B. The peptides assessed included control 69601 (labeled 601) and antagonist 85189 (labeled 189). The antibody tested was LM609. Tumor volume in mm³ is plotted on the Y-axis against the various treatments on the X-axis.

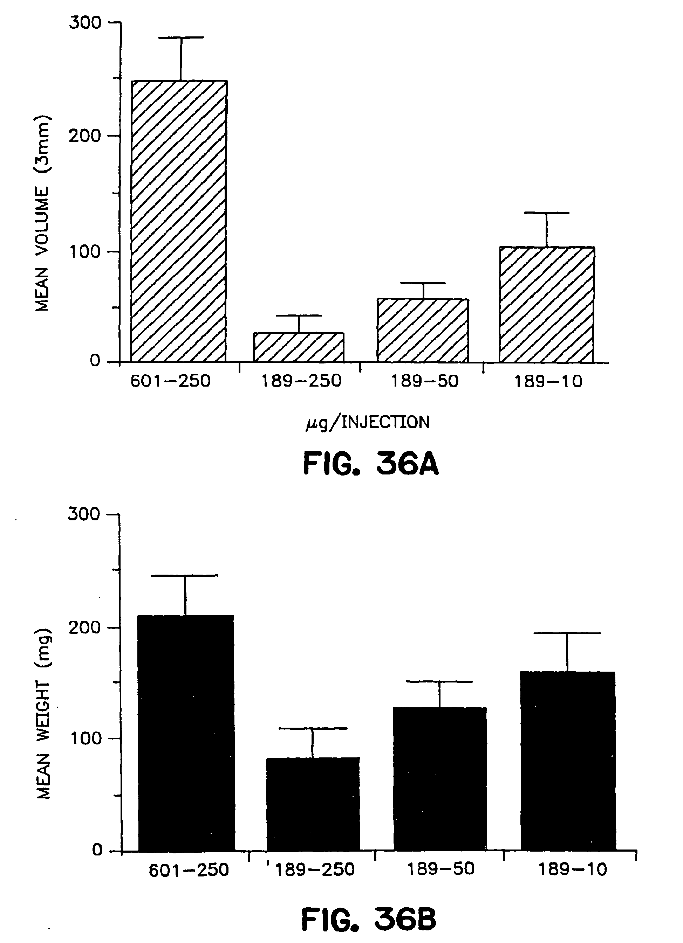

Figures 36A and B respectively show the effect of antagonist 85189 (labeled 189) compared to control peptide 69601 (labeled 601) in reducing the volume and wet weight of M21L tumors over a dosage range of 10, 50 and 250 ug/injection as further described in Example 11C.

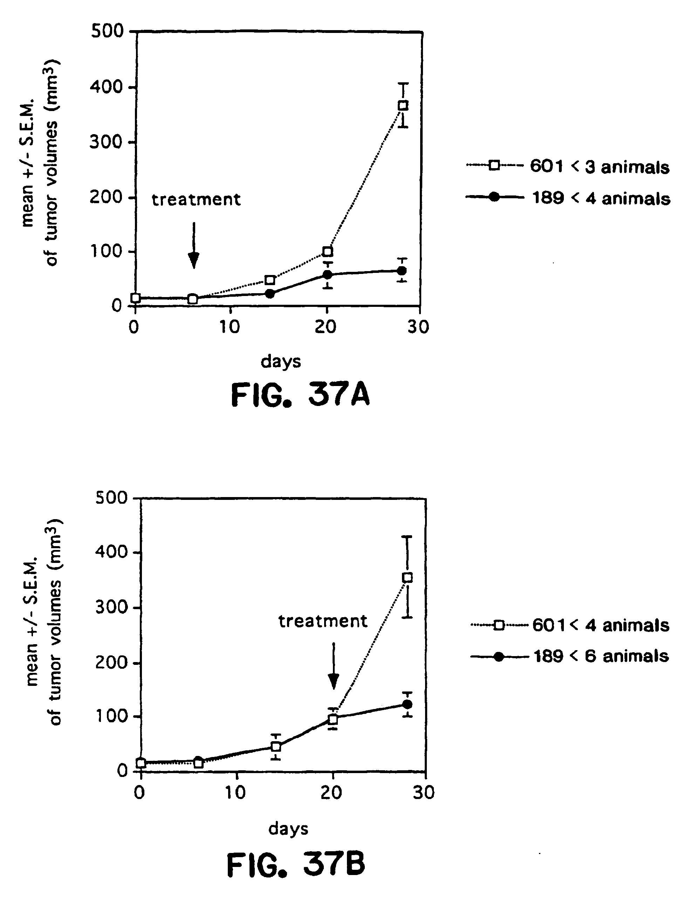

Figures 37A and 37B show the effectiveness of antagonist peptide 85189 (labeled 189 with a solid line and filled circles) against control peptide 69601 (labeled 601 on a dotted line and open squares) at inhibiting M21L tumor volume in the mouse:human model with two different treatment regimens as further described in Example 11C. Tumor volume in mm³ is plotted on the Y-axis against days on the X-axis.

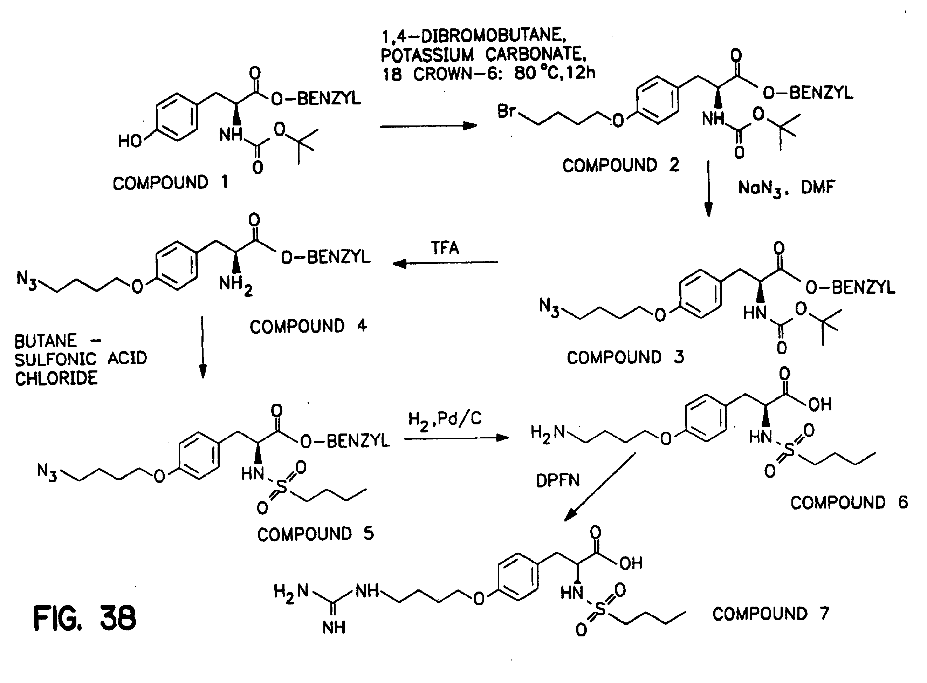

Figures 38 through 42 schematically illustrate the various chemical syntheses of organic molecule αᵥβ₃ antagonists as further described in Example 13.

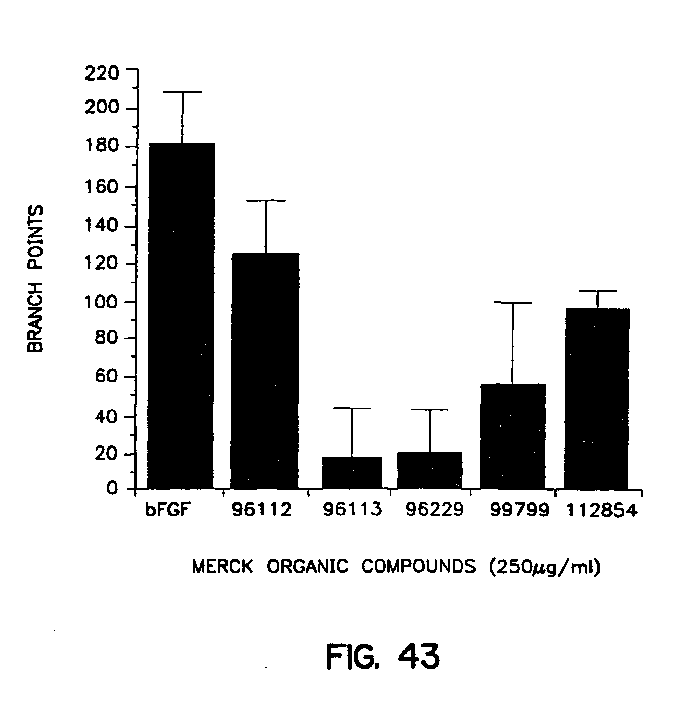

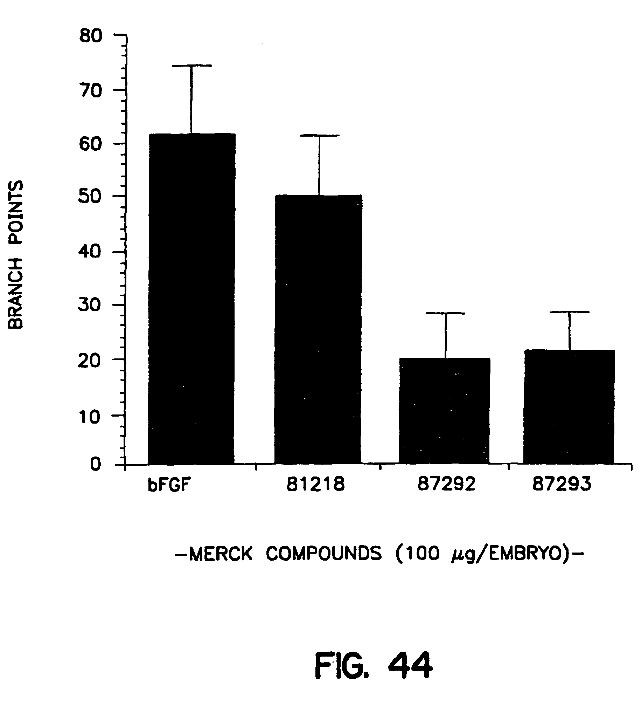

Figures 43 and 44 show the effects of various organic molecules on bFGF-induced angiogenesis in a CAM assay as further described in Example 14. Branch points are plotted on the Y-axis against the various compounds used at 250 ug/ml on the X-axis in Figure 43 and 100 ug/ml in Figure 44.

### Detailed Description of the Invention

### A. Definitions

Amino Acid Residue: An amino acid formed upon chemical digestion (hydrolysis) of a polypeptide at its peptide linkages. The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature (described in J. Biol. Chem., 243:3552-59 (1969) and adopted at 37 CFR §1.822(b)(2)), abbreviations for amino acid residues are shown in the following Table of Correspondence:

**TABLE OF CORRESPONDENCE**

| SYMBOL | | AMINO ACID |
|---|---|---|
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| Z | Glx | Glu and/or Gln |
| W | Trp | tryptophan |
| R | Arg | arginine |
| D | Asp | aspartic acid |
| N | Asn | asparagine |
| B | Asx | Asn and/or Asp |
| C | Cys | cysteine |
| X | Xaa | unknown/other |

In addition the following have the meanings below:

| | |
|---|---|
| BOC | tert-butyloxycarbonyl |
| DCCI | dicylcohexylcarbodiimide |
| DMF | dimethylformamide |
| OMe | methoxy |
| HOBt | 1-hydroxybezotriazole |

It should be noted that all amino acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino acid residues.

Polypeptide: refers to a linear series of amino acid residues connected to one another by peptide bonds between the alpha-amino group and carboxy group of contiguous amino acid residues.

Peptide: as used herein refers to a linear series of no more than about 50 amino acid residues connected one to the other as in a polypeptide.

Cyclic peptide: refers to a compound having a heteroatom ring structure that includes several amide bonds as in a typical peptide. The cyclic peptide can be a "head to tail" cyclized linear polypeptide in which a linear peptide's n-terminus has formed an amide bond with the terminal carboxylate of the linear peptide, or it can contain a ring structure in which the polymer is homodetic or heterodetic and comprises amide bonds and/or other bonds to close the ring, such as disulfide bridges, thioesters, thioamides, guanidino, and the like linkages.

Protein: refers to a linear series of greater than 50 amino acid residues connected one to the other as in a polypeptide.

Fusion protein: refers to a polypeptide containing at least two different polypeptide domains operatively linked by a typical peptide bond ("fused"), where the two domains correspond to peptides no found fused in nature.

Synthetic peptide: refers to a chemically produced chain of amino acid residues linked together by peptide bonds that is free of naturally occurring proteins and fragments thereof.

### B. General Considerations

The present invention relates generally to the discovery that angiogenesis is mediated by the specific vitronectin receptor αᵥβ₃, and that inhibition of αᵥβ₃ function inhibits angiogenesis. This discovery is important because of the role that angiogenesis plays in a variety of disease processes. By inhibiting angiogenesis, one can intervene in the disease, ameliorate the symptoms, and in some cases cure the disease.

Where the growth of new blood vessels is the cause of, or contributes to, the pathology associated with a disease, inhibition of angiogenesis will reduce the deleterious effects of the disease. Examples include rheumatoid arthritis, diabetic retinopathy, inflammatory diseases, restenosis, and the like. Where the growth of new blood vessels is required to support growth of a deleterious tissue, inhibition of angiogenesis will reduce the blood supply to the tissue and thereby contribute to reduction in tissue mass based on blood supply requirements. Examples include growth of tumors where neovascularization is a continual requirement in order that the tumor grow beyond a few millimeters in thickness, and for the establishment of solid tumor metastases.

The compounds for use according to the present invention are effective in part because the therapy is highly selective for angiogenesis and not other biological processes. As shown in the Examples, only new vessel growth contains substantial αᵥβ₃, and therefore the therapeutic methods do not adversely effect mature vessels. Furthermore, αᵥβ₃ is not widely distributed in normal tissues, but rather is found selectively on new vessels, thereby assuring that the therapy can be selectively targeted to new vessel growth.

Prior to the discoveries of the present invention, it was not known that angiogenesis, and any of the processes dependent on angiogenesis, could be inhibited in vivo by the use of reagents defined in claim 1 that antagonize the biological function of αᵥβ₃.

### C. Methods For Inhibition of Angiogenesis

The invention provides compounds, as defined in claim 1, for use in a method for the inhibition of angiogenesis in a tissue, and thereby inhibiting events in the tissue which depend upon angiogenesis. Generally, the compounds are to be administered to the tissue in a composition comprising an angiogenesis-inhibiting amount of said αᵥβ₃ antagonist.

As described earlier, angiogenesis includes a variety of processes involving neovascularization of a tissue including "sprouting", vasculogenesis, or vessel enlargement, all of which angiogenesis processes are mediated by and dependent upon the expression of αᵥβ₃. With the exception of traumatic wound healing, corpus leuteum formation and embryogenesis, it is believed that the majority of angiogenesis processes are associated with disease processes and therefore the use of the present therapeutic methods are selective for the disease and do not have deleterious side effects.

There are a variety of diseases in which angiogenesis is believed to be important, referred to as angiogenic diseases, including but not limited to, inflammatory disorders such as immune and non-immune inflammation, chronic articular rheumatism and psoriasis, disorders associated with inappropriate or inopportune invasion of vessels such as diabetic retinopathy, neovascular glaucoma, restenosis, capillary proliferation in atherosclerotic plaques and osteoporosis, and cancer associated disorders, such as solid tumors, solid tumor metastases, angiofibromas, retrolental fibroplasia, hemangiomas, Kaposi sarcoma and the like cancers which require neovascularization to support tumor growth.

Thus, methods which inhibit angiogenesis in a diseased tissue ameliorates symptoms of the disease and, depending upon the disease, can contribute to cure of the disease. In one embodiment, the invention contemplates the compounds as defined in claim 1 for use in inhibition of angiogenesis, per se, in a tissue. The extent of angiogenesis in a tissue, and therefore the extent of inhibition achieved by the present compounds, can be evaluated by a variety of methods, such as are described in the Examples for detecting αᵥβ₃-immunopositive immature and nascent vessel structures by immunohistochemistry.

As described herein, any of a variety of tissues, or organs comprised of organized tissues, can support angiogenesis in disease conditions including skin, muscle, gut, connective tissue, joints, bones and the like tissue in which blood vessels can invade upon angiogenic stimuli.

Thus, in one related embodiment, a tissue to be treated is an inflamed tissue and the angiogenesis to be inhibited is inflamed tissue angiogenesis where there is neovascularization of inflamed tissue. In this class the compounds defined in claim 1 are contemplated for use in inhibition of angiogenesis in arthritic tissues, such as in a patient with chronic articular rheumatism, in immune or non-immune inflamed tissues, in psoriatic tissue and the like.

The patient to be treated according to the present invention in its many embodiments is desirably a human patient, although it is to be understood that the principles of the invention indicate that the invention is effective with respect to all mammals, which are intended to be included in the term "patient". In this context, a mammal is understood to include any mammalian species in which treatment of diseases associated with angiogenesis is desirable, particularly agricultural and domestic mammalian species.

In another related embodiment, a tissue to be treated is a retinal tissue of a patient with a retinal disease such as diabetic retinopathy, macular degeneration or neovascular glaucoma and the angiogenesis to be inhibited is retinal tissue angiogenesis where there is neovascularization of retinal tissue.

In an additional related embodiment, a tissue to be treated is a tumor tissue of a patient with a solid tumor, a metastases, a skin cancer, a breast cancer, a hemangioma or angiofibroma and the like cancer, and the angiogenesis to be inhibited is tumor tissue angiogenesis where there is neovascularization of a tumor tissue. Typical solid tumor tissues treatable by the present methods include lung, pancreas, breast, colon, laryngeal, ovarian, and the like tissues. Exemplary tumor tissue angiogenesis, and inhibition thereof, is described in the Examples.

Inhibition of tumor tissue angiogenesis is a particularly preferred embodiment because of the important role neovascularization plays in tumor growth. In the absence of neovascularization of tumor tissue, the tumor tissue does not obtain the required nutrients, slows in growth, ceases additional growth, regresses and ultimately becomes necrotic resulting in killing of the tumor.

Stated in other words, the present invention provides compounds for use in a method of inhibiting tumor neovascularization by inhibiting tumor angiogenesis according to the present methods. Similarly, the invention provides angiogenesis-inhibiting compounds, for use in inhibiting tumor growth.

The compounds are also particularly effective against the formation of metastases because (1) their formation requires vascularization of a primary tumor so that the metastatic cancer cells can exit the primary tumor and (2) their establishment in a secondary site requires neovascularization to support growth of the metastases.

In a related embodiment, the invention contemplates the practice of the compounds for use in the method in conjunction with other therapies such as conventional chemotherapy directed against solid tumors and for control of establishment of metastases. The angiogenesis inhibitor is typically to be administered during or after chemotherapy, although it is preferable to inhibit angiogenesis after a regimen of chemotherapy at times where the tumor tissue will be responding to the toxic assault by inducing angiogenesis to recover by the provision of a blood supply and nutrients to the tumor tissue. In addition, it is preferred that the compounds for use in angiogenesis inhibition are to be administered after surgery where solid tumors have been removed as a prophylaxis against metastases.

Insofar as the compounds are for use in inhibition of tumor neovascularization, they can also be for use in inhibition of tumor tissue growth, inhibition of tumor metastases formation, and regression of established tumors. The Examples demonstrate regression of an established tumor following a single intravenous administration of an αᵥβ₃ antagonist according to this invention.

Restenosis is a process of smooth muscle cell (SMC) migration and proliferation at the site of percutaneous transluminal coronary angioplasty which hampers the success of angioplasty. The migration and proliferation of SMC's during restenosis can be considered a process of angiogenesis which is inhibited by the present compounds. Therefore, the invention also contemplates compounds for use in inhibition of restenosis by inhibiting angiogenesis in a patient following angioplasty procedures. For inhibition of restenosis, the αᵥβ₃ antagonist is typically to be administered after the angioplasty procedure for from about 2 to about 28 days, and more typically for about the first 14 days following the procedure.

The present compounds for use in inhibiting angiogenesis in a tissue, and therefore also for treatment of angiogenesis-related diseases, are to be administered to a tissue in which angiogenesis is occurring, or is at risk for occurring, whereby the compounds are in a composition comprising a therapeutically effective amount of an αᵥβ₃ antagonist capable of inhibiting αᵥβ₃ binding to its natural ligand. Thus the compounds for use in the method are to be administered to a patient in a therapeutically effective amount of a physiologically tolerable composition containing an αᵥβ₃ antagonist.

The dosage ranges for the administration of the αᵥβ₃ antagonist depend upon the form of the antagonist, and its potency, as described further herein, and are amounts large enough to produce the desired effect in which angiogenesis and the disease symptoms mediated by angiogenesis are ameliorated. The dosage should not be so large as to cause adverse side effects, such as hyperviscosity syndromes, pulmonary edema, congestive heart failure, and the like. Generally, the dosage will vary with the age, condition, sex and extent of the disease in the patient and can be determined by one of skill in the art. The dosage can also be adjusted by the individual physician in the event of any complication.

A therapeutically effective amount is an amount of αᵥβ₃ antagonist sufficient to produce a measurable inhibition of angiogenesis in the tissue being treated, ie., an angiogenesis-inhibiting amount. Inhibition of angiogenesis can be measured in situ by immunohistochemistry, as described herein, or by other methods known to one skilled in the art.

Insofar as an αᵥβ₃ antagonist can take the form of any of five different organic mimetics, as defined in claim 1, it is to be appreciated that the potency, and therefore an expression of a "therapeutically effective" amount can vary. However, as shown by the present assay methods, one skilled in the art can readily assess the potency of an αᵥβ₃ antagonist for use according to this invention.

Potency of an αᵥβ₃ antagonist can be measured by a variety of means including inhibition of angiogenesis in the CAM assay, in the in vivo rabbit eye assay, in the in vivo chimeric mouse:human assay, and by measuring inhibition of binding of natural ligand to αᵥβ₃, all as described herein, and the like assays.

A preferred αᵥβ₃ antagonist has the ability to substantially inhibit binding of a natural ligand such as fibrinogen or vitronectin to αᵥβ₃ in solution at antagonist concentrations of less than 0.5 micromolar (um), preferably less than 0.1 um, and more preferably less than 0.05 um. By "substantially" is meant that at least a 50 percent reduction in binding of fibrinogen is observed by inhibition in the presence of the αᵥβ₃ antagonist, and at 50% inhibition is referred to herein as an IC₅₀ value.

A more preferred αᵥβ₃ antagonist exhibits selectivity for αᵥβ₃ over other integrins. Thus, a preferred αᵥβ₃ antagonist substantially inhibits fibrinogen binding to αᵥβ₃ but does not substantially inhibit binding of fibrinogen to another integrin, such as αᵥβ₁, αᵥβ₅ or α_{IIb}β₃. Particularly preferred is an αᵥβ₃ antagonist that exhibits a 10-fold to 100-fold lower IC₅₀ activity at inhibiting fibrinogen binding to αᵥβ₃ compared to the IC₅₀ activity at inhibiting fibrinogen binding to another integrin. Exemplary assays for measuring IC₅₀ activity at inhibiting fibrinogen binding to an integrin are described in the Examples.

A therapeutically effective amount of an αᵥβ₃ antagonist is described as an amount of polypeptide such that when administered in a physiologically tolerable composition is sufficient to achieve a plasma concentration of from about 0.1 microgram (ug) per milliliter (ml) to about 200 ug/ml, preferably from about 1 ug/ml to about 150 ug/ml. Based on a polypeptide having a mass of about 500 grams per mole, the preferred plasma concentration in molarity is from about 2 micromolar (uM) to about 5 millimolar (mM) and preferably about 100 uM to 1 mM polypeptide antagonist. Stated differently, the dosage per body weight can vary from about 0.1 mg/kg to about 300 mg/kg, and preferably from about 0.2 mg/kg to about 200 mg/kg, in one or more dose administrations daily, for one or several days.

The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

In one preferred embodiment as shown in the Examples, the αᵥβ₃ antagonist is to be administered in a single dosage intravenously.

The compositions are to be administered in a manner compatible with the dosage formulation, and in a therapeutically effective amount. The quantity to be administered and timing depends on the subject to be treated, capacity of the subject's system to utilize the active ingredient, and degree of therapeutic effect desired. Precise amounts of active ingredient required to be administered depend on the judgement of the practitioner and are peculiar to each individual. However, suitable dosage ranges for systemic application are disclosed herein and depend on the route of administration. Suitable regimes for administration are also variable, but are typified by an initial administration followed by repeated doses at one or more hour intervals by a subsequent injection or other administration. Alternatively, continuous intravenous infusion sufficient to maintain concentrations in the blood in the ranges specified for in vivo therapies are contemplated.

As demonstrated by the present Examples, inhibition of angiogenesis and tumor regression occurs as early as 7 days after the initial contacting with antagonist. Additional or prolonged exposure to antagonist is preferable for 7 days to 6 weeks, preferably about 14 to 28 days.

In a related embodiment, the Examples demonstrate the relationship between inhibition of αᵥβ₃ and induction of apoptosis in the neovasculature cells bearing αᵥβ₃. Thus, compounds for use in methods for inhibition of apoptosis in neovasculature of a tissue are also described. The compounds are used substantially as described herein for inhibition of angiogenesis in all tissues and conditions described therefor. The only noticeable difference is one of timing of effect, which is that apoptosis is manifest quickly, typically about 48 hours after contacting antagonist, whereas inhibition of angiogenesis and tumor regression is manifest more slowly, as described herein. This difference affects the therapeutic regimen in terms of time of administration, and effect desired. Typically, administration for apoptosis of neovasculature can be for 24 hours to about 4 weeks, although 48 hours to 7 days is preferred.

### D. Therapeutic Compositions

The present invention contemplates therapeutic compositions useful for practicing the therapeutic methods described herein. Therapeutic compositions for use according to the present invention contain a physiologically tolerable carrier together with an αᵥβ₃ antagonist as described herein, dissolved or dispersed therein as an active ingredient. In a preferred embodiment, the therapeutic αᵥβ₃ antagonist composition is not immunogenic when administered to a mammal or human patient for therapeutic purposes.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Particularly preferred are the salts of TFA and HCl, when used in the preparation of cyclic polypeptide αᵥβ₃ antagonists. Representative salts of peptides are described in the Examples.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

A therapeutic composition contains an angiogenesis-inhibiting amount of an αᵥβ₃ antagonist of the present invention, typically formulated to contain an amount of at least 0.1 weight percent of antagonist per weight of total therapeutic composition. A weight percent is a ratio by weight of inhibitor to total composition. Thus, for example, 0.1 weight percent is 0.1 grams of inhibitor per 100 grams of total composition.

### E. Antagonists of Integrin αᵥβ₃

The present invention relates to certain αᵥβ₃ antagonists, designated "mimetics", which possess the ability to "mimic" a binding domain on the αᵥβ₃ ligand involved in the functional interaction of the receptor and ligand, and thereby interfere with (i.e., inhibit) normal function, for use in inhibiting αᵥβ₃-mediated angiogenesis in a tissue.

A mimetic of this invention is an organic-based molecule and thus is referred to as organic mimetic. The organic mimetic molecules that function as αᵥβ₃ antagonists by being a mimetic to a ligand of αᵥβ₃ are Compounds 7, 9, 10, 12 and 14, as described in Example 10.

### F. Methods For Assaying Antagonism of αᵥβ₃

A number of methods are available for assaying antagonism of αᵥβ₃.

The first assay measures inhibition of direct binding of natural ligand to αᵥβ₃, and a preferred embodiment is described in detail in the Examples. The assay typically measures the degree of inhibition of binding of a natural ligand, such as fibrinogen, to isolated αᵥβ₃ in the solid phase by ELISA.

The second assay measures angiogenesis in the chick chorioallantoic membrane (CAM) and is referred to as the CAM assay. The CAM assay has been described in detail by others, and further has been used to measure both angiogenesis and neovascularization of tumor tissues. See Ausprunk et al., Am. J. Pathol., 79:597-618 (1975) and Ossonski et al., Cancer Res., 40:2300-2309 (1980).

The CAM assay is a well recognized assay model for in vivo angiogenesis because neovascularization of whole tissue is occurring, and actual chick embryo blood vessels are growing into the CAM or into the tissue grown on the CAM.

As demonstrated herein, the CAM assay illustrates inhibition of neovascularization based on both the amount and extent of new vessel growth. Furthermore, it is easy to monitor the growth of any tissue transplanted upon the CAM, such as a tumor tissue. Finally, the assay is particularly useful because there is an internal control for toxicity in the assay system. The chick embryo is exposed to any test reagent, and therefore the health of the embryo is an indication of toxicity.

The third assay that measures angiogenesis is the in vivo rabbit eye model and is referred to as the rabbit eye assay. The rabbit eye assay has been described in detail by others, and further has been used to measure both angiogenesis and neovascularization in the presence of angiogenic inhibitors such as thalidomide. See D'Amato, et al., Proc. Natl. Acad. Sci., USA, 91:4082-4085 (1994).

The rabbit eye assay is a well recognized assay model for in vivo angiogenesis because the neovascularization process, exemplified by rabbit blood vessels growing from the rim of the cornea into the cornea, is easily visualized through the naturally transparent cornea of the eye. Additionally, both the extent and the amount of stimulation or inhibition of neovascularization or regression of neovascularization can easily be monitored over time.

Finally, the rabbit is exposed to any test reagent, and therefore the health of the rabbit is an indication of toxicity of the test reagent.

The fourth assay measures angiogenesis in the chimeric mouse:human mouse model and is referred to as the chimeric mouse assay. The assay has been described in detail by others, and further has been described herein to measure angiogenesis, neovascularization, and regression of tumor tissues. See Yan, et al., J. Clin. Invest., 91:986-996 (1993). The chimeric mouse assay is a useful assay model for in vivo angiogenesis because the transplanted skin grafts closely resemble normal human skin histologically and neovascularization of whole tissue is occurring wherein actual human blood vessels are growing from the grafted human skin into the human tumor tissue on the surface of the grafted human skin. The origin of the neovascularization into the human graft can be demonstrated by immunohistochemical staining of the neovasculature with human-specific endothelial cell markers.

As demonstrated herein, the chimeric mouse assay demonstrates regression of neovascularization based on both the amount and extent of regression of new vessel growth. Furthermore, it is easy to monitor effects on the growth of any tissue transplanted upon the grafted skin, such as a tumor tissue. Finally, the assay is useful because there is an internal control for toxicity in the assay system. The chimeric mouse is exposed to any test reagent, and therefore the health of the mouse is an indication of toxicity.

### G. Article of Manufacture

Also described is an article of manufacture which is a labelled container for providing an αᵥβ₃ antagonist for use according to the invention. An article of manufacture comprises packaging material and a pharmaceutical agent contained within the packaging material.

The pharmaceutical agent in an article of manufacture is any of the αᵥβ₃ antagonists for use according to the present invention, formulated into a pharmaceutically acceptable form as described herein according the the disclosed indications. The article of manufacture contains an amount of pharmaceutical agent sufficient for use in treating a condition indicated herein, either in unit or multiple dosages.

The packaging material comprises a label which indicates the use of the pharmaceutical agent contained therein, e.g., for treating conditions assisted by the inhibition of angiogenesis, and the like conditions disclosed herein. The label can further include instructions for use and related information as may be required for marketing. The packaging material can include container(s) for storage of the pharmaceutical agent.

As used herein, the term packaging material refers to a material such as glass, plastic, paper, foil, and the like capable of holding within fixed means a pharmaceutical agent. Thus, for example, the packaging material can be plastic or glass vials, laminated envelopes and the like containers used to contain a pharamaceutical composition including the pharmaceutical agent.

The packaging material may include a label that is a tangible expression describing the contents of the article of manufacture and the use of the pharmaceutical agent contained therein.

### Examples

The following examples relating to this invention are illustrative and should not, of course, be construed as specifically limiting the invention.

### 1. Preparation of Synthetic Peptides (not part of the invention)

### a. Synthesis Procedure

The linear and cyclic polypeptides listed in Table 1 were synthesized using standard solid-phase synthesis techniques as, for example, described by Merrifield, Adv. Enzymol., 32:221-96, (1969), and Fields, G.B. and Noble, R.L., Int. J. Peptide Protein Res., 35:161-214, (1990).

Two grams (g) of BOC-Gly-D-Arg-Gly-Asp-Phe-Val-OMe (SEQ ID NO 1) were first dissolved in 60 milliliters (ml) of methanol to which was added 1.5 ml of 2 N sodium hydroxide solution to form an admixture. The admixture was then stirred for 3 hours at 20 degrees C (20C). After evaporation, the residue was taken up in water, acidified to pH 3 with diluted HCl and extracted with ethyl acetate. The extract was dried over Na₂SO₄, evaporated again and the resultant BOC-Gly-D-Arg-Gly-Asp-Phe-Val-OH (SEQ ID NO 2) was stirred at 20C for 2 hours with 20 ml of 2 N HCl in dioxane. The resultant admixture was evaporated to obtain H-Gly-D-Arg-Gly-Asp-Phe-Val-OH (SEQ ID NO 3) that was subsequently dissolved in a mixture of 1800 ml of dichloromethane and 200 ml of dimethylformamide (DMF) followed by cooling to 0C. Thereafter, 0.5 g of dicyclohexylcarbodiimide (DCCI), 0.3 g of 1-hydroxybenzotriazole (HOBt) and 0.23 ml of N-methylmorpholine were added successively with stirring.

The resultant admixture was stirred for a further 24 hours at 0C and then at 20C for another 48 hours. The solution was concentrated and treated with a mixed bed ion exchanger to free it from salts. After the resulting resin was removed by filtration, the clarified solution was evaporated and the residue was purified by chromatography resulting in the recovery of cyclo(-Gly-D-Arg-Gly-Asp-Phe-Val) (SEQ ID NO 4). The following peptides, listed in Table 1 using single letter code amino acid residue abbreviations and identified by a peptide number designation, were obtained analogously: cyclo(Arg-Gly-Asp-D-Phe-Val) (SEQ ID NO 5); cyclo(Arg-Ala-Asp-D-Phe-Val) (SEQ ID NO 6); cyclo(Arg-D-Ala-Asp-Phe-Val) (SEQ ID NO 9); cyclo(Arg-Gly-Asp-Phe-D-Val) (SEQ ID NO 7); and cyclo (Arg-Gly-Asp-D-Phe-NMeVal) (methylation is at the alpha-amino nitrogen of the amide bond of the valine residue) (SEQ ID NO 15).

A peptide designated as 66203, having an identical sequence to that of peptide 62184, only differed from the latter by containing the salt HCl rather than the TFA salt present in 62184. The same is true for the peptides 69601 and 62185 and for 85189 and 121974.

### b. Alternate Synthesis Procedure

### i. Synthesis of cyclo-(Arg-Gly-Asp-DPhe-NmeVal). TFA salt

Fmoc-Arg(Mtr)-Gly-Asp(OBut)-DPhe-NMeVal-ONa is synthesized using solid-phase Merrifield-type procedures by sequentially adding NMeVal, DPhe, Asp(OBut), Gly and Fmoc-Arg(Mtr) in a step-wise manner to a 4-hydroxymethyl-phenoxymethyl-polystyrene resin (Wang type resin) (customary Merrifield-type methods of peptide synthesis are applied as described in Houben-Weyl, 1.c., Volume 15/II, Pages 1 to 806 (1974). The polystyrene resin and amino acid residues precursors are commercially available from Aldrich, Sigma or Fluka chemical companies). After completion of sequential addition of the amino acid residues, the resin is then eliminated from the peptide chain using a 1:1 mixture of TFA/dichloromethane which provides the Fmoc-Arg(Mtr)-Gly-Asp(OBut)-DPhe-NMeVal-OH product. The Fmoc group is then removed with a 1:1 mixture of piperidine/DMF which provides the crude Arg(Mtr)-Gly-Asp(OBut)-DPhe-NMeVal-OH precursor which is then purified by HPLC in the customary manner.

For cyclization, a solution of 0.6 g of Arg(Mtr)-Gly-Asp(OBut)-DPhe-NMeVal-OH (synthesized above) in 15 ml of DMF (dimethylformamide; Aldrich) is diluted with 85 ml of dichloromethane (Aldrich), and 50 mg of NaHCO₃ are added. After cooling in a dry ice/acetone mixture, 40 µl of diphenylphosphoryl azide (Aldrich) are added. After standing at room temperature for 16 hours, the solution is concentrated. The concentrate is gel-filtered (Sephadex G10 column in isopropanol/water 8:2) and then purified by HPLC in the customary manner. Treatment with TFA (trifluoroacetic acid)/H₂O (98:2) gives cyclo-(Arg-Gly-Asp-DPhe-NmeVal) x TFA which is then purified by HPLC in the customary manner; RT = 19.5; FAB-MS (M+H): 589.

### ii. Synthesis of "Inner Salt"

TFA salt is removed from the above-produced cyclic peptide by suspending the cyclo-(Arg-Gly-Asp-DPhe-NmeVal) x TFA in water followed by evaporation under vacuum to remove the TFA. The cyclic peptide formed is referred to as an "inner salt" and is designated cyclo-(Arg-Gly-Asp-DPhe-NMeVal). The term "inner salt" is used because the cyclic peptide contains two oppositely charged residues which intra-electronically counterbalance each other to form an overall noncharged molecule. One of the charged residues contains an acid moiety and the other charged residue contains an amino moiety. When the acid moiety and the amino moiety are in close proximity to one another, the acid moiety can be deprotonated by the amino moiety which forms a carboxylate/ammonium salt species with an overall neutral charge.

### iii. HCl treatment to give cyclo-(Arg-Gly-Asp-DPhe-NMeVal) x HCl

80 mg of cyclo-(Arg-Gly-Asp-DPhe-NMeVal) are dissolved in 0.01 M HCl five to six times and freeze dried after each dissolving operation. Subsequent purification by HPLC give cyclo-(Arg-Gly-Asp-DPhe-NMeVal) x HCl; FAB-MS (M+H): 589.

### iv. Methane sulfonic acid treatment to give cyclo-(Arg-Gly-Asp-DPhe-NMeVal) x MeSO₃H

80 mg of cyclo-(Arg-Gly-Asp-DPhe-NMeVal) are dissolved in 0.01 M MeSO₃H (methane sulfonic acid) five to six times and freeze dried after each dissolving operation. Subsequent purification by HPLC give cyclo-(Arg-Gly-Asp-DPhe-NMeVal) x MeSO₃H; RT = 17.8 ; FAB-MS (M+H): 589.

Alternative methods of cyclization include derivatizing the side group chains of an acyclic peptide precursor with sulfhydryl moieties, and when exposed to slightly higher than normal physiological pH conditions (pH 7.5), intramolecularly forms disulfide bonds with other sulfhydryl groups present in the molecule to form a cyclic peptide. Additionally, the C-terminus carboxylate moiety of an acyclic peptide precurosor can be reacted with a free sulfhydryl moiety present within the molecule for producing thioester cyclized peptides.

In inhibition of angiogenesis assays as described in Example 7 where the synthetic peptides were used, the 66203 peptide in HCl was slightly more effective in inhibiting angiogenesis than the identical peptide in TFA.

**Table 1**

| Peptide | | Amino Acid Sequence | SEO ID NO |
|---|---|---|---|
| Designation | | | |
| 62181 | | cyclo(GrGDFV) | 4 |
| 62184 | (66203*) | cyclo(RGDfV) | 5 |
| 62185 | (69601*) | cyclo (RADfV) | 6 |
| 62187 | | cyclo(RGDFv) | 7 |
| 62880 | | YTAECKPQVTRGDVF | 8 |
| 62186 | | cyclo(RaDFV) | 9 |
| 62175 | | cyclo(ARGDfL) | 10 |
| 62179 | | cyclo (GRGDfL) | 11 |
| 62411 | | TRQVVCDLGNPM | 12 |
| 62503 | | GVVRNNEALARLS | 13 |
| 62502 | | TDVNGDGRHDL | 14 |
| 121974 | (85189*) | cyclo (RGD-NH₂Me-V) | 15 |
| 112784 | | cyclo (RGEf-NH₂Me-V) | 16 |
| hump-2 | (410-631)** | | 17 |
| huMMP-2 | (439-631)** | | 18 |
| huMMP-2 | (439-512)** | | 19 |
| huMMP-2 | (439-546)** | | 20 |
| huMMP-2 | (510-631)** | | 21 |
| huMMP-2 | (543-631)** | | 22 |
| chMMP-2 | (410-637)*** | | 23 |
| chMMP-2 | (445-637)*** | | 24 |
| chMMP-2 | (445-518)*** | | 25 |
| chMMP-2 | (445-552)*** | | 26 |
| chMMP-2 | (516-637)*** | | 27 |
| chMMP-2 | (549-637)*** | | 28 |

| | | | |
|---|---|---|---|
| * The peptides designated with an asterisk are prepared in HCl and are identical in sequence to the peptide designated on the same line; the peptides without an asterisk are prepared in TFA. Lower case letters indicate a D-amino acid; capital letters indicate a L-amino acid. ** The human MMP-2 amino acid residue sequences for synthetic peptides are indicated by the corresponding residue positions shown in Figures 22A and 22B. (MMP-2 refers to a member of the family of matrix metalloproteinase enzymes). The human MMP-2 sequences are listed with the natural cysteine residues but are not listed with engineered cysteine residues as described for the fusion peptides. The non-natural cysteine residues were substituted for the natural amino acid residue at the indicated residue positions in order to facilitate solubility of the synthetic as well as expressed fusion proteins and to ensure proper folding for presentation of the binding site. *** The chicken MMP-2 amino acid residue sequences for synthetic peptides are indicated by the corresponding residue positions shown in Figures 22A and 22B. The chicken MMP-2 sequences are listed with the natural cysteine residues but not with the engineered cysteine residues as described for the fusion peptides as described above. | | | |

### 2. Monoclonal Antibodies (not part of the invention)

The monoclonal antibody LM609 secreted by hybridoma ATCC HB 9537 was produced using standard hybridoma methods by immunization with isolated αᵥβ₃ adsorbed onto Sepharose-lentil lectin beads. The αᵥβ₃ had been isolated from human melanoma cells designated M21, and antibody was produced as described by Cheresh et al., J. Biol. Chem., 262:17703-17711 (1987). M21 cells were provided by Dr. D. L. Morton (University of California at Los Angeles, CA) and grown in suspension cultures in RPMI 1640 culture medium containing 2 mM L-glutamine, 50 mg/ml gentamicin sulfate and 10% fetal calf serum.

Monoclonal antibody LM609 has been shown to specifically immunoreact with αᵥβ₃ complex, and not immunoreact with αᵥ subunit, with β₃ subunit, or with other integrins.

### 3. Characterization of the Tissue Distribution of αᵥβ₃ Expression

### A. Immunofluorescence with Anti-Integrin Receptor Antibodies

During wound healing, the basement membranes of blood vessels express several adhesive proteins, including von Willebrand factor, fibronectin, and fibrin. In addition, several members of the integrin family of adhesion receptors are expressed on the surface of cultured smooth muscle and endothelial cells. See, Cheresh, Proc. Natl. Acad. Sci.. USA, 84:6471 (1987); Janat et al., J. Cell Physiol., 151:588 (1992); and Cheng et al., J. Cell Physiol., 139:275 (1989). Among these integrins is αᵥβ₃, the endothelial cell receptor for von Willebrand factor, fibrinogen (fibrin), and fibronectin as described by Cheresh, Proc. Natl. Acad. Sci.. USA, 84:6471 (1987). This integrin initiates a calcium-dependent signaling pathway leading to endothelial cell migration, and therefore appears to play a fundamental role in vascular cell biology as described by Leavelsey et al., J. Cell Biol., 121:163 (1993).

To investigate the expression of αᵥβ₃ during angiogenesis, human wound granulation tissue or adjacent normal skin was obtained from consenting patients, washed with 1 ml of phosphate buffered saline and embedded in O.T.C medium (Tissue Tek). The embedded tissues were snap frozen in liquid nitrogen for approximately 30 to 45 seconds. Six micron thick sections were cut from the frozen blocks on a cryostat microtome for subsequent immunoperoxidase staining with antibodies specific for either β₃ integrins (αᵥβ₃ or α_{IIb}β₃) or the β₁ subfamily of integrins.

The results of the staining of normal human skin and wound granulation tissue are shown in Figures 1A-1D. Monoclonal antibodies AP3 and LM534, directed to β₃ and β₁ integrins, respectively, were used for immunohistochemical analysis of frozen sections. Experiments with tissue from four different human donors yielded identical results. The photomicrographs are shown at magnification of 300x.

The αᵥβ₃ integrin was abundantly expressed on blood vessels in granulation tissue (Figure 1B) but was not detectable in the dermis and epithelium of normal skin from the same donor (Figure 1A). In contrast, β₁ integrins were abundantly expressed on blood vessels and stromal cells in both normal skin (Figure 1C) and granulation tissue (Figure 1D) and, as previously shown as described by Adams et al., Cell, 63:425 (1991), on the basal cells within the epithelium.

### B. Immunofluorescence with Anti-Ligand Antibodies

Additional sections of the human normal skin and granulation tissues prepared above were also examined for the presence of the ligands for the β₃ and β₁ integrins, von Willebrand factor and laminin, respectively. Von Willebrand factor localized to the blood vessels in normal skin (Figure 2A) and granulation tissue (Figure 2B), whereas laminin localized to all blood vessels as well as the epithelial basement membrane in both tissue preparations (Figures 2C and 2D).

### C. Distribution of Anti-αᵥβ₃ Antibodies on Cancer Tissue

In addition to the above analyses, biopsies of cancer tissue from human patients were also examined for the presence and distribution of αᵥβ₃. The tissues were prepared as described in Example 1A with the exception that they were stained with monoclonal antibody LM609 prepared in Example 2 that is specific for the integrin receptor complex, αᵥβ₃. In addition, tumors were also prepared for microscopic histological analysis by fixing representative examples of tumors in Bulins Fixative for 8 hours and serial sections cut and H&E stained.

The results of immunoperoxidase staining of bladder, colon breast and lung cancer tissues are shown in Figures 3A-3D, respectively. αᵥβ₃ is abundantly expressed only on the blood vessels present in the four cancer biopsies analyzed and not on any other cells present in the tissue.

The results described herein thus show that the αᵥβ₃ integrin receptor is selectively expressed in specific tissue types, namely granulated, metastatic tissues and other tissues in which angiogenesis is occurring and not normal tissue where the formation of new blood vessels has stopped. These tissues therefore provide an ideal target for therapeutic aspects of this invention.

### 4. Identification of αᵥβ₃-Specific Synthetic Peptides Detected by Inhibition of Cell Attachment and by a Ligand-Receptor Binding Assay (not part of the invention)

### A. Inhibition of Cell Attachment

As one means to determine integrin receptor specificity of the antagonists of this invention, inhibition of cell attachment assays were performed as described below.

Briefly, CS-1 hamster melanoma cells lacking expression of αᵥβ₃ and αᵥβ₃ were first transfected with an plasmid for expressing the β₃ subunit as previously described by Filardo et al., J. Cell Biol., 130:441-450 (1995). Specificity of potential αᵥβ₃ antagonists was determined by the ability to block the binding of αᵥβ₃-expressing CS-1 cells to VN or laminin coated plates. As an example of a typical assay, the wells were first coated with 10 ug/ml substrate overnight. After rinsing and blocking with 1% heat-denatured BSA in PBS at room temperature for 30 minutes, peptide 85189 (SEQ ID NO 15) over a concentration range of 0.0001 uM to 100 uM, was separately mixed with CS-1 cells for applying to wells with a cell number of 50,000 cells/well. After a 10-15 minute incubation at 37C, the solution containing the cells and peptides was discarded. The number of attached cells was then determined following staining with 1% crystal violet. Cell associated crystal violet was eluted by the addition of 100 microliters (ul) of 10% acetic acid. Cell adhesion was quantified by measuring the optical density of the eluted crystal violet at a wave length of 600 nm.

Figure 21 shows the result of a typical assay with an αᵥβ₃ antagonist, here peptide 85189. No inhibition was detected with the peptide on laminin-coated surfaces. In contrast, complete inhibition of binding was obtained on VN-coated surfaces with a peptide concentration of 10 uM or greater, as shown with the dose-response curve.

Similar assays were performed with fusion proteins containing various regions of the MMP-2 protein. The MMP-2-derived polypeptides include regions of the C-terminus of MMP-2 active in the binding interaction with αᵥβ₃ and thereby capable of inhibiting MMP-2 activation and associated activities. These polypeptides are prepared' either as synthetic polypeptides having a sequence derived from the C-terminal domain of MMP-2 as described in Example 1 or as fusion proteins including all or a portion of the C-terminal domain of MMP-2, prepared as described below. MMP-2 C-terminal molecules are presented for both chicken and human specific sequences.

The chicken-derived MMP-2 C-terminal domain, also referred to as the hemopexin domain immediately contiguous with the hinge region, comprises the amino acid residues 445-637 of MMP-2. The complete nucleotide and encoded amino acid sequence of chicken MMP-2 is described below. The human MMP-2 nucleotide and encoded amino acid sequence is also described below. The C-terminal domain in the human MMP-2 that corresponds to the chicken region of 445-637 begin at amino acid residue 439 and ends with 631 due to six missing residues from the human sequence as shown in Figures 22A and 22B. Both human- and chicken-derived C-terminal MMP-2 synthetic peptides for use in practicing the methods of this invention are listed in Table 1. The amino acid residue sequences of the synthetic peptides are the same as those generated by the recombinant fusion protein counterparts but without the GST fusion component. The C-terminal MMP-2 fusion proteins derived from both chicken and human are prepared as described below.

A MMP-2 fusion protein is a chimeric polypeptide having a sequence of MMP-2 C-terminal domain or a portion thereof fused (operatively linked by covalent peptide bond) to a carrier (fusion) protein, such as glutathione sulfhydryl transferase (GST).

To amplify various regions of chicken and human MMP-2, primer sequences were designed based on the known respective cDNA sequences of chicken and human MMP-2. The complete top strand of the cDNA nucleotide sequence of unprocessed chicken MMP-2, also referred to as progelatinase, is shown in Figures 22A and 22B along with the deduced amino acid sequence shown on the second line (Aimes et al., Biochem. J., 300:729-736, 1994). The third and fourth lines of the figure respectively show the deduced amino acid sequence of human (Collier et al., J. Biol, Chem., 263:6579-6587 (1988)) and mouse MMP-2 (Reponen et al., J. Biol. Chem., 267:7856-7862 (1992)). Identical residues are indicated by dots while the differing residues are given by their one letter IUPAC lettering. Missing residues are indicated by a dash. The numbering of the amino acid residues starts from the first residue of the proenzyme, with the residues of the signal peptide being given negative numbers. The nucleotide sequence is numbered accordingly. The putative initation of translation (ATG) is marked with three forward arrowheads and the translation termination signal (TGA) is indicated by an asterisk. The amino terminal sequences for the chicken proenzyme and active enzyme are contained with diamonds and single arrowheads. The chicken progelatinase nucleotide and amino acid residue sequences are listed together as SEQ ID NO 29 while the encoded amino acid residue sequence is listed separately as SEQ ID NO 30.

Templates for generating amplified regions of chicken MMP-2 were either a cDNA encoding the full-length mature chicken MMP-2 polypeptide provided by Dr. J. P. Quigley of the State University of New York at Stoney Brook, New York or a cDNA generated from a total cellular RNA template derived by standard techniques from an excised sample of chicken chorioallantoic membrane tissue. For the latter, the cDNA was obtained with MuLV reverse transcriptase and a downstream primer specific for the 3'-terminal nucleotides, 5'ATTGAATTCTTCTACAGTTCA3' (SEQ ID NO 31), the 5' and 3' ends of which was respectively complementary to nucleotides 1932-1912 of the published chick MMP-2 sequence. Reverse transcriptase polymerase chain reaction (RT-PCR) was performed according to the specifications of the manufacturer for the GeneAmp RNA PCR Kit (Perkin Elmer). The primer was also engineered to contain an internal EcoRI restriction site.

From either of the above-described cDNA templates, a number of C-terminal regions of chicken MMP-2, each having the natural cysteine residue at position 637 at the carboxy terminus, were obtained by PCR with the 3' primer listed above (SEQ ID NO 31) paired with one of a number of 5' primers listed below. The amplified regions encoded the following MMP-2 fusion proteins, having sequences corresponding to the amino acid residue positions as shown in Figures 22A and 22B and also listed in SEQ ID NO 30: 1) 203-637; 2) 274-637; 3) 292-637; 4) 410-637; 5) 445-637. Upstream or 5' primers for amplifying each of the nucleotide regions for encoding the above-listed MMP-2 fusion proteins were designed to encode the polypeptide start sites 3' to an engineered, i.e., PCR-introduced, internal BamHI restriction site to allow for directional ligation into either pGEX-1λT or pGEX-3X expression vectors. The 5' primers included the following sequences, the 5' and 3' ends of which correspond to the indicated 5' and 3' nucleotide positions of the chicken MMP-2 sequence as shown in Figure 22A and 22B (the amino acid residue position start sites are also indicated for each primer): 1) Nucleotides 599-619, encoding a 203 start site 5'ATGGGATCCACTGCAAATTTC3' (SEQ ID NO 32); 2) Nucleotides 809-830, encoding a 274 start site 5'GCCGGATCCATGACCAGTGTA3' (SEQ ID NO 33); 3) Nucleotides 863-883, encoding a 292 start site 5'GTGGGATCCCTGAAGACTATG3' (SEQ ID NO 34); 4) Nucleotides 1217-1237, encoding a 410 start 5'AGGGGATCCTTAAGGGGATTC3' (SEQ ID NO 35); and 5) Nucleotides 1325-1345, encoding a 445 start site 5'CTCGGATCCTCTGCAAGCACG3' (SEQ ID NO 36).

The indicated nucleotide regions of the template cDNA were subsequently amplified for 35 cycles (annealing temperature 55C) according to the manufacturer's instructions for the Expand High Fidelity PCR System (Boehringer Mannheim). The resulting PCR products were gel-purified, digested with BamHI and EcoRI restriction enzymes, and repurified before ligation into either pGEX-1λT or pGEX-3X vector (Pharmacia Biotech, Uppsala, Sweden) which had been similarly digested as well as dephosphorylated prior to the ligation reaction. The choice of plasmid was based upon the required reading frame of the amplification product. Competent E. coli strain BSJ72 or BL21 cells were transformed with the separate constructs by heat shock. The resulting colonies were screened for incorporation of the respective MMP-2 fusion protein-encoding plasmid by PCR prior to dideoxy sequencing of positive clones to verify the integrity of the introduced coding sequence. In addition, verification of incorporation of plasmid was confirmed by expression of the appropriately-sized GST-MMP-2 fusion protein.

Purification of each of the recombinant GST-MMP-2 fusion proteins was performed using IPTG-induced log-phase cultures essentially as described by the manufacturer for the GST Gene Fusion System (Pharmacia Biotech). Briefly, recovered bacteria were lysed by sonication and incubated with detergent prior to clarification and immobilization of the recombinant protein on sepharose 4B-coupled glutathione (Pharmacia Biotech). After extensive washing, the immobilized fusion proteins were separately eluted from the affinity matrix with 10 mM reduced glutathione in 50 mM Tris-HCl, pH 8.0, and dialyzed extensively against PBS to remove residual glutathione prior to use.

Prior attempts to produce fusion proteins between chicken MMP-2 residues 445 and 637 that only had one encoded cysteine residue resulted in insoluble products. Therefore, in order to generate additional soluble MMP-2 fusion proteins derived from the C-terminal region that did not include an endogenous terminal cysteine residue as present in the previously-described fusion protein, nucleotide sequences were introduced into amplified MMP-2 regions to encode a cysteine residue if necessary depending on the particular fusion protein. A cysteine residue is naturally present in the chicken MMP-2 sequence at position 446 and at position 637. In the human sequence, these positions correspond respectively to 440 and 631. Therefore, fusion proteins were designed to contain engineered terminal cysteine residues at the amino- or carboxy-terminus of the chicken MMP-2 sequences of interest so as to provide for disulfide-bonding with the naturally occurring cysteine at the other terminus, as required by the construct.

Oligonucleotide primers were accordingly designed to allow for amplification of chicken MMP-2 C-terminal regions for expression of soluble MMP-2/GST fusion proteins. Amplified chicken MMP-2 C-terminal regions included those for encoding amino acid residue positions 445-518, 445-552, 516-637 and 549-637. For fusion proteins containing residue 517, the naturally encoded tyrosine residue was substituted for a cysteine to allow for disulfide bonding with either cysteine at residue position 446 or 637. For fusion proteins containing residue 551, the naturally encoded tryptophan residue was substituted for a cysteine to allow for disulfide bonding with either naturally encoded cysteine at residue position 446 or 637.

Briefly, the pGEX-3X plasmid construct encoding the recombinant GST/MMP-2(410-637) fusion protein prepared above was used as a template for amplification according to the manufacturer's protocol for the Expand High Fidelity PCR Kit (Boehringer Mannheim) utilizing a set of oligonucleotide primers whose design was based on the published chicken MMP-2 sequence (also shown in Figures 22A and 22B. One upstream primer, designed to encode a chicken MMP-2 protein start site at position 445 after an engineered internal BamHI endonuclease restriction site for insertion into the pGEX-3X GST vector, had the nucleotide sequence (5'CTCGGATCCTCTGCAAGCACG3' (SEQ ID NO 37)). The 5' and 3' ends of the primer respectively corresponded to positions 1325-1345 of the chicken MMP-2 sequence in the figure. Another upstream primer, designed to encode a chicken MMP-2 protein start site at position 516 after an engineered internal BamHI restriction site for insertion into the pGEX-1λT GST vector and to encode a cysteine residue at position 517, had the nucleotide sequence (5'GCAGGATCCGAGTGCTGGGTTTATAC3' (SEQ ID NO 38)). The 5' and 3' ends of the primer respectively corresponded to positions 1537-1562 of the chicken MMP-2 sequence. A third upstream primer, designed to encode a chicken MMP-2 protein start site at position 549 following an engineered internal EcoRI endonuclease restriction site for insertion into the pGEX-1λT GST vector and to encode a cysteine residue at position 551, had the nucleotide sequence (5'GCAGAATTCAACTGTGGCAGAAACAAG3' (SEQ ID NO 39)). The 5' and 3' ends of the primer respectively corresponded to positions 1639-1665 of the chicken MMP-2 sequence.

These upstream primers were separately used with one of the following downstream primers listed below to produce the above-described regions from the C-terminal domain of chicken MMP-2. A first downstream primer (antisense), designed to encode a chicken MMP-2 protein termination site at position 518, to encode a cysteine residue at position 517, and to contain an internal EcoRI endonuclease restriction site for insertion into a GST vector, had the nucleotide sequence (5'GTAGAATTCCAGCACTCATTTCCTGC3' (SEQ ID NO 40)). The 5' and 3' ends of the primer, written in the 5'-3' direction, were respectively complementary in part to positions 1562-1537 of the chicken MMP-2 sequence. A second downstream primer, designed to encode a chicken MMP-2 protein termination site at position 552, to encode a cysteine residue at position 551, and to contain an internal-EcoRI endonuclease restriction site for insertion into a GST vector, had the nucleotide sequence (5'TCTGAATTCTGCCACAGTTGAAGG3' (SEQ ID NO 41)). The 5' and 3' ends of the primer, written in the 5'-3' direction, were respectively complementary in part to positions 1666-1643 of the chicken MMP-2 sequence. A third downstream primer, designed to encode a chicken MMP-2 protein termination site at position 637 and to contain an internal EcoRI endonuclease restriction site for insertion into a GST vector, had the nucleotide sequence (5'ATTGAATTCTTCTACAGTTCA3' (SEQ ID NO 42)). The 5' and 3' ends of the primer, written in the 5'-3' direction, were respectively complementary in part to positions 1932-1912 of the chicken MMP-2 sequence.

The regions of the chicken MMP-2 carboxy terminus bounded by the above upstream and downstream primers, used in particular combinations to produce the fusion proteins containing at least one engineered cysteine residue as described above, were separately amplified for 30 cycles with an annealing temperature of 55C according to the manufacturer's instructions for the Expand High Fidelity PCR System (Boehringer Mannheim). The resulting amplification products were separately purified, digested with BamHI and or EcoRI restriction enzymes as necessary, and repurified before ligation into the appropriate GST fusion protein vector, either pGEX-3X or pGEX-1λT, as indicated above by the reading frame of the upstream oligonucleotide primer. For ligating the amplified MMP-2 products, the vectors were similarly digested as well as dephosphorylated prior to the ligation reaction. Competent E. coli strain BL21 cells were then separately transformed with the resultant MMP-2-containing vector constructs by heat shock. Resulting colonies were then screened for incorporation of the appropriate fusion protein-encoding plasmid by PCR and production of the appropriate sized GST-fusion protein prior to dideoxy sequencing of positive clones to verify the integrity of the introduced coding sequence. Purification of recombinant GST fusion proteins were then performed using IPTG-induced log-phase cultures essentially as described above for producing the other GST-MMP-2 fusion proteins.

The results of inhibition of cell attachment assays with various chicken MMP-2 proteins as well as with other peptides indicate that intact MMP-2, the fusion protein CTMMP-2(2-4) from residues 445-637 and peptide 66203 (SEQ ID NO 5) but not MMP-2 (1-445) and control peptide 69601 inhibited β₃-expressing CS-1 cell adhesion to vitronectin but not laminin, and thereby inhibited vitronectin receptor (αᵥβ₃) binding to vitronectin by interfering with normal αᵥβ₃ binding activity. Other tested CTMMP-2 fusion proteins 7-1 from residues 274-637, 10-1 from residues 292-637 and 4-3 from residues 274-400 had less affect on cell adhesion compared to 2-4.

In addition to the chicken MMP-2 GST-fusion proteins described above, two human MMP-2 GST fusion proteins were produced for expressing amino acid regions 203-631 and 439-631 of the mature human MMP-2 proenzyme polypeptide. The indicated regions correspond respectively to chicken MMP-2 regions 203-637 and 445-637. Human MMP-2-GST fusion proteins were produced by PCR as described above for the chicken MMP-2-GST fusion proteins utilizing a cDNA template that encoded the entire human MMP-2 open reading frame provided by Dr. W. G. Stetler-Stevenson at the National Cancer Institute, Bethesda, MD. Upstream 5' primer sequences were designed based upon the previously published sequence of human MMP-2 (Collier et al., J. Biol. Chem., 263:6579-6587 (1988) and to encode an introduced internal EcoRI restriction site to allow for insertion of the amplified products into the appropriate expression vector.

One upstream primer, designed to encode a human MMP-2 protein start site at position 203 after an engineered internal EcoRI endonuclease restriction site for insertion into the pGEX-1λT GST vector, had the nucleotide sequence (5'GATGAATTCTACTGCAAGTT3' (SEQ ID NO 43)). The 5' and 3' ends of the primer respectively corresponded to positions 685-704 of the human MMP-2 open reading frame sequence. Another upstream primer, designed to encode a human MMP-2 protein start site at position 439 after an engineered internal EcoRI restriction site for insertion into the pGEX-1λT GST vector, had the nucleotide sequence (5'CACTGAATTCATCTGCAAACA3' (SEQ ID NO 44)). The 5' and 3' ends of the primer respectively corresponded to positions 1392 and 1412 of the human MMP-2 open reading frame sequence.

Each of the above primers were used separately with a downstream primer, having 5' and 3' ends respectively complementary to bases 1998 and 1978 of the human MMP-2 sequence that ends distal to the MMP-2 open reading frame and directs protein termination after amino acid residue 631. The amplified products produced expressed fusion proteins containing human MMP-2 amino acid residues 203-631 (SEQ ID NO 45) and 439-631 (SEQ ID NO 18).

The resulting PCR products were purified, digested with EcoRI and repurified for ligation into a pGEX-1λT plasmid that was similarly digested and dephosphorylated prior to the ligation reaction. Cells were transformed as described above.

Other human MMP-2 fusion proteins containing amino acid residues 410-631 (SEQ ID NO 17), 439-512 (SEQ ID NO 19), 439-546 (SEQ ID NO 20), 510-631 (SEQ ID NO 21) and 543-631 (SEQ ID NO 22) are also prepared as described above for use in the methods of this invention.

### B. Ligand-Receptor Binding Assay

The synthetic peptides prepared in Example 1 along with the MMP-2 fusion proteins described above were further screened by measuring their ability to antagonize αᵥβ₃ and α_{IIb}β₃ receptor binding activity in purified ligand-receptor binding assays. The method for these binding studies has been described by Barbas et al., Proc. Natl. Acad. Sci.. USA. 90:10003-10007 (1993), Smith et al., J. Biol. Chem., 265:11008-11013 (1990), and Pfaff et al., J. Biol. Chem., 269:20233-20238 (1994), the disclosures of which are hereby incorporated by reference.

Herein described is a method of identifying antagonists in a ligand-receptor binding assay in which the receptor is immobilized to a solid support and the ligand and antagonist are soluble. Also described is a ligand-receptor binding assay in which the ligand is immobilized to a solid support and the receptor and antagonists are soluble.

Briefly, selected purified integrins were separately immobilized in Titertek microtiter wells at a coating concentration of 50 nanograms (ng) per well. The purification of the receptors used in the ligand-receptor binding assays are well known in the art and are readily obtainable with methods familiar to one of ordinary skill in the art. After incubation for 18 hours at 4C, nonspecific binding sites on the plate were blocked with 10 milligrams/milliliter (mg/ml) of bovine serum albumin (BSA) in Tris-buffered saline. For inhibition studies, various concentrations of selected peptides from Table 1 were tested for the ability to block the binding of ¹²⁵I-vitronectin or ¹²⁵I-fibrinogen to the integrin receptors, αᵥβ₃ and α_{IIb}β₃. Although these ligands exhibit optimal binding for a particular integrin, vitronectin for αᵥβ₃ and fibrinogen for α_{IIb}β₃. inhibition of binding studies using peptides to block the binding of fibrinogen to either receptor allowed for the accurate determination of the amount in micromoles (uM) of peptide necessary to half-maximally inhibit the binding of receptor to ligand. Radiolabeled ligands were used at concentrations of 1 nM and binding was challenged separately with unlabeled synthetic peptides.

Following a three hour incubation, free ligand was removed by washing and bound ligand was detected by gamma counting. The data from the assays where selected cyclic peptides listed in Table 1 were used to inhibit the binding of receptors and radiolabeled fibrinogen to separately immobilized αᵥβ₃ and α_{IIb}β₃ receptors were highly reproducible with the error between data points typically below 11%. The IC₅₀ data in micromoles (IC₅₀ uM) are expressed as the average of duplicate data points ± the standard deviation as shown in Table 2.

**Table 2**

| Peptide No. | αᵥβ₃ (IC₅₀ µM) | α_{IIb}β₃ (IC₅₀ µM) |
|---|---|---|
| 62181 | 1.96 ± 0.62 | 14.95 ± 7.84 |
| 62184 | 0.05 ± 0.001 | 0.525 ± 0.10 |
| 62185 | 0.885 ± 0.16 | 100 ± 0.001 |
| 62187 | 0.05 ± 0.001 | 0.26 ± 0.056 |
| 62186 | 57.45 ± 7.84 | 100 ± 0.001 |
| 62175 | 1.05 ± 0.07 | 0.63 ± 0.18 |
| 62179 | 0.395 ± .21 | 0.055 ± 0.007 |

Thus, the RGD-containing or RGD-derivatized cyclic peptides 62181, 62184, 62185 and 62187, each having one D- amino acid residue, exhibited preferential inhibition of fibrinogen binding to the αᵥβ₃ receptor as measured by the lower concentration of peptide required for half-maximal inhibition as compared to that for the α_{IIb}β₃ receptor. In contrast, the other RGD-containing or RGD-derivatized cyclic peptides, 62186, 62175 and 62179, were either not as effective in blocking fibrinogen binding to αᵥβ₃ or exhibited preferential inhibition of fibrinogen binding to α_{IIb}β₃ as compared to αᵥβ₃. These results are consistent with those recently published by Pfaff, et al., J. Biol. Chem., 269:20233-20238 (1994) in which the cyclic peptide RGDFV (wherein F indicates a D- amino acid residue) specifically inhibited binding of fibrinogen to the αᵥβ₃ integrin and not to the α_{IIb}β₃ or α₅β₁ integrins. Similar inhibition of binding assays were performed with linearized peptides having or lacking an RGD motif, the sequences of which were derived from the αᵥ receptor subunit, α_{IIb} receptor subunit or vitronectin ligand amino acid residue sequences. The sequences of the linear peptides, 62880 (VN-derived amino acid residues 35-49), 62411 (αᵥ-derived amino acid residues 676-687); 62503 (αᵥ-derived amino acid residues 655-667) and 62502 (α_{IIb}-derived amino acid residues 296-306), are listed in Table 1. Each of these peptides were used in separate assays to inhibit the binding of either vitronectin (VN) or fibrinogen (FG) to either (α_{IIb}β₃ or αᵥβ₃. The IC₅₀ data in micromoles (IC₅₀ uM) of an individual assay for each experiment is shown in Table 3.

**Table 3**

| Peptide No. | α_{IIb}β₃ IC₅₀ | (uM) | αᵥβ₃ IC₅₀ | (uM) |
|---|---|---|---|---|
| | FG | VN | FG | VN |
| 62880 | 4.2 | 0.98 | <0.1 | 0.5 |
| 62411 | >100 | >100 | >100 | >100 |
| 62503 | >100 | >100 | >100 | >100 |
| 62502 | 90 | 5 | >100 | >100 |

The results of inhibition of ligand binding assays to selected integrin receptors with linearized peptides show that only peptide 62880 was effective at inhibiting the half-maximal binding of either FG or VN to aᵥβ₃ as measured by the lower concentration of peptide required for half-maximal inhibition as compared to α_{IIb}β₃ receptor. None of the other linearized peptides were effective at blocking ligand binding to αᵥβ₃ although peptide 62502 was effective at blocking VN binding to α_{IIb}β₃.

In other ligand receptor binding assays performed as described above with the exception that detection of binding or inhibition thereof was with ELISA and peroxidase-conjugated goat anti-rabbit IgG, the ligands VN, MMP-2 and fibronectin at a range of 5 - 50 ng/well and listed in the order of effectiveness were shown to bind to immobilized αᵥβ₃ receptor while collagen did not. In addition, the ability of peptides to inhibit the binding of either MMP-2 or VN to immobilized αᵥβ₃ was assessed with peptides 69601 (SEQ ID NO 6) and 66203 (SEQ ID NO 5). Only peptide 66203 was effective at inhibiting the binding of either substrate to the αᵥβ₃ receptor while the control peptide 69601 failed to have an effect with either ligand.

Specificity of MMP-2 binding to integrin receptors was confirmed with a solid phase receptor binding assay in which iodinated MMP-2 was shown to bind to αᵥβ₃ and not to α_{IIb}β₃ that had been immobilized on a solid phase (300 bound cpm versus approximately 10 bound CPM). The ability of an MMP-2 derived peptide or fusion protein to inhibit the specific binding of MMP-2 to αᵥβ₃ was demonstrated in a comparable assay, the results of which are shown in Figure 23. The GST-CTMMP-2(445-637) (also referred to as CTMMP-2(2-4)) fusion protein prepared as described above, labeled GST-MAID, inhibited the binding of iodinated MMP-2 to αᵥβ₃ while GST alone had no effect with levels of bound CPM comparable to wells receiving no inhibitor at all (labeled NT). The MMP-2 fusion protein referred to as CTMMP-2(274-637), also referred to as CTMMP-2(10-1), failed to inhibit the binding of labeled MMP-2 to αᵥβ_{3.}

Specificity of receptor interaction with MMP-2-derived antagonists was confirmed with binding and inhibition of binding solid phase assays. CTMMP-2(2-4), labeled in Figure 24 as [125I] GST2-4, bound to αᵥβ₃ and not to α_{IIb}β₃ while CTMMP-2(10-1), labeled in Figure 24 as [125I]GST10-1, did not bind to either receptor in the in vitro solid phase assay. In addition, the binding of labeled GST2-4 was competed by unlabeled GST2-4.

Thus, the ligand-receptor assay described herein can be used to screen for both circular or linearized synthetic peptides that exhibit selective specificity for a particular integrin receptor, specifically αᵥβ₃, as used as vitronectin receptor (αᵥβ₃) antagonists in practicing this invention.

### 5. Characterization of the Untreated Chick Chorioallantoic Membrane (CAM)

### A. Preparation of the CAM

Angiogenesis can be induced on the chick chorioallantoic membrane (CAM) after normal embryonic angiogenesis has resulted in the formation of mature blood vessels. Angiogenesis has been shown to be induced in response to specific cytokines or tumor fragments as described by Leibovich et al., Nature, 329:630 (1987) and Ausprunk et al., Am. J. Pathol., 79:597 (1975). CAMs were prepared from chick embryos for subsequent induction of angiogenesis and inhibition thereof as described in Examples 6 and 7, respectively. Ten day old chick embryos were obtained from McIntyre Poultry (Lakeside, CA) and incubated at 37C with 60% humidity. A small hole was made through the shell at the end of the egg directly over the air sac with the use of a small crafts drill (Dremel, Division of Emerson Electric Co. Racine WI). A second hole was drilled on the broad side of the egg in a region devoid of embryonic blood vessels determined previously by candling the egg. Negative pressure was applied to the original hole, which resulted in the CAM (chorioallantoic membrane) pulling away from the shell membrane and creating a false air sac over the CAM. A 1.0 centimeter (cm) x 1.0 cm square window was cut through the shell over the dropped CAM with the use of a small model grinding wheel (Dremel). The small window allowed direct access to the underlying CAM.

The resultant CAM preparation was then either used at 6 days of embryogenesis, a stage marked by active neovascularization, without additional treatment to the CAM reflecting the model used for evaluating effects on embryonic neovascularization or used at 10 days of embryogenesis where angiogenesis has subsided. The latter preparation was thus used in this invention for inducing renewed angiogenesis in response to cytokine treatment or tumor contact as described in Example 6.

### B. Histology of the CAM

To analyze the microscopic structure of the chick embryo CAMs and/or human tumors that were resected from the chick embryos as described in Example 8, the CAMs and tumors were prepared for frozen sectioning as described in Example 3A. Six micron (um) thick sections were cut from the frozen blocks on a cryostat microtome for immunofluorescence analysis.

Figure 4 shows a typical photomicrograph of an area devoid of blood vessels in an untreated 10 day old CAM. As angiogenesis in the CAM system is subsiding by this stage of embryogenesis, the system is useful in this invention for stimulating the production of new vasculature from existing vessels from adjacent areas into areas of the CAM currently lacking any vessels.

### C. Integrin Profiled in the CAM Detected by Immunofluorescence

To view the tissue distribution of integrin receptors present in CAM tissues, 6 um frozen sections of both tumor tissue and chick embryo CAM tissues were fixed in acetone for 30 seconds and stained by immunofluorescence with 10 micrograms/milliliter (ug/ml) mAb CSAT, a monoclonal antibody specific for the β₁ integrin subunit as described by Buck et al., J. Cell Biol., 107:2351 (1988) and thus used for controls, or LM609 as prepared in Example 2. Primary staining was followed by staining with a 1:250 dilution of goat anti-mouse rhodamine labeled secondary antibody (Tago) to allow for the detection of the primary immunoreaction product. The sections were then analyzed with a Zeiss immunofluorescence compound microscope.

The results of the immunofluorescence analysis show that the mature blood vessels present in an untreated 10 day chick embryo expressed the integrin β₁ subunit (Figure 5A). In contrast, in a serial section of the tissue shown in Figure 5A, no immunoreactivity with LM609 was revealed (Figure 5B). Thus, the integrin αᵥβ₃ detected by the LM609 antibody was not actively being expressed by the mature blood vessels present in a 10 day old untreated chick embryo. As shown in the CAM model and in the following Examples, while the blood vessels are undergoing new growth in normal embryogenesis or induced by either cytokines or tumors, the blood vessels are expressing αᵥβ₃. However, following active neovascularization, once the vessels have stopped developing, the expression of αᵥβ₃ diminishes to levels not detectable by immunofluorescence analysis. This regulation of αᵥβ₃ expression in blood vessels undergoing angiogenesis as contrasted to the lack of expression in mature vessels provides for the unique ability of this invention to control and inhibit angiogenesis as shown in the following Examples using the CAM angiogenesis assay system.

In other profiles, the metalloproteinase MMP-2 and αᵥβ₃ colocalized on endothelial cells undergoing angiogenesis three days following bFGF induction in the 10 day old CAM model. MMP-2 was only minimally expressed on vessels that lacked the αᵥβ₃ receptor. In addition, MMP-2 colocalized with αᵥβ₃ on angiogenic M21-L tumor-associated blood vessels in vivo (tumors resulting from injection of M21-L human melanoma cells into the dermis of human skin grafts grown on SCID mice as described in Example 11) but not with preexisting non-tumor associated blood vessels. Similar results of the selective association of MMP-2 and αᵥβ₃ were also obtained with αᵥβ₃ bearing CS-1 melanoma tumors in the CAM model but not with CS-1 cells lacking αᵥβ₃.

### 6. CAM Angiogenesis Assay

### A. Angiogenesis Induced by Growth Factors

Angiogenesis has been shown to be induced by cytokines or growth factors as referenced in Example 5A. In the experiments described herein, angiogenesis in the CAM preparation described in Example 5 was induced by growth factors that were topically applied onto the CAM blood vessels as described herein.

Angiogenesis was induced by placing a 5 millimeter (mm) X 5 mm Whatman filter disk (Whatman Filter paper No.1) saturated with Hanks Balanced Salt Solution (HBSS, GIBCO, Grand Island, NY) or HBSS containing 150 nanograms/milliliter (ng/ml) recombinant basic fibroblast growth factor (bFGF) (Genzyme, Cambridge, MA) on the CAM of a 10-day chick embryo in a region devoid of blood vessels and the windows were latter sealed with tape. In other assays, 125 ng/ml bFGF was also effective at inducing blood vessel growth. For assays where inhibition of angiogenesis ws evaluated with intravenous injections of antagonists, angiogenesis was first induced with 1-2 ug/ml bFGF in fibroblast growth medium. Angiogenesis was monitored by photomicroscopy after 72 hours. CAMs were snap frozen, and 6 um cryostat sections were fixed with acetone and stained by immunofluorescence as described in Example 5C with 10 ug/ml of either anti-β₁ monoclonal antibody CSAT or LM609.

The immunofluorescence photomicrograph in Figure 5C shows enhanced expression of αᵥβ₃ during bFGF-induced angiogenesis on the chick CAM in contrast with the absence of αᵥβ₃ expression in an untreated chick CAM as shown in Figure 5B. αᵥβ₃ was readily detectable on many (75% to 80%) of the vessels on the bFGF-treated CAMs. In addition, the expression of integrin β₁ did not change from that seen in an untreated CAM as β₁ was also readily detectable on stimulated blood vessels.

The relative expression of αᵥβ₃ and β₁ integrins was then quantified during bFGF-induced angiogenesis by laser confocal image analysis of the CAM cryostat sections. The stained sections were then analyzed with a Zeiss laser confocal microscope. Twenty-five vessels stained with LM609 and 15 stained with CSAT (average size - 1200 sq mm², range 350 to 3,500 mm²) were selected from random fields and the average rhodamine fluorescence for each vessel per unit area was measured in arbitrary units by laser confocal image analysis. Data are expressed as the mean fluorescence intensity in arbitrary units of vessels ± standard error (SE).

The results plotted in Figure 6 show that staining of αᵥβ₃ was significantly enhanced (four times higher) on CAMs treated with bFGF as determined by the Wilcoxon Rank Sum Test (P<0.0001) whereas β₁ staining was not significantly different with bFGF treatment.

The CAM assay was further used to examine the effect of another potent angiogenesis inducer, tumor necrosis factor-alpha (TNFα), on the expression of β₁ and β₃ integrins. Filter disks impregnated with either bFGF or TNFα and placed on CAMs from 10 day embryos were found to promote local angiogenesis after 72 hours.

The results are shown in the photomicrographs of CAMs either untreated (Figure 7A), treated with bFGF (Figure 7B) or treated with TNFα (Figure 7C). Blood vessels are readily apparent in both the bFGF and TNFα treated preparations but are not present in the untreated CAM. Thus, the topical application of a growth factor/cytokine resulted in the induction of angiogenesis from mature vessels in an adjacent area into the area originally devoid of blood vessels. In view of the bFGF-induced blood vessels and concomitant expression of αᵥβ₃ as shown in Figure 5C, treatment of TNFα results in comparable activities.

These findings indicate that in both human and chick, blood vessels involved in angiogenesis show enhanced expression of αᵥβ₃. Consistent with this, expression of αᵥβ₃ on cultured endothelial cells can be induced by various cytokines in vitro as described by Janat et al., J. Cell Physiol., 151:588 (1992); Enenstein et al., Exp. Cell Res., 203:499 (1992) and Swerlick et al., J. Invest. Derm., 99:715 (1993).

The effect on growth-factor induced angiogenesis by antibody and peptide inhibitors is presented in Examples 7A and 7B.

### B. Embryonic Angiogenesis

The CAM preparation for evaluating the effect of angiogenesis inhibitors on the natural formation of embryonic neovasculature was the 6 day embryonic chick embryo as previously described. At this stage in development, the blood vessels are undergoing de novo growth and thus provides a useful system for determining if αᵥβ₃ participates in embryonic angiogenesis. The CAM system was prepared as described above with the exception that the assay was performed at embryonic day 6 rather than at day 10. The effect on embryonic angiogenesis by treatment with antibodies and peptides of this invention are presented in Example 7C.

### C. Angiogenesis Induced by Tumors

To investigate the role of αᵥβ₃ in tumor-induced angiogenesis, various αᵥβ₃-negative human melanoma and carcinoma fragments were used in the CAM assay that were previously grown and isolated from the CAM of 17-day chick embryo as described by Brooks et al., J. Cell Biol., 122:1351 (1993) and as described herein. The fragments induced extensive neovascularization in the presence of buffer alone.

Angiogenesis was induced in the CAM assay system by direct apposition of a tumor fragment on the CAM. Preparation of the chick embryo CAM was identical to the procedure described above. Instead of a filter paper disk, a 50 milligram (mg) to 55 mg in weight fragment of one of human melanoma tumor M21-L, human lung carcinoma tumor UCLAP-3, human pancreatic carcinoma cell line FG (Cheresh et al., Cell 58:945-953, 1989), or human laryngeal carcinoma cell line HEp3, all of which are αᵥβ₃ negative tumors, was placed on the CAM in an area originally devoid of blood vessels.

The M21-L human melanoma cell line, UCLAP-3 human lung carcinoma cell line, FG pancreatic carcinoma cell line, or HEp3 human laryngeal carcinoma cell line, all αᵥβ₃ negative, were used to grow the solid human tumors on the CAMs of chick embryos. A single cell suspension of 8 x 10⁶ M21-L, UCLAP-3, and FB or 5 x 10⁵ HEp3 cells was first applied to the CAMs in a total volume of 30 ul of sterile HBSS. The windows were sealed with tape and the embryos were incubated for 7 days to allow growth of human tumor lesions. At the end of 7 days, now a 17-day embryo, the tumors were resected from the CAMs and trimmed free of surrounding CAM tissue. The tumors were sliced into 50 mg to 55 mg tumor fragments for use in either angiogenesis or tumor growth assays. The tumor fragments were placed on a new set of 10 day chick embryo CAMs as described in Example 6A in an area devoid of blood vessels.

Tumors grown in vivo on the chick embryo CAMs were stained for αᵥβ₃ expression with mAb LM609 as described in Example 3A. No specific staining of tumor cells was observed indicating a lack of αᵥβ₃ expression.

These CAM tumor preparations were then subsequently treated as described in Examples 7D and 7E for measuring the effects of antibodies and peptides on tumor-induced angiogenesis. The CAM tumor preparations were also treated as described in Examples 8, 9, and 12 for measuring the effects of antibodies and peptides on regression of tumors and apoptosis of angiogenic blood vessels and vascular cells.

### 7: Inhibition of Angiogenesis as Measured in the CAM Assay

### A. Inhibition of Growth Factor-Induced Angiogenesis by Topical Application of Inhibitors

### 1) Treatment with Monoclonal Antibodies (not part of the invention)

To determine whether αᵥβ₃ plays an active role in angiogenesis, filter disks saturated with bFGF or TNFα were placed on CAMs then the monoclonal antibodies (also referred to as mAb), LM609 (specific for αᵥβ₃), CSAT (specific for β₁), or P3G2 or also P1F6 (both specific for αᵥβ₅) were added to the preparation.

Angiogenesis was induced on CAMs from 10 day chick embryos by filter disks saturated with bFGF. Disks were then treated with 50 ml HBSS containing 25 mg of mAb in a total volume of 25 ul of sterile HBSS at 0, 24, and 48 hours. At 72 hours, CAMs were harvested and placed in a 35 mm petri dish and washed once with 1 ml of phosphate buffered saline. The bottom side of the filter paper and CAM tissue was then analyzed under an Olympus stereo microscope, with two observers in a double-blind fashion. Angiogenesis inhibition was considered significant when CAMs exhibited >50% reduction in blood vessel infiltration of the CAM directly under the disk. Experiments were repeated four times per antibody, with 6 to 7 embryos per condition.

The results of the effects of mAb treatment on bFGF-induced angiogenesis is shown in Figures 8A-8B. An untreated CAM preparation devoid of blood vessels is shown in Figure 8A to provide a comparison with the bFGF-blood vessel induction shows in Figure 8B and effects thereon by the mAbs in Figures 8C-8E. About 75% of these CAMs treated with mAb LM609 exhibited >50% inhibition of angiogenesis as shown in Figure 8E, and many of these appeared devoid of vessel infiltration. In contrast, the buffer control (Figure 8A) and disks treated with mAbs CSAT (Figure 8C) and P3G2 (Figure 8D) consistently showed extensive vascularization.

Identical results were obtained when angiogenesis was induced with TNFα. To examine the effects of these same antibodies on preexisting mature blood vessels present from normal vessel development adjacent to the areas devoid of vessels, filter disks saturated with mAbs were placed on vascularized regions of CAMs from 10 day embryos that did not receive topical application of cytokine. None of the three mAbs affected preexisting vessels, as assessed by visualization under a stereo microscope. Thus, mAb LM609 selectively inhibited only new blood vessel growth and did not effect mature blood vessels present in adjacent areas. This same effect was seen with the application of synthetic peptides either applied topically or intravenously as described in Examples 7A2) and 7E2), respectively.

### 2) Treatment with synthetic Peptides (not part of the invention)

CAM assays were also performed with the synthetic peptides of this invention to determine the effect of cyclic and linearized peptides on growth factor induced angiogenesis. The peptides were prepared as described in Example 1 and 80 ug of peptide were presented in a total volume of 25 ul of sterile HBSS. The peptide solution was applied to the CAM preparation immediately and then again at 24 and 48 hrs. At 72 hours the filter paper and surrounding CAM tissue was dissected and viewed as described above.

Results from this assay revealed were similar to those shown in Figures 9A-9C as described in Example 7E2) where synthetic peptides were intravenously injected into tumor induced blood vessels. Here, with the control peptide, 62186, the bFGF-induced blood vessels remained undisturbed as shown in Figure 9A. In contrast when the cyclic RGD peptide, 62814, was applied to the filter, the formation of blood vessels was inhibited leaving the area devoid of new vasculature. This effect was similar in appearance to that shown in Figure 9B as described in Example 7E2) below. In addition, also as shown in Figure 9C for intravenously injected peptides, in areas in which mature blood vessels were present yet distant from the placement of the growth-factor saturated filter, no effect was seen with the topical treatment of synthetic peptides on these outlying vessels. The inhibitory activity of the peptides on angiogenesis thus is limited to the areas of angiogenesis induced by growth factors and does not effect adjacent preexisting mature vessels or result in any deleterious cytotoxicity to the surrounding area.

Similar assays are performed with the other synthetic peptides prepared in Example 1 and listed in Table 1.

### 3) Treatment with MMP-2 Peptide Fragments (not part of the invention)

To demonstrate the biological effects of MMP-2 peptide fragments on angiogenesis, CAM assays were performed as described above with the exception that angiogenesis was induced with filter discs saturated for 10 minutes with bFGF at a concentration of 1.0 ug/ml in HBS. The discs were then positioned on the CAM in an area that was reduced in the number of preexisting vessels. The C-terminal CTMMP-2(410-637) fusion protein, prepared as described above, or control GST receptor associated fusion protein (RAP) (1.5 ug in 30 ul of HBSS) was applied then topically to the filter disc once per day for a total of three days. At the end of the incubation period, the embryos were sacrificed and the filter disc and underlying CAM tissue was resected and analyzed for angiogenesis with a stereo microscope. Angiogenesis was quantified by counting the number of blood vessels branch points that occur within the confines of the filter discs. The branched blood vessels are considered to correspond primarily to new angiogenic sprouting blood vessels.

Quantification was performed in a double blind manner by at least two independent observers. The results are expressed as the Angiogenic Index where the angiogenic index is the number of branch points (bFGF stimulated) minus the number of branch points (control unstimulated) per filter disc. Experiments routinely had 6-10 embryos per condition.

The results of the CAM angiogenesis assay are shown in Figures 25A-D, 26 and 27. In Figure 25, a series of photographs divided into four figures, Figures 25A-D, illustrate the comparison of angiogenesis inhibited in the presence of the CTMMP-2 fusion protein (CTMMP-2(410-637) (Figures 25C-D) and not inhibited in the presence of control GST fusion protein (Figures 25A-B). Figures 26 and 27 are bar graphs illustrating the angiogenesis index of CAM angiogenesis assays with CTMMP-2, the same fusion protein as above, compared to controls (bFGF only or GST-RAP fusion protein). In Figure 27, the results of two separate experiments (#1 & #2) using CTMMP-2(410-637) fusion protein are shown.

These results demonstrated in all three figures indicate that a CTMMP-2 fusion protein or polypeptide containing a C-terminal domain of MMP-2 is a useful composition for inhibition of bFGF-mediated angiogenesis by inhibiting αᵥβ₃.

### B. Inhibition of Growth Factor-Induced Angiogenesis by Intravenous Application of Inhibitors

### 1) Treatment with Monoclonal Antibodies (not part of the invention)

The effect on growth factor-induced angiogenesis with monoclonal antibodies intravenously injected into the CAM preparation was also evaluated for use in this invention.

The preparation of the chick embryo CAMs for intravenous injections were essentially as described in Example 7A with some modifications. During the candling procedures prominent blood vessels were selected and marks were made on the egg shell to indicate their positions. The holes were drilled in the shell and the CAMs were dropped and bFGF saturated filter papers were placed on the CAMs as described above. The windows were sealed with sterile tape and the embryos were replaced in the incubator. Twenty four hours later, a second small window was carefully cut on the lateral side of the egg shell directly over prominent blood vessels selected previously. The outer egg shell was carefully removed leaving the embryonic membranes intact. The shell membrane was made transparent with a small drop of mineral oil (Perkin-Elmer Corp, Norwalk, CT) which allowed the blood vessels to be visualized easily. Purified sterile mAbs, or synthetic peptides, the latter of which are described below, were inoculated directly into the blood vessels once with a 30 gauge needle at a dose of 200 ug of IgG per embryo in a total volume of 100 ul of sterile PBS. The windows were sealed with tape and the embryos were allowed to incubate until 72 hours. The filter disks and surrounding CAM tissues were analyzed as described before.

To determine the localization of LM609 mAb in CAM tissues or in tumor tissues, as shown herein and in the following Examples, that were previously inoculated intravenously with LM609, the fixed sections were blocked with 2.5% BSA in HBSS for 1 hour at room temperature followed by staining with a 1:250 dilution of goat anti-mouse rhodamine labeled secondary antibody (Tago). The sections were then analyzed with a Zeiss immunofluorescence compound microscope.

The results of intravenous antibody treatment to the bFGF induced blood vessel CAM preparation are shown in Figures 10A-10C. In Figure 10A, angiogenesis induced as a result of bFGF treatment is shown. No change to the presence of bFGF induced vasculature was seen with intravenous exposure to mAb P3G2, an anti-αᵥβ₃ antibody, as shown in Figure 10B. In contrast, treatment of the bFGF induced angiogenesis CAM preparation with LM609, an anti-αᵥβ₃ antibody, resulted in the complete inhibition of growth of new vessels into the filter area as shown in Figure 10C. The inhibitory effect on angiogenesis is thus resulting from the inhibition of αᵥβ₃ receptor activity by the LM609 anti-αᵥβ₃-specific antibody. Since the blocking of the αᵥβ₅ does not inhibit the formation of neovasculature into the CAMs filter site, αᵥβ₅ thus is not essential as compared to αᵥβ₃ for growth of new vessels.

### 2) Treatment with Synthetic Peptides (not part of the invention)

For CAM preparations in which angiogenesis was induced with 1-2 ug/ml bFGF as previously described, synthetic peptides 69601 (control) and 66203 (SEQ ID NO 5) were separately intravenously injected into CAM preparations 18 hours after bFGF induction of angiogenesis. The preparations were maintained for an additional 36-40 hours after which time the number of branch points were determined as previously described.

The results are shown in Figure 28 where peptide 66203 completely inhibited bFGF-induced angiogenesis in contrast to the absence of inhibition with the control peptide.

In other assays, peptide 85189 (SEQ ID NO 15) was evaluated for inhibiting bFGF-induced angiogenesis in the CAM assay over a dosage range of 10 ug/embryo to 300 ug/embryo. The assay was performed as previously described. The results are shown in Figure 29 where the lowest effective dose was 30 ug with 100 and 300 ug nearly completely inhibiting angiogenesis.

In still further assays, peptide 85189 was compared to peptides 69601 and 66203 for anti-angiogenesis activity. The assay was performed as described above with the exception that 50 ug peptide were used. The results, plotted in Figure 30, showed that peptides 66203 (labeled 203) and 85189 (labeled 189) were effective inhibitors of bFGF-mediated angiogenesis compared to bFGF-treated (labeled bFGF) and 69601-treated (labeled 601) controls.

The effectiveness of the different salt formulations of peptide 85189 was also evaluated in similar bFGF-induced CAM assays. The peptides were used at 100 ug/embryo. The same peptide sequence in HCl (peptide 85189) and in TFA (peptide 121974) inhibited bFGF-induced angiogenesis with the HCl formulated peptide being slightly more effective than that in TFA (the respective number of branch points for peptide 85189 versus 121974 is 30 versus 60). Untreated CAMs, labeled as "no cytokine" had approximately half as many branch points as that seen with bFGF treatment, respectively 70 versus 190. Treatment with control peptide 69601 had no effect on inhibiting angiogenesis (230 branch points).

The other synthetic peptides prepared in Example 1 are separately intravenously injected into the growth factor induced blood vessels in the CAM preparation as described above. The effect of the peptides on the viability of the vessels is similarly assessed.

### 3) Treatment with MMP-2 Fragment (not part of the invention)

With the above-described protocol, the effect of MMP-2 fusion proteins, CTMMP-2(2-4), also referred to as CTMMP-2(445-637) and CTMMP-2(10-1), also referred to as CTMMP-2(274-637) was also evaluated. The assay was performed as previously described with the exception that 50 ug of fusion protein was administered to the bFGF-treated embryos. The effect of fusion protein treatment was assessed at 24 hours, 48 hours and 72 hours.

The results are shown for these selected time periods in Figures 31A-L where angiogenesis was photographically assessed under assay conditions of no treatment, bFGF treatment, bFGF treatment followed by CTMMP-2(2-4), labeled as bFGF + MAID (MAID = MMP-2 angiogenesis inhibiting domain), and bFGF treatment followed by CTMMP-2(10-1), labeled as bFGF + Control. The significant induction of angiogenesis after 48 and 72 hours following bFGF treatment was almost completely inhibited only with exposure to CTMMP-2(2-4). The extent of inhibition with CTMMP-2(2-4) was greater than that seen with CTMMP-2(10-1) which exhibited some in vivo anti-angiogenesis activity.

The other MMP-2 compositions, whole MMP-2, fragments and fusion proteins, prepared as previously described are also separately intravenously injected into the growth factor induced blood vessels in the CAM preparation as described above. The effect of the peptides on the viability of the vessels is similarly assessed.

### C. Inhibition of Embryonic Angiogenesis by Topical Application

### 1) Treatment with Monoclonal Antibodies (not part of the invention)

To determine whether αᵥβ₃ participates in embryonic angiogenesis, the effect of LM609 on de novo growth of blood vessels on CAMs was examined in 6 day embryos, a stage marked by active neovascularization as described in Example 5A. The CAM assay was prepared as described in Example 6C with the subsequent topical application of disks saturated with mAbs placed on CAMs of 6 day old embryos in the absence of cytokines. After 3 days, CAMS were resected and photographed. Each experiment included 6 embryos per group and was repeated 2 times.

Antibody LM609 (Figure 11C), but not CSAT (Figure 11A) or P3G2 (Figure 11B), prevented vascular growth under these conditions; this indicates that αᵥβ₃ plays a substantial role in embryonic neovascularization that was independent of added growth factors for induction of angiogenesis.

### 2) Treatment with Synthetic Peptides (not part of the invention)

The synthetic peptides prepared in Example 1 are separately added to the embryonic CAM preparation prepared above and as described in Example 5A2) by either topical application to the CAM or intravenous application to blood vessels. The effect of the peptides on the viability of the vessels is similarly assessed.

### D. Inhibition of Tumor-Induced Angiogenesis by Topical Application

### 1) Treatment with Monoclonal_ Antibodies (not part of the invention)

In addition to the angiogenesis assays described above where the effects of anti-αᵥβ₃ antagonists, LM609 and various peptides, on embryonic angiogenesis were evaluated, the role of αᵥβ₃ in tumor-induced angiogenesis was also investigated. As an inducer, αᵥβ₃-negative human M21-L melanoma fragments previously grown and isolated from the CAM of a 17-day chick embryo were used. The fragments were prepared as described in Example 6C.

As described above in Example 7A1), mAbs were separately topically applied to the tumor fragments at a concentration of 25 ug in 25 ul of HBSS and the windows were then sealed with tape. The mAbs were added again in the same fashion at 24 hours and 48 hours. At 72 hours, the tumors and surrounding CAM tissues were analyzed as described above in Example 7A1).

As described in Example 6C, tumors were initially derived by transplanting cultured M21-L cells, which do not to express integrin αᵥβ₃ as described by Felding-Habermann et al., J. Clin. Invest., 89:2018 (1992) onto the CAMs of 10-day old chick embryos. These αᵥβ₃-negative fragments induced extensive neovascularization in the presence of buffer alone, or mAbs CSAT (anti-β₁) or P3G2 (anti-αᵥβ₃). In contrast, mAb LM609 (anti-αᵥβ₃) abolished the infiltration of most vessels into the tumor mass and surrounding CAM.

In order to quantitate the effect of the mAbs on the tumor-induced angiogenesis, blood vessels entering the tumor within the focal plane of the CAM were counted under a stereo microscope by two observers in a double-blind fashion. Each data bar presented in Figure 12 represents the mean number of vessels ± SE from 12 CAMs in each group representing duplicate experiments.

This quantitative analysis revealed a three-fold reduction in the number of vessels entering tumors treated with mAb LM609 compared to tumors treated with buffer or the other mAbs, P3G2 or CSAT (P< 0.0001) as determined by Wilcoxon Rank.Sum Test. The fact that M21-L tumors do not express αᵥβ₃ indicates that mAb LM609 inhibits angiogenesis by directly affecting blood vessels rather than the tumor cells. These results correspond with the histological distribution of αᵥβ₃ in cancer tissue biopsies shown in Figure 3A-3D where the distribution of αᵥβ₃ was limited to the blood vessels in the tumor and not to the tumor cells themselves.

### 2) Treatment with synthetic Peptides (not part of the invention)

The synthetic peptides prepared in Example 1, including MMP-2-derived peptides and fusion proteins are topically applied to the tumor-induced angiogenic CAM assay system as described above. The effect of the peptides on the viability of the vessels is similarly assessed.

### E. Inhibition of Tumor-Induced Angiogenesis by Intravenous Application

### 1) Treatment with Monoclonal Antibodies (not part of the invention)

Tumor-induced blood vessels prepared as described in Example 7E1) were also treated with mAbs applied by intravenous injection. Tumors were placed on the CAMs as described in Example 7D1) and the windows sealed with tape and 24 hours latter, 200 ug of purified mAbs were inoculated once intravenously in chick embryo blood vessels as described previously. The chick embryos were then allowed to incubate for 7 days. The extent of angiogenesis was then observed as described in above. As described in Example 8 below, after this time period, the tumors were resected and analyzed by their weight to determine the effect of antibody exposure on tumor growth or suppression.

### 2) Treatment with Synthetic Peptides (not part of the invention)

The effects of peptide exposure to tumor-induced vasculature in the CAM assay system was also assessed. The tumor-CAM preparation was used as described above with the exception that instead of intravenous injection of a mAb, synthetic peptides prepared as described in Example 1 and Example 7A2) were separately intravenously injected into visible blood vessels.

The results of CAM assays with the cyclic peptide, 66203 containing the HCl salt, and control peptide, 62186, are shown in Figures 9A-9C. In Figure 9A, the treatment with the control peptide did not effect the abundant large blood vessels that were induced by the tumor treatment to grow into an area originally devoid of blood vessels of the CAM. In contrast when the cyclic RGD peptide, 66203, an antagonist to αᵥβ₃, was applied to the filter, the formation of blood vessels was inhibited leaving the area devoid of new vasculature as shown in Figure 9B. The inhibitory effect of the RGD-containing peptide was specific and localized as evidenced by an absence of any deleterious effects to vessels located adjacent to the tumor placement. Thus, in Figure 9C, when inhibitory peptides are intravenously injected into the CAM assay system, no effect was seen on the preexisting mature vessels present in the CAM in areas adjacent yet distant from the placement of the tumor. The preexisting vessels in this location were not affected by the inhibitory peptide that flowed within those vessels although the generation of new vessels from these preexisting vessels into the tumor mass was inhibited. Thus, synthetic peptides including 66203 and 62184, previously shown in ligand-receptor assays in Example 4 to be antagonists of αᵥβ₃, have now been demonstrated to inhibit angiogenesis that is limited to vessels undergoing development and not to mature preexisting vessels. In addition, the intravenous infusion of peptides does not result in any deleterious cytotoxicity to the surrounding area as evidence by the intact vasculature in Figure 9C.

Similar assays are performed with the other synthetic peptides prepared in Example 1 and listed in Table 1 along with the MMP-2 compositions of this invention.

### 3) Treatment with MMP-2 Fragments (not part of the invention)

A CS-1 tumor (β₃-negative) was prepared in a CAM as described above. After 24 hours of tumor growth, a composition of MMP-2 fragment, designated CTMMP-2(2-4) and prepared as described in Example 4A, was administered intraveneously at 50 ug fragment in 100 ul of PBS. After 6 days, the tumor was evaluated for mass. Tumors treated with CTMMP-2(2-4) were reduced in growth rate by about 50% when compared to the growth rate of control tumors treated with CTMMP-2(10-1) or with PBS control. Thus, the αᵥβ₃ antagonist inhibited tumor growth.

### 8. Inhibition of Tumor Tissue Growth With αᵥβ₃ Antagonists As Measured in the CAM Assay (not part of the invention)

As described in Example 7E1), in addition to visually assessing the effect of anti-αᵥβ₃ antagonists on growth factor or tumor induced angiogenesis, the effect of the antagonists was also assessed by measuring any changes to the tumor mass following exposure. For this analysis, the tumor-induced angiogenesis CAM assay system was prepared as described in Example 6C and 7D. At the end of the 7 day incubation period, the resulting tumors were resected from the CAMs and trimmed free of any residual CAM tissue, washed with 1 ml of phosphate buffer saline and wet weights were determined for each tumor.

In addition, preparation of the tumor for microscopic histological analysis included fixing representative examples of tumors in Bulins Fixative for 8 hours and embedding in paraffin. Serial sections were cut and stained with hematoxylin and eosin (H&E) for microscopic analysis. Gladson, et al., J. Clin. Invest., 88:1924 (1991). Sections were photographed with an Olympus compound microscope at 250x.

### A. Topical Application

The results of typical human melanoma tumor (M21L) weights resulting from topical application of control buffer (HBSS), P3G2 (anti-αᵥβ₅) or LM609 (anti-αᵥβ₃) are listed in Table 4. A number of embryos were evaluated for each treatment with the average tumor weight in milligrams (mg) from each being calculated along with the SE of the mean as shown at the bottom of the table.

**Table 4**

| Embryo No. | mAb Treatment | Tumor Weight (mg) |
|---|---|---|
| 1 | HBSS | 108 |
| 2 | | 152 |
| 3 | | 216 |
| 4 | | 270 |
| 5 | | 109 |
| 6 | | 174 |
| | | |
| 1 | P3G2 | 134 |
| 2 | | 144 |
| 3 | | 408 |
| 4 | | 157 |
| 5 | | 198 |
| 6 | | 102 |
| 7 | | 124 |
| 8 | | 99 |
| | | |
| 1 | LM609 | 24 |
| 2 | | 135 |
| 3 | | 17 |
| 4 | | 27 |
| 5 | | 35 |
| 6 | | 68 |
| 7 | | 48 |
| 8 | | 59 |

| mAb Treatment | Average Tumor Weight (mg) |
|---|---|
| HBSS control | 172 ± 26 |
| P3G2 | 171 ± 36 |
| LM609 | 52 ± 13 |

Exposure of a αᵥβ₃-negative human melanoma tumor mass in the CAM assay system to LM609 caused the decrease of the untreated average tumor weight of 172 mg ± 26 to 52 mg ± 13. The P3G2 antibody had no effect on the tumor mass. Thus, the blocking of the αᵥβ₃ receptor by the topical application of αᵥβ₃-specific LM609 antibody resulted in a regression of tumor mass along with an inhibition of angiogenesis as shown in the preceding Examples. The measured diameter of the tumor mass resulting from exposure to P3G2 was approximately 8 millimeters to 1 centimeter on average. In contrast, the LM609-treated tumors were on average 2 to 3 millimeters in diameter.

Frozen sections of these tumors revealed an intact tumor cytoarchitecture for the tumor exposed to P3G2 in contrast to a lack or organized cellular structure in the tumor exposed to LM609. αᵥβ₃ receptor activity is therefore essential for an αᵥβ₃ negative tumor to maintain its mass nourished by development of αᵥβ₃-expressing neovasculature. The blocking of αᵥβ₃ with the αᵥβ₃ antagonists of this invention results in the inhibition of angiogenesis into the tumor ultimately resulting in the diminution of tumor mass.

### B. Intravenous Application

The results of typical carcinoma tumor (UCLAP-3) weights resulting from intravenous application of control buffer (PBS, phosphate buffered saline), CSAT (anti-β₁) or LM609 (anti-αᵥβ₃) are listed in Table 5. A number of embryos were evaluated for each treatment with the average tumor weight from each being calculated along with the SE of the mean as shown at the bottom of the table.

**Table 5**

| Embryo No. | mAb Treatment | Tumor Weight (mg) |
|---|---|---|
| 1 | PBS | 101 |
| 2 | | 80 |
| 3 | | 67 |
| 4 | | 90 |
| | | |
| 1 | CSAT | 151 |
| 2 | | 92 |
| 3 | | 168 |
| 4 | | 61 |
| 5 | | 70 |
| | | |
| 1 | LM609 | 16 |
| 2 | | 54 |
| 3 | | 30 |
| 4 | | 20 |
| 5 | | 37 |
| 6 | | 39 |
| 7 | | 12 |

| mAb Treatment | Average Tumor Weight (mg) |
|---|---|
| PBS control | 85 ± 7 |
| CSAT | 108 ± 22 |
| LM609 | 30 ± 6 |

Exposure of a αᵥβ₃-negative human carcinoma tumor mass in the CAM assay system to LM609 caused the decrease of the untreated average tumor weight of 85 mg t 7 to 30 mg ± 6. The CSAT antibody did not significantly effect the weight of the tumor mass. Thus, the blocking of the αᵥβ₃ receptor by the intravenous application of αᵥβ₃-specific LM609 antibody resulted in a regression of a carcinoma as it did for the melanoma tumor mass above along with an inhibition of angiogenesis as shown in the preceding Examples. In addition, human melanoma tumor growth was similarly inhibited by intravenous injection of LM609.

### 9. Regression of Tumor Tissue Growth With αᵥβ₃ Antagonists As Measured in the CAM Assay

To further assess the effects of αᵥβ₃ antagonists on tumor growth and survival, fragments of human melanoma and fragments of carcinomas of the lung, pancreas, and larynx were placed on CAMS of 10-day old embryos as described in Example 5A.

### A. Intravenous Application

### 1) Treatment with Monoclonal Antibodies

### a. Treatment with LM609 (Anti-αᵥβ₃) and CSAT (Anti-β₁) (not part of the invention)

Twenty four hours after implantation of CAM with carcinoma fragments of αᵥβ₃-negative human melanoma M21-L, pancreatic carcinoma FG, human lung carcinoma UCLAP-3, or human laryngeal carcinoma HEp3, embryos were injected intravenously with PBS alone or a single dose (300 ug/100 ul) of either mAb LM609 (anti-αᵥβ₃) or CSAT (anti-β₃). Tumors were allowed to propagate for six additional days. At the end of the incubation period the tumors were carefully resected and trimmed free of surrounding CAM tissue. Tumor resections were performed by two independent investigators removing only the easily definable solid tumor mass. The tumors had well defined margins, thus the thin semi-transparent membrane (CAM) which is readily distinguishable from the solid tumor mass was removed without disturbing the tumor mass itself. The resected tumors were weighed and examined morphologically and histologically.

As shown in Figure 13, wet tumor weights at the end of 7 days were determined and compared to initial tumor weights before treatments. Each bar represents the mean ± S.E. of 5-10 tumors per group. mAb LM609 inhibited tumor growth significantly (p <0.001) as compared to controls in all tumors tested. Tumors treated with PBS or CSAT proliferated in all cases. In contrast, mAb LM609 not only prevented the growth of these tumors but induced extensive regression in most cases. Importantly, these tumor cells do not express integrin αᵥβ₃ demonstrating that the inhibition of growth was due to the anti-angiogenic effects of this antibody on neovasculature rather than upon the tumor cells directly.

### b. Treatment with LM609 (Anti-αᵥβ₃) and P3G2 (Anti-αᵥβ₅) (not part of the invention)

Human M21-L melanoma tumor fragments (50 mg) were implanted on the CAMs of 10 day old embryos as described in Example 5A. Twenty four hours later, embryos were injected intravenously with PBS alone or a single dose (300 ug/100 ul) of either mAb LM609 (anti-αᵥβ₃) or P3G2 (anti-αᵥβ₅). Tumors were allowed to propagate as described in Example 9A1)a above and were examined morphologically and histologically as herein described.

Representative examples of M21-L tumors treated with mAbs P3G2 (anti-αᵥβ₅) or LM609 (anti-αᵥβ₃) were examined morphologically. The P3G2-treated tumors were large (8 mm in diameter) and well vascularized whereas those treated with mAb LM609 were much smaller (3 mm in diameter) and lacked detectable blood vessels.

The tumors were further examined by the preparation of histological sections and staining with hematoxylin and eosin as described in Example 9A1)a. As shown in Figure 14 (upper panel), tumors treated with mAb P3G2 (anti-αᵥβ₅) showed numerous viable and actively dividing tumor cells as indicated by mitotic figures (arrowheads) as well as by multiple blood vessels (arrows) throughout the tumor stroma. In contrast, few if any viable tumor cells or blood vessels were detected in tumors treated with mAb LM609 (anti-αᵥβ₃) (Figure 14, lower panel). These results demonstrate that antagonists of integrin αᵥβ₃ inhibit tumor-induced angiogenesis leading to the growth arrest and regression of a variety of human tumors in vivo. It is important to point out that embryos examined after seven days of tumor growth (embryonic day 17) appeared normal upon gross examination whether or not they were treated with an αᵥβ₃ antagonist. These findings indicate that antagonists of this integrin appear non-toxic to the developing embryos.

### 2) Treatment With Synthetic Peptides (not part of the invention)

Human M21-L melanoma tumor fragments (50 mg) were implanted on the CAMs of 10 day oldembryos as described in Example 5A. Twenty four hours later, embryos received a single intravenous injection of 300 ug/100 ul of either the cyclo-RADfV (69601) and or cyclo-RGDfV (66203). After a total of 72 hours, tumors were removed, examined morphologically, and photographed with a stereo microscope as described in Example 9A1).

The panels shown in Figures 15A through 15E correspond as follows: Figure 15A, duplicate samples treated with cyclo-RADfV peptide (69601); Figure 15B, duplicate samples treated with cyclo-RGDfV peptide (66203); Figure 15C, adjacent CAM tissue taken from the same embryos treated with cyclo-RGDfV peptide (66203) and Figures 15D and 15E, high magnification (13x) of peptide treated tumors. Figure 15D depicts normal blood vessels from control peptide (69601) treated tumor. Figure 15E depicts examples of disrupted blood vessels from cyclo-RGDfV peptide (66203) treated tumors (arrows).

The results illustrate that only peptide 66203 in contrast to control peptide 69601 inhibited vessel formation, and further that vessels in the CAM tissue adjacent to the tumor were not affected.

Additional tumor regression assays were performed with the αᵥβ₃-reactive peptide 85189 (SEQ ID NO 15) against 69601 as a control. The assays were performed as described above with the exception that 100 ug of peptide was intravenously injected into the CAM at 18 hourst postimplantation. After 48 hours more, the tumors were then resected and wet weights were obtained.

Figures 32, 33 and 34 respectively show the reduction in tumor weight for UCLAP-3, M21-L and FgM tumors following intravenous exposure to peptide 85189 in contrast to the lack of effect with either PBS or peptide 69601.

### 10. Regression of Tumor Tissue Growth With αᵥβ₃ Antagonists as Measured by In Vivo Rabbit Eye Model Assay

The effect of anti-αᵥβ₃ antagonists on growth factor-induced angiogenesis can be observed in naturally transparent structures as exemplified by the cornea of the eye. New blood vessels grow from the rim of the cornea, which has a rich blood supply, toward the center of the cornea, which normally does not have a blood supply. Stimulators of angiogenesis, such as bFGF, when applied to the cornea induce the growth of new blood vessels from the rim of the cornea. Antagonists of angiogenesis, applied to the cornea, inhibit the growth of new blood vessels from the rim of the cornea. Thus, the cornea undergoes angiogenesis through an invasion of endothelial cells from the rim of the cornea into the tough collagen-packed corneal tissue which is easily visible. The rabbit eye model assay therefore provides an in vivo model for the direct observation of stimulation and inhibition of angiogenesis following the implantation of compounds directly into the cornea of the eye.

### A. In Vivo Rabbit Eye Model Assay

### 1) Angiogenesis Induced by Growth Factors

Angiogenesis was induced in the in vivo rabbit eye model assay with the growth factor bFGF and is described in the following sections.

### a. Preparation of Hydron Pellets Containing

### Growth Factor and Monoclonal Antibodies

Hydron polymer pellets containing growth factor and mAbs were prepared as described by D'Amato, et al., Proc. Natl. Acid. Sci., USA, 91:4082-4085 (1994). The individual pellets contained 650 ng of the growth factor bFGF bound to sucralfate (Carafet, Marion Merrell Dow Corporation) to stabilize the bFGF and ensure its slow release into the surrounding tissue. In addition, hydron pellets were prepared which contained either 40 ug of the mAb LM609 (anti-αᵥβ₃) or mAb P1F6 (anti-αᵥβ₅) in PBS. The pellets were cast in specially prepared Teflon pegs that have a 2.5 mm core drilled into their surfaces. Approximately 12 ul of casting material was placed into each peg and polymerized overnight in a sterile hood. Pellets were then sterilized by ultraviolet irradiation.

### b. Treatment with Monoclonal Antibodies (not part of the invention)

Each experiment consisted of three rabbits in which one eye received a pellet comprising bFGF and LM609 and the other eye received a pellet comprising bFGF and a mouse mAb P1F6 (anti-αᵥβ₅). The use of paired eye testing to compare LM609 (anti-αᵥβ₃) to other mAb and PBS controls provides a means for rigorous testing to demonstrate significant differences between the mAbs tested.

The P1F6 mAb immunoreacts with the integrin αᵥβ₅ which is found on the surface of vascular endothelial cells but is presumably not involved in angiogenesis. To determine whether the mAb P1F6 was involved in angiogenesis, pellets containing only this mAb were prepared and assayed as described below to confirm that the mAb did not induce angiogenesis.

All of the mAbs tested were purified from ascites fluid using Protein-A Sepharose CL-4B affinity column chromatography according to well-known methods. The eluted immunoglobulin was then dialyzed against PBS and treated with Detoxi-gel (Pierce Chemicals) to remove endotoxin. Endotoxin has been shown to be a potent angiogenic and inflammatory stimulant. mAbs were therefore tested for the presence of endotoxin with the Chromogenic Limulus Amebocyte Lysate Assay (Bio-Whittaker) and only those mAbs without detectable endotoxin were used in the rabbit eye model assay.

A hydron pellet comprising bFGF and mAb LM609 (anti-αᵥβ₃) or P1F6 (anti-αᵥβ₅) was inserted into a corneal pocket formed in the eye of rabbits. The hydron pellet also contained sucralfate to stabilize the bFGF during the assay. Individual pellets were implanted into surgically created "pockets" formed in the mid-stroma of the cornea of rabbits. The surgical procedure was done under sterile technique using a Wild model M691 operating microscope equipped with a beamsplitter to which was mounted a camera for photographically recording individual corneas. A 3 mm by 5 mm "pocket" was created in the corneal stroma by making a 3 mm incision to half the corneal thickness with a 69 Beaver blade. The stroma was dissected peripherally using an iris spatula and the pellet was implanted with its peripheral margin 2 mm from the limbus.

During the following 14 days, bFGF and mAb diffused from the implanted pellet into the surrounding tissue and thereby effected angiogenesis from the rim of the cornea.

Representative results of each treatment are depicted in Figures 16A through 16E. The amount of vessels present are quantitated and described in terms of clock hours which are defined as follows. The eye is divided into 12 equal sections in the same manner as a clock is divided into hours. "One clock hour of vessels" refers to that amount of vessels which fills an area of the eye equivalent to one hour on a clock. The five rabbits which received only bFGF exhibited florid angiogenesis in which new blood vessels had grown from the rim of the cornea toward the center of the cornea, which normally does not have blood vessels. One of these rabbits had only 1 clock hour of vessels to the pellet. Two of the rabbits which received both bFGF and mAb LM609 had absolutely no detectable angiogenesis up to 14 days following surgery. One of these rabbits had 3 foci of hemorrhagic and budding vessels by day 14. Two of the rabbits which received bFGF and mAb P3G2 (anti-αᵥβ₅) showed extensive vascularization in which new blood vessels had grown from the rim of the cornea into the cornea. One of these rabbits had only 1 to 2 hours of vessels to the pellet.

As evidenced in the rabbit eye model assay, no angiogenic effect was observed on normal paralimbal vessels in the presence of the growth factor bFGF in rabbits which received mAb LM609 (anti-αᵥβ₃). In contrast, angiogenesis was observed on paralimbal vessels in the presence of the growth factor bFGF in rabbits which received the mAb P3G2 (anti-αᵥβ₅). The complete inhibition of corneal angiogenesis by mAb LM609 is substantially greater than any previously reported anti-angiogenic reagent.

### c. Treatment with Polypeptides (not part of the invention)

Each experiment consisted of eight rabbits in which one eye received a pellet comprising 100 nanograms (ng) bFGF and the other eye received a pellet comprising 1 microgram (ug) VEGF. The pellets were inserted into the corneal pocket as described above, and the cytokines subsequently stimulated the growth of new blood vessels into the cornea. Peptides were administered subcutaneously (s.q.) in 1 ml PBS at an initial dosage of 50 ug per kg rabbit the day of pellet insertion, and daily s.q. dosages were given at 20 ug/kg thereafter. After 7 days, the cornea were evaluated as described above.

Rabbits receiving control peptide 69601 showed substantial corneal blood vessel growth at 7 days, in both vFGF and VEGF stimulated eyes. Rabbits receiving peptide 85189 showed less than 50% of the amount of corneal blood vessel growth compared to controls in vFGF-stimulated eyes and nearly 100% inhibition in VEGF-stimulated eyes.

### 11. In Vivo Regression of Tumor Tissue Growth With αᵥβ₃

### Antagonists As Measured by Chimeric Mouse:Human Assay

An in vivo chimeric mouse:human model was generated by replacing a portion of skin from a SCID mouse with human neonatal foreskin (Figure 17). After the skin graft was established, the human foreskin was inoculated with carcinoma cells. After a measurable tumor was established, either mAb LM609 (anti-αᵥβ₃) or PBS was injected into the mouse tail vein. Following a 2-3 week period, the tumor was excised and analyzed by weight and histology.

### A. In Vivo Chimeric Mouse:Human Assay

The in vivo chimeric mouse:human model is prepared essentially as described in Yan, et al., J. Clin. Invest., 91:986-996 (1993). Briefly, a 2 cm² square area of skin was surgically removed from a SCID mouse (6-8 weeks of age) and replaced with a human foreskin. The mouse was anesthetized and the hair removed from a 5 cm² area on each side of the lateral abdominal region by shaving. Two circular graft beds of 2 cm² were prepared by removing the full thickness of skin down to the fascia. Full thickness human skin grafts of the same size derived from human neonatal foreskin were placed onto the wound beds and sutured into place. The graft was covered with a Band-Aid which was sutured to the skin. Micropore cloth tape was also applied to cover the wound.

The M21-L human melanoma cell line or MDA 23.1 breast carcinoma cell line (ATCC HTB 26; αᵥβ₃ negative by immunoreactivity of tissue sections with mAb LM609), were used to form the solid human tumors on the human skin grafts on the SCID mice. A single cell suspension of 5 x 10⁶ M21-L or MDA 23.1 cells was injected intradermally into the human skin graft. The mice were then observed for 2 to 4 weeks to allow growth of measurable human tumors.

### B. Intravenous Application

### 1) Treatment With Monoclonal Antibodies (not part of the invention)

Following the growth of measurable tumors, SCID mice, which had been injected with M21L tumor cells, were injected intravenously into the tail vein with 250 µg of either the mAb LM609 (anti-αᵥβ₃) or PBS twice a week for 2 to 3 weeks. After this time, the tumors were resected from the skin and trimmed free of surrounding tissue. Several mice were evaluated for each treatment with the average tumor weight from each treatment being calculated and shown at the bottom of Table 6.

**Table 6**

| M21L Tumor Number | Treatment | Tumor Weight (mg) |
|---|---|---|
| 1 | PBS | 158 |
| 2 | | 192 |
| 3 | | 216 |
| 4 | | 227 |
| | | |
| 5 | LM609 | 195 |
| 6 | | 42 |
| 7 | | 82 |
| 8 | | 48 |
| 9 | | 37 |
| 10 | | 100 |
| 11 | | 172 |

| Treatment | Average Tumor Weight (mg) |
|---|---|
| PBS | 198 |
| LM609 | 113 |

Exposure of the M21L αᵥβ₃-negative human carcinoma tumor mass in the mouse:human chimeric assay system to LM609 (anti-αᵥβ₃) caused the decrease from the PBS treated average tumor weight of 198 mg to 113 mg.

Representative examples of M21L tumors treated with the mAb LM609 (anti-αᵥβ₃) and PBS were examined morphologically. The PBS-treated tumors were large (8 to 10 mm in diameter) and well vascularized whereas those treated with mAb LM609 (anti-αᵥβ₃) were much smaller (3 to 4 mm in diameter) and lacked detectable blood vessels.

In other experiments with M21-L melanoma tumor cells in the mouse:human chimeric assay system, the response with mAB LM609 was compared with the response obtained with the synthetic peptide 85189 (SEQ ID NO 15) as compared to control synthetic peptide 69601 (SEQ ID NO 6). The assays were performed as described above. The results, shown in Figure 35, demonstrate that the synthetic peptide 85189 reduced tumor volume to below 25 mm³ as compared to control peptide where the tumor volume was approximately 360 mm³. The mAB LM609 also significantly reduced tumor volume to approximately 60 mm³.

Tumors formed in skin grafts which had been injected with MDA 23.1 cells were detectable and measurable. Morphological examination of the established tumors revealed that neovascularization from the grafted human tissue into the MDA 23.1 tumor cells had occurred.

Thus, blocking of the αᵥβ₃ receptor by the intravenous application of αᵥβ₃-specific LM609 antibody and peptides resulted in a regression of a carcinoma in this model system in the same manner as the CAM and rabbit eye model systems as described in Examples 9 and 10, respectively.

### 2) Treatment with Synthetic Peptides (not part of the invention)

In a procedure similar to that described above for monoclonal antibodies, peptide antagonists of αᵥβ₃ were injected intravenously into the tail vein of SCID mice having measurable M21-L tumors. In a preliminary analysis, a dose response curve was performed for peptides 69601 (control) and 85189 (test) injected over a concentration range of 10 to 250 ug/ml. The mean volume and weight of resected tumors following treatment were determined with the results respectively shown in Figures 36A and 36B. Peptide 85189 was effective at inhibiting M21-L tumor growth over the concentration range tested compared to treatment with control peptide with the most effective dosage being 250 ug/ml.

For analyzing peptide 85189 treatment effectiveness over a time course, two treatment regimens were evaluated in the same SCID tumor model. In one assay, treatment with either peptide 85189 or 69601 was initiated on day 6, with day 0 being the day of M21-L tumor injection of 3 X 10⁶ cells subcutaneously into mouse skin, with intraperitoneal injections of 250 ug/ml peptide 85189 or control 69601 every other day until day 29. The other assay was identically performed with the exception that treatment was initiated on day 20. At the end of the assays, the tumors were resected and the mean tumor volume in mm³ was determined. The data was plotted as this value +/- the standard error of the mean.

The results of these assays, respectively shown in Figures 37A and 37B, indicate that peptide 85189 but not 69601 inhibited tumor growth at various days after treatment was initiated, depending on the particular treatment regimen. Thus, peptide 85189 is an effective αᵥβ₃ antagonist of both angiogenesis and thus tumor growth.

### 12. Stimulation of Vascular Cells to Enter the Cell cycle and

### Undergo Apoptosis in the Presence of Antagonists of Integrin αᵥβ₃ as Measured in the CAM Assay

The angiogenic process clearly depends on the capacity of cytokines such as bFGF and VEGF to stimulate vascular cell proliferation. Mignatti et al., J. Cell. Biochem., 471:201 (1991); Takeshita et al., J. Clin. Invest., 93:662 (1994); and Koyama et al., J. Cell. Physiol., 158:1 (1994). However, it is also apparent that signaling events may regulate the differentiation of these vascular cells into mature blood vessels. Thus, it is conceivable that interfering with signals related to either growth or differentiation of vascular cells undergoing new growth or angiogenesis may result in the perturbation of angiogenesis.

Integrin ligation events have been shown to participate in both cell proliferation as well as apoptosis or programmed cell death in vitro. Schwartz, Cancer Res., 51:1503 (1993); Meredith et al., Mol. Biol. Cell., 4:953 (1993); Frisch et al., J. Cell Biol., 124:619 (1994); and Ruoslahti et al., Cell, 77:477 (1994). Close examination of the effects of αᵥβ₃ antagonists on angiogenesis reveals the presence of discontinuous and disrupted tumor-associated blood vessels. Therefore, it is possible that the loss of blood vessel continuity may be due to selective necrosis or apoptosis of vascular cells.

To explore this possibility, CAMs were examined after induction of angiogenesis with the growth factor bFGF and treatment with the mAb and cyclic peptides of this invention.

### A. Treatment with Monoclonal Antibodies (not part of the invention)

Apoptosis can be detected by a variety of methods which include direct examination of DNA isolated from tissue to detect fragmentation of the DNA and the detection of 3'OH in intact tissue with an antibody that specifically detects free 3'OH groups of fragmented DNA.

### 1) Analysts of DNA Fragmentation

Angiogenesis was induced by placing filter disks saturated with bFGF on the CAMs of 10-day old embryos as described in Examples 6A. Immunohistological analysis of CAMs with LM609 (anti-αᵥβ₃) revealed peak expression of αᵥβ₃ on blood vessels 12 to 24 hours after initiation of angiogenesis with bFGF. Thus, 24 hours after stimulation with bFGF, embryos were inoculated intravenously with 100 µl of PBS alone or PBS containing 300 µg of either mAb CSAT (anti-β₁) or LM609 (anti-αᵥβ₃).

DNA fragmentation was detected by resecting the CAM tissue directly below bFGF saturated filter disks 24 or 48 hours after intravenous inoculations with mAb LM609 (anti-αᵥβ₃), CSAT (anti-β₁), or PBS. Resected CAM tissues were washed three times with sterile PBS and finely minced, resuspended in 0.25% bacterial collagenase (Worthington Biochemical; Freehold, NJ) and incubated for 90 minutes at 37C with occasional vortexing. DNA was extracted from equal numbers of CAM cells from single cell suspension as previously described. Bissonette, et al., Nature. 359:552 (1992). Briefly, equal numbers of CAM cells were lysed in 10 mM Tris-HCl, pH 8.0, 10 mM EDTA in 0.5% (v/v) Triton X-100 (Sigma, St. Louis, MO). Cell lysates were centrifuged at 16,000x g for 15 minutes at 4C to separate soluble fragmented DNA from the intact chromatin pellet. Fragmented DNA was washed, precipitated, and analyzed on a 1.2% (w/v) agarose gel.

Soluble fragmented DNA was isolated from an equal number of CAM cells from each treatment, separated electrophoretically on an agarose gel, and visualized by staining with ethidium bromide. No difference was detected in the relative amount of DNA fragmentation resulting from the three different treatments 24 hours after treatment. However, by 48 hours following treatment with mAb LM609 (anti-αᵥβ₃), a significant increase in DNA fragmentation was observed when compared to embryos treated with either mAb CSAT (anti-β₁) or PBS alone.

### 2) Stimulation of Vascular Cells to Enter the Cell Cycle

To experimentally examine the role of αᵥβ₃ in these processes, cells derived from CAMs treated with or without bFGF were stained with propidium iodide and immunoreacted with mAb LM609 (anti-αᵥβ₃).

CAMs isolated from embryos 24 and 48 hours after treatment with mAb LM609 (anti-αᵥβ₃), CSAT (anti-β₁), or PBS were dissociated into single cell suspensions by incubation with bacterial collagenase as described above. Single cells were then permeabilized and stained with Apop Tag Insitu Detection Kit according to the manufacturer's instructions (Oncor, Gaithersburg, MD). Apop Tag is an antibody that specifically detects free 3'OH groups of fragmented DNA. Detection of such free 3'OH groups is an established method for the detection of apoptotic cells. Gavrieli et al., J. Cell Biol., 119:493 (1992).

Apop Tag stained cells were then rinsed in 0.1% (v/v) Triton X-100 in PBS and resuspended in FACS buffer containing 0.5% (w/v) BSA, 0.02% (w/v) sodium azide and 200 ug/ml RNase A in PBS. Cells were incubated for 1.5 hrs, washed, and analyzed by fluorescence activated cell sorting. Cell fluorescence was measured using a FACScan flow cytometer and data analyzed as described below.

Cell fluorescence was measured with a FACScan flow cytometer (Becton Dickinson, Mountain View, CA). Side scatter (SSC) and forward scatter (FSC) were determined simultaneously and all data were collected with a Hewlet Packard (HP9000) computer equipped with FACScan research software (Becton Dickinson, Mountain View, CA). The data were analyzed with P.C Lysis version I software (Becton Dickinson, Mountain View, CA). Negative control gates were set by using cell suspensions without the addition of primary antibodies from the Apop Tag kit. Identical gating was applied to both cell populations resulting in the analysis of approximately 8,000 cells per different cell treatment.

The percent of single cells derived from mAb treated CAMs and stained with Apop Tag as determined by FACS analysis is shown in Figure 18. The black bar represents cells from embryos treated 24 hours prior to analysis. The stippled bar represents cells from embryos treated 48 hours prior to analysis. Each bar represents the mean ± S.E. of three replicates.

As shown in Figure 18, CAMs treated two days prior with mAb LM609 (anti-αᵥβ₃) showed a 3 to 4-fold increase in Apop Tag staining as compared to CAMs treated with either PBS alone or CSAT (anti-β₁).

### B. Treatment With Synthetic Peptides (not part of the invention)

CAM assays with growth factor-induced angiogenesis, as described in Example 6A, were also performed with the synthetic peptides of this invention to determine the effect of cyclic peptides on apoptosis. The peptides cyclo-RGDfV (66203) and cyclo-RADfV (69601) were prepared as described in Example 1. The peptide solutions or PBS were injected into the CAM preparation at a concentration of 300 ug/ml. At 24 and 48 hours, the filter paper and surrounding CAM tissue was dissected and stained with the Apop Tag to detect apoptosis as described above in Example 12A2).

As shown in Figure 18, CAMs treated two days prior with peptide 69203 (cyclo-RGDfV) showed a 3 to 4-fold increase in Apop Tag staining as compared to CAMs treated with either PBS alone or control cyclic peptide 69601 (cyclo-RADfV).

### C. Effect of Treatment With Monoclonal Antibodies on Apaptosis and Cell Cycle (not part of the invention)

Single cell suspensions were also examined for the number of copies of chromosomal DNA by staining with propidium iodide to determine the effect of treatment with monoclonal antibodies on the cell cycle and for apoptosis by staining with the Apop Tag.

Single cell suspensions of CAMS treated 24 or 48 hours prior with mAb LM609 (anti-αᵥβ₃) or CSAT (anti-β₁) or PBS were prepared as described in Example 12A1).

For staining of cells with the Apop Tag, cell suspensions were washed three times with buffer containing 2.5% (w/v) BSA and 0.25% (w/v) sodium azide in PBS. Cells were then fixed in 1% (w/v) paraformaldehyde in PBS for 15 minutes followed by three washes as described above. To prevent nonspecific binding, single cell suspensions were blocked with 5% (w/v) BSA in PBS overnight at 4C. Cells were then washed as before, stained with Apop Tag, and cell fluorescence measured with a FACScan as described above in Example 12A.

Cells from each experimental condition were stained with propidium iodide (Sigma, St. Louis, MO) at 10 ug/ml in PBS for 1 hour, washed two times with PBS, and analyzed for nuclear characteristics typical of apoptosis, including chromatin condensation and segmentation. The percentage of apoptotic cells were estimated by morphological analysis of cells from at least 10 to 15 randomly selected microscopic fields.

The combined results of single cell suspensions of CAMs from embryos treated with either CSAT (anti-β₁) or LM609 (ant-αᵥβ₃), stained with Apop Tag and propidium iodide, and analyzed by FACS are given in Figure 19. The Y axis represents Apop Tag staining (apoptosis), the X axis represents propidium iodide staining (DNA content). The horizontal line represents the negative gate for Apop Tag staining. The left and right panels indicate CAM cells from CSAT and LM609 treated embryos, respectively. Cell cycle analysis was performed by analysis of approximately 8,000 events per condition and data represented in a contour plot.

Samples of single cells stained with the DNA dye propidium iodide revealed that 25-30% of the LM609 (anti-αᵥβ₃) treated CAM cells 48 hours after treatment showed evidence of nuclear condensation and/or segmentation. These processes are characteristic of cells undergoing apoptosis. This is in contrast to CAMs treated with CSAT (anti-β₁) where 90-95% of the cells showed normal nuclear staining.

As shown in Figure 19, consistent with the induction of apoptosis by LM609, a significant number of cells in a peak containing less than one copy of DNA was observed (AO). This peak has been previously shown to represent fragmented DNA in late stage apoptotic cells. Telford et al., Cytometry, 13:137 (1992). Furthermore, these AO cells readily stain with Apop Tag confirming the ability of this reagent to detect apoptotic cells. However, in addition to the staining of cells in AO, a significant number of cells containing greater than one copy of DNA also stained with Apop Tag (Figure 19). These results demonstrate the LM609 has the ability to promote apoptosis among vascular cells that had already entered the cell cycle. In contrast, cells derived from control CAMs which had entered the cell cycle showed minimal Apop Tag staining consistent with the few apoptotic cells detected in control treated CAMs.

Among those cells in the bFGF stimulated CAMs that had entered the cell cycle (S and G2/M phase), 70% showed positive staining with LM609 (anti-αᵥβ₃). This is compared to 10% LM609 staining observed among cycling cells from non-bFGF treated CAMs. These findings indicate that after bFGF stimulation, the majority of the αᵥβ₃-bearing cells show active proliferation.

Taken together these findings indicate that intravenous injection of mAb LM609 or cyclic peptide antagonist of αᵥβ₃ promote apoptosis within the chick CAM following induction of angiogenesis.

CAMs were also examined histologically for expression of αᵥβ₃ by immunoreactivity with LM609 and for cells which were undergoing apoptosis by immunoreactivity with Apop Tag. CAM sections resected from embryos treated 48 hours prior with LM609 (anti-αᵥβ₃), CSAT (anti-β₁), or PBS prepared in Example 5A were washed, embedded in OTC (Baxter) and snap frozen in liquid nitrogen. Six micron sections of CAM tissues were cut, fixed in acetone for 30 seconds, and stored at -70C until use. Tissue sections were prepared for staining by a brief rinse in 70% (v/v) ethanol (ETOH) followed by washing three times in PBS. Next, sections were blocked with 5% (w/v) BSA in PBS for 2 hours, followed by incubation with 10 ug/ml of mAb LM609 for 2 hours. The sections were then washed and incubated with a 1:50 dilution of rhodamine conjugated goat anti-mouse IgG (Fisher Scientific, Pittsburg, PA) for 2 hours. Finally, the same sections were washed and stained with the Apop Tag as described in Example 12A2). The stained tissue sections were mounted and analyzed by confocal immunofluorescent microscopy.

In Figure 20, panels A through C represent CAM tissue from CSAT (anti-β₁) treated embryos and panels D through F represent CAM tissue from LM609 (anti-αᵥβ₃) treated embryos. Panels A and D depict tissues stained with Apop Tag and visualized by fluorescence (FITC) superimposed on a D.I.C. image. Panels B and E depict the same tissues stained with mAb LM609 (anti-αᵥβ₃) and visualized by fluorescence (rhodamine). Panels C and F represent merged images of the same tissues stained with both Apop Tag and LM609 where yellow staining represents colocalization. The bar represents 15 and 50 µm in the left and right panels, respectively.

As shown in Figure 20 (A-C), after intravenous injection of CSAT or PBS control, staining with Apop Tag appeared minimal and random, indicating a minimal level of apoptosis within the tissue. In contrast, CAMs from embryos previously treated with LM609 or cyclic peptide 203 showed a majority of the vessels staining intensely with Apop Tag while minimal reactivity was observed among surrounding nonvascular cells (Figure 20 D-F). Furthermore, when both Apop Tag and LM609 were used to stain these tissues (19C and 19F) significant co-localization was only observed between these markers in CAMs derived from embryos treated with αᵥβ₃ antagonists (Figure 20F). These findings demonstrate that after induction of angiogenesis in vivo, inhibitors of integrin αᵥβ₃ selectively promote apoptosis of αᵥβ₃-bearing blood vessels.

While angiogenesis is a complex process involving many molecular and cell biological events, several lines of evidence suggest that vascular cell integrin αᵥβ₃ plays a relatively late role in this process. First, immunohistological analysis reveals that expression of αᵥβ₃ on vascular cells reached a maximum 12-24 hours after the induction of angiogenesis with bFGF. Secondly, antagonists of αᵥβ₃ perturb angiogenesis induced by multiple activators suggesting that this receptor is involved in common pathway downstream from perhaps all primary signaling events leading to angiogenesis. Thirdly, mAb LM609 or cyclic peptide treated CAMs did not show a significant increase in apoptosis as measured by DNA laddering until 48 hours post treatment with these antagonists. Finally, antagonists of αᵥβ₃ promote apoptosis of vascular cells that have already been induced to enter the cell cycle.

The results presented herein provide the first direct evidence that integrin ligation events can regulate cell survival in vivo. It is therefore hypothesized that once angiogenesis begins, individual vascular cells divide and begin to move toward the angiogenic source, after which, αᵥβ₃ ligation provides a signal allowing continued cell survival which leads to differentiation and the formation of mature blood vessels. However, if αᵥβ₃ ligation is prevented then the cells fail to receive this molecular cue and the cells go into apoptosis by default. This hypothesis would also predict that after differentiation has occurred mature blood vessels no longer require αᵥβ₃ signaling for survival and thus are refractory to antagonists of this integrin.

Finally, the results presented herein provide evidence that antagonists of integrin αᵥβ₃ may provide a powerful therapeutic approach for the treatment of neoplasia or other diseases characterized by angiogenesis. First, antagonists of αᵥβ₃ disrupt newly forming blood vessels without affecting the pre-existing vasculature. Second, these antagonists had no significant effect on chick embryo viability, suggesting they are non-toxic. Third, angiogenesis was significantly blocked regardless of the angiogenic stimuli. Finally, systemic administration of αᵥβ₃ antagonists causes dramatic regression of various histologically distinct human tumors.

### 13. Preparation of Organic Molecule αᵥβ₃ Antagonists

The synthesis of organic αᵥβ₃ antagonist Compounds 7 (96112), 9 (99799), 10 (96229), 12 (112854), 14 (96113), 15 (79959)^{*}, 16 (81218)^{*}, 17 (87292)^{*} and 18 (87293)^{*} is described below and is also shown in the noted figures. Organic antagonists are also referred to by the numbers in parentheses. The resultant organic molecules, referred to as organic mimetics of this invention as previously defined, are then used in the methods for inhibiting αᵥβ₃-mediated angiogenesis as described in Example 11.
^{*} (not part of the invention)

For each of the syntheses described below, optical rotations were measured on Perkin-Elmer 241 spectrophotometer UV and visible spectra were recorded on a Beckmann DU-70 spectrometer. ¹H and ¹³C NMR spectra were recorded at 400 and 500 MHz on Bruker AMX-400 and AMX-500 spectrometer. High-resolution mass spectra (HRMS) were recorded on a VG ZAB-ZSE mass spectrometer under fast atom bombardment (FAB) conditions. Column chromatography was carried out with silica gel of 70-230 mesh. Preparative TLC was carried out on Merck Art. 5744 (0.5 mm). Melting points were taken on a Thomas Hoover apparatus.

### A. Compound 1: t-Boc-L-tyrosine benzyl ester as illustrated in Figure 38

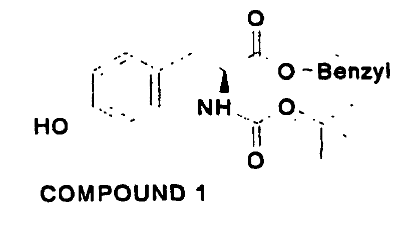

To a solution of N-(tert-butoxycarbonyl)-L-tyrosine(t-Boc-L-tyrosine) (1.0 equivalents; Aldrich) in 0.10 M (M) methylene chloride was added dicyclohexylcarbodiimide (DCC) (1.5 equivalents) at 25C and allowed to stir for 1 hour. Next, 1.5 equivalents benzyl alcohol was added and the mixture was stirred for an additional 12 hours at 25C. The reaction mixture was then diluted with ethyl acetate (0.10 M) and washed twice (2X) with water, once (1X) with brine and dried over magnesium sulfate. The solvent was then removed in vacuo and the crude product was then purified by silica gel column chromatography. Compound **1**, t-Boc-L-tyrosine benzyl ester can also be commercially purchased from Sigma.

### B. Compound 2: (S)-3-(4-(4-Bromobutyloxy)phenyl-2-N-tert-butyloxycarbonyl-propionic acid benzyl ester as illustrated in Figure 38 step i

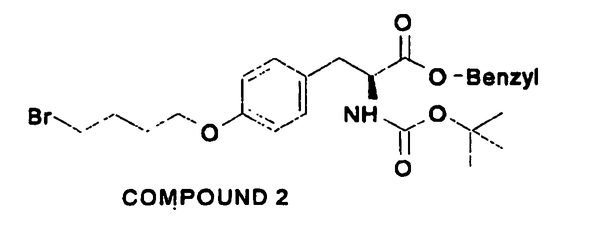

A mixture of t-Boc-L-tyrosine benzyl ester (2 grams, 5.38 mmol; synthesized as described above), 1,4-dibromobutane (1.9 ml, 16.2 mmol; Aldrich), potassium carbonate (5 g) and 18-crown-6 (0.1 g; Aldrich), was heated at 80C for 12 hours. After cooling, the precipate was filtered off and the reaction mixture was evaporated to dryness in vacuo. The crude product was then purified by crystallization using 100% hexane to yield 2.5 g (92%) of Compound **2.**

### C. Compound 3: (S)-3-(4-(4-Azidobutyloxy)phenyl-2-N-tert-butyloxycarbonyl-pronionic acid benzyl ester as illustrated in Figure 38 step ii

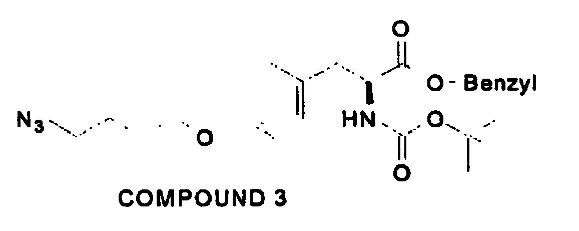

Compound **2** (2.5 g, 4.9 mmol) was stirred with sodium azide (1.6 g, 25 mmol) in dimethylformamide (DMF) (20 ml) at 25C for 12 hours. The solvent was then evaporated and the residue was treated with water (approx 10 ml) and extracted twice with ethyl acetate. The organic layers were combined, dried via magnesium sulfate and evaporated to yield 2.0 grams (90%) of Compound **3** as a colorless syrup (FAB-MS: 469 (M+H⁺).

### D. Compound 4: (S)-3-(4-(4-Azidobutyloxy)phenyl-2-amino-propionic acid benzyl ester as illustrated in Figure 38 step iii

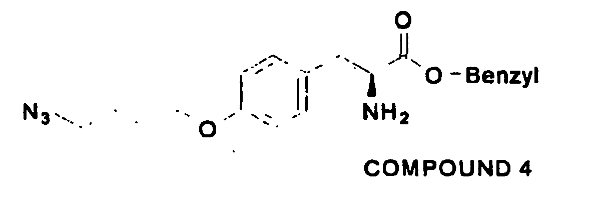

Compound **3** (2.0g (4.4 mmol)) was dissolved in trifluoroacetic acid (TFA; 2 ml) and stirred for 3 hours at room temperature. Evaporation in vacuo yielded 1.6 grams (quantitative) of Compound **4** as a colorless syrup that was used without further purification for the next step. FAB-MS: 369 (M⁺H⁺).

### E. Compound 5: (S)-3-(4-(4-Azidobutyloxy)phenyl-2-butylsulfonamido-propionic acid benzyl ester as illustrated in Figure 38 step iv

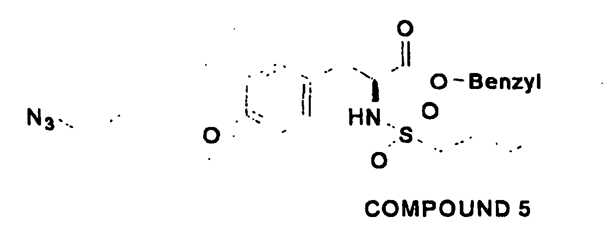

A mixture of Compound **4** (1.6g; 4.3 mmol), butane sulfonic acid chloride (0.84 ml; 6.6 mmol) and triethyl amine (1.5 equivalents) were stirred in methylene chloride (20 ml) for 12 hours at room temperature. The reaction mixture was then evaporated and the residue was dissolved in ethylacetate, washed with dilute HCl, aqueous sodium bicarbonate and water. After evaporation to dryness the crude product was purified by flash chromatography (silica gel, toluene/ ethylacetate 15:1) to yield 1.4 grams (67%) of Compound **5** as an amorphous solid.

### F. Compounds 6: (S)-3-(4-(4-Aminobutyloxy)phenyl-2-butylsulfonamido-propionic acid as illustrated in Figure 38 step v

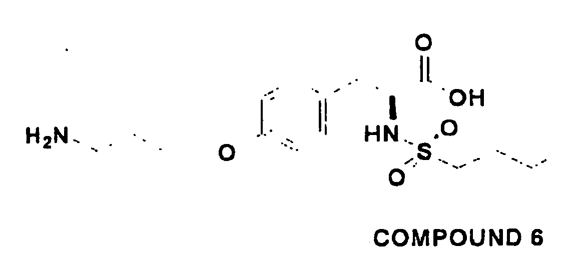

Compound **5** (1.3 g (2.6 mmol) was dissolved in 20 ml of ethyl acetate/ methanol/ water 5/3/1 and 0.2 ml trifluoroacetic acid (TFA) and hydrogenated under hydrogen (1 atmosphere; Parr Shaker apparatus) at 25C in the presence of 100 mg palladium (10% on charcoal). After 3 hours, the catalyst was filtered off and the solvent was evaporated to yield Compound 6 as an oily residue. After lyophilization from water 1.0 gram (quantitative) of Compound **6** was obtained as a white powder. FAB-MS: 373 (M⁺H⁺).

### G. Compound 7: (S)-3-(4-(4-Guanidinobutyloxy)phenyl-2-butylsulfonamido-propionic acid as illustrated in Figure 38 step vi

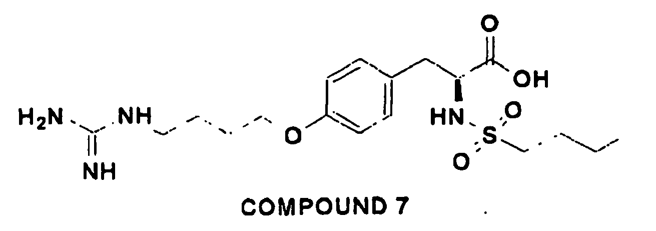

Compound **6** (200 mg; 0.5 mmol), 3,5-dimethylpyrazol-1-carboxamidine nitrate (DPFN) (170 mg; 0.8 mmol; Aldrich Chemical Company) and triethylamine (0.15 ml, 1.0 mmol) in dimethylformamide (DMF; 5 ml) were heated at 60C for 12 hours. After cooling, the solvent was evaporated in vacuo, and the residue was purified by HPLC (Lichrocart RP-18, gradient acetonitrile/ water + 0.3% TFA 99:1 to 1:99) to yield 50 mg (25%) of Compound **7** as a white, amorphous powder, after lyophilization. FAB-MS: 415 (M⁺H⁺), m.p.: 70C.

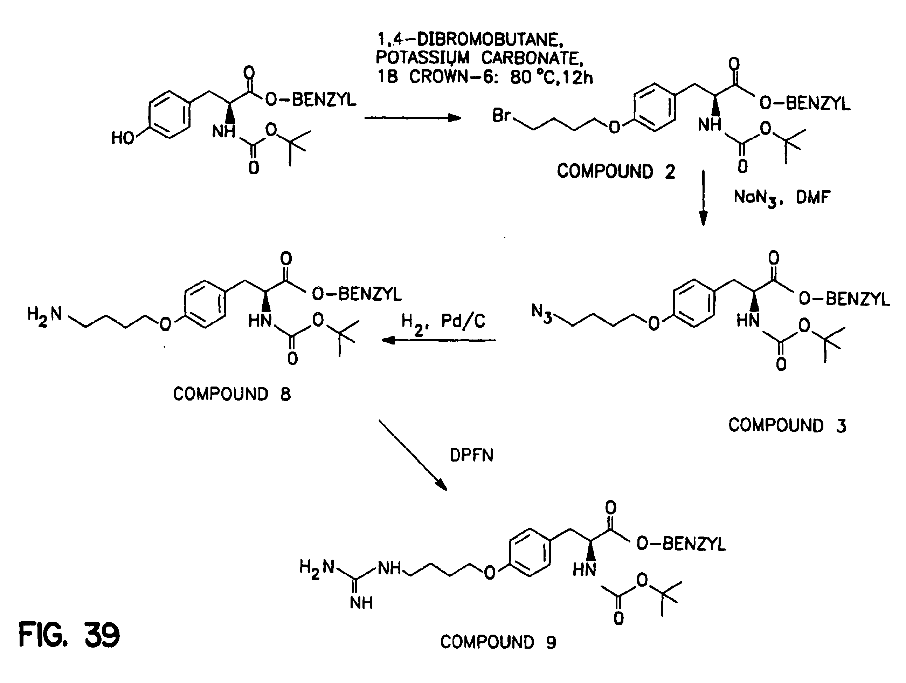

### H. Compound 8: (S)-3-(4-(4-Aminobutyloxy)ghenyl-2-N-tert.butyloxycarbonyl-propionic acid as illustrated in Figure 39 step iii

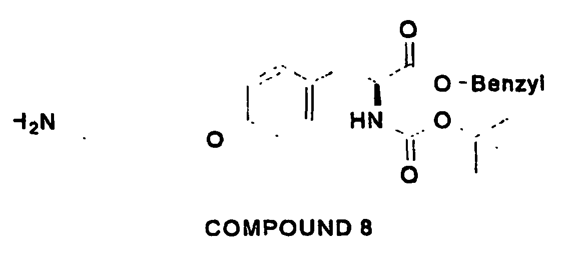

Compound **3** (0.5 g (1.07 mmol) was dissolved in 10 ml of ethyl acetate/ methanol/ water 5/3/1 and 0.1 ml trifluoroacetic acid (TFA) and hydrogenated under hydrogen (1 atmosphere; Parr Shaker apparatus) at 25C in the presence of 30 mg palladium (10% on charcoal). After 3 hours, the catalyst was filtered off and the solvent was evaporated to yield Compound 8 as an oily residue. After lyophilization from water 370 milligram (quantitative) of Compound **8** was obtained as a white powder. FAB-MS: 353 (M⁺H⁺).

### I. Compound 9: (S)-3-(4-(4-Guanidinobutyloxy)phenyl-2-N-tert.butyloxycarbonyl-propionic acid as illustrated in Figure 39 step iv

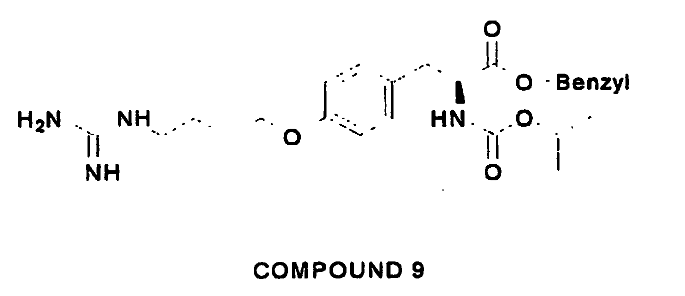

Compound **8** (200 mg; 0.5 mmol), 3,5-dimethylpyrazol-1-carboxamidine nitrate (DPFN) (170 mg; 0.8 mmol; Aldrich Chemical Company) and triethylamine (0.15 ml, 1.0 mmol) in dimethylformamide (DMF; 5 ml) were heated at 60C for 12 hours. After cooling, the solvent was evaporated in vacuo, and the residue was purified by HPLC (Lichrocart RP-18, gradient acetonitrile/ water + 0.3% TFA 99:1 to 1:99) to yield 160 mg (90%) of Compound **9** as a white, amorphous powder, after lyophilization. FAB-MS: 395 (M⁺H⁺).

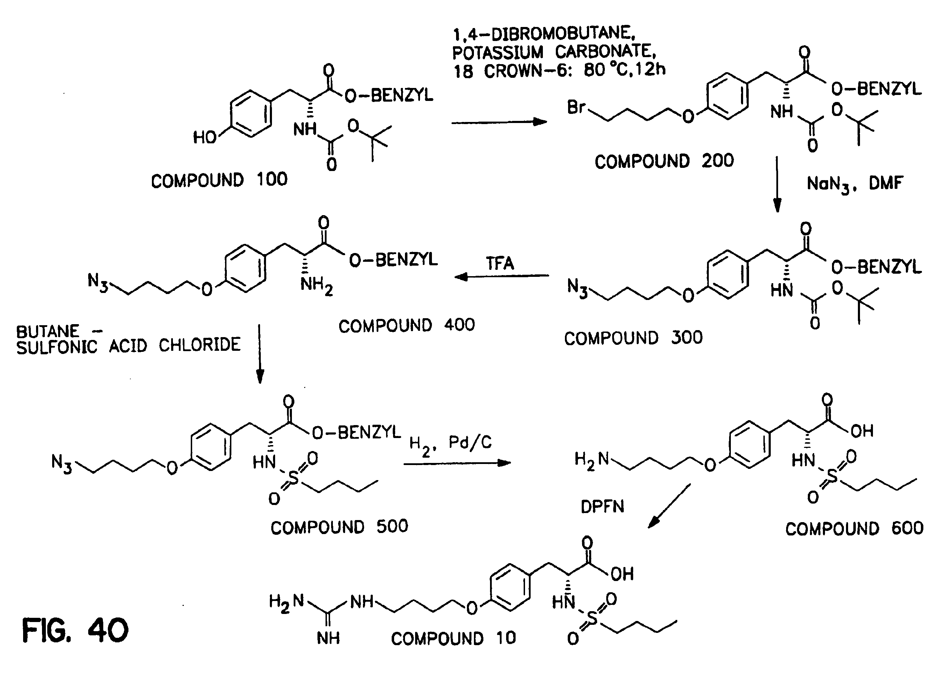

### J. Compound 10: (R)-3-(4-(4-Guanidinobutyloxy)phenyl-2-butylsulfonamido-pronignic acid as illustrated in Figure 40 steps i-vi

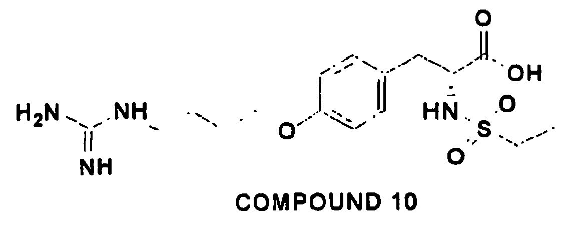

The identical reaction sequence to synthesize Compound **7** was used to prepare the D-tyrosine analog **10** of which 205 mg were obtained as a white amorphous material FAB-MS: 415 (M⁺H⁺) as follows using intermediate Compounds **100-600** to form Compound **10:**

### 1) Compound 100: t-Boc-D-tyrosine benzyl ester as illustrated in Figure 40

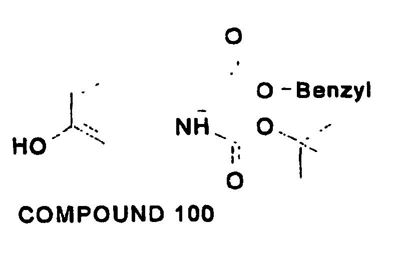

To a solution of N-(tert-butoxycarbonyl)D-tyrosine(t-Boc-L-tyrosine) (1.0 equivalents; Aldrich in 0.10 M methylene chloride was added dicyclohexylcarbodiimide (DCC) (1.5 equivalents) at 25C and allowed to stir for 1 hour. Next, 1.5 equivalents benzyl alcohol was added and the mixture was stirred for an additional 12 hours at 25C. The reaction mixture was then diluted with ethyl acetate (0.10 M) and washed 2X with water, 1X with brine and dried over magnesium sulfate. The solvent was then removed in vacuo and the crude product was then purified by silica gel column chromatography.

### 2) Compound 200: (R)-3-(4-(4-Bromobutyl)phenyl-2-N-tert-bucyloxycarbonyl-propionic acid benzyl ester as illustrated in Figure 40 step i

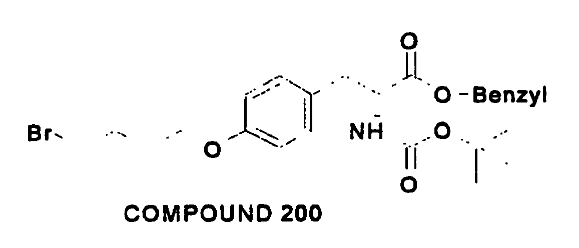

A mixture of t-Boc-D-tyrosine benzyl ester (2 grams, 5.38 mmol; synthesized as described above), 1,4-dibromobutane (1.9 ml, 16.2 mmol; Aldrich), potassium carbonate (5 g) and 18-crown-6 (0.1 g; Aldrich), was heated at 80C for 12 hours. After cooling, the precipate was filtered off and the reaction mixture was evaporated to dryness in vacuo. The crude product was then purified by crystallization using 100% hexane to yield 2.5 g (92%) of Compound **200.**

### 3) Compound 300: (R)-3-(4-(4-Azidobutyloxy)phenyl-2-N-tert-butyloxycarbonyl-propionic acid benzyl ester as illustrated in Figure 40 step ii

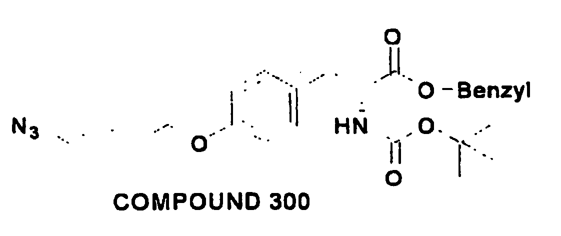

Compound **200** (2.5 g, 4.9 mmol) was stirred with sodium azide (1.6 g, 25 mmol) in dimethylformamide (DMF) (20 ml) at 25C for 12 hours. The solvent was then evaporated and the residue was treated with water (approx 10 ml) and extracted twice with ethyl acetate. The organic layers were combined, dried via magnesium sulfate and evaporated to yield 2.0 grams (90%) of Compound **300** as a colorless syrup (FAB-MS: 469 (M+H⁺).

### 4) Compound 400: (R)-3-(4-(4-Azidobutyloxy)phenyl-2-amino-pronionic acid benzyl ester as illustrated in Figure 40 step iii

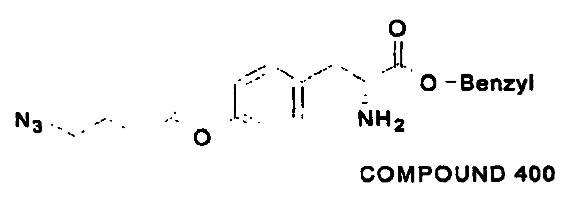

Compound **300** (2.0g (4.4 mmol) was dissolved in trifluoroacetic acid (TFA; 2 ml) and stirred for 3 hours at room temperature. Evaporation in vacuo yielded 1.6 grams (quantitative) of Compound **400** as a colorless syrup that was used without further purification for the next step. FAB-MS: 369 (M⁺H⁺)

### 5) Compound 500: (R)-3-(4-(4-Azidobutyloxy)phenyl-2-butylsulfonamido-propionic acid benzyl ester as illustrates in Figure 40 step iv

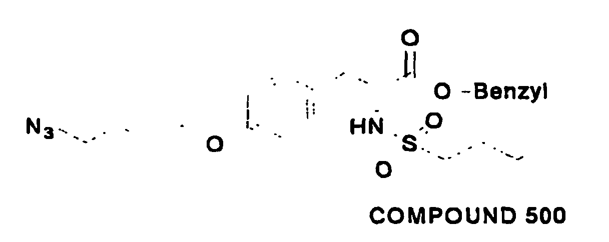

A mixture of Compound **400** (1.6g; 4.3 mmol), butane sulfonic acid chloride (0.84 ml; 6.6 mmol) and triethyl amine (1.5 equivalents) were stirred in methylene chloride (20 ml) for 12 hours at room temperature. The reaction mixture was then evaporated and the residue was dissolved in ethylacetate, washed with dilute HCl, aqueous sodium bicarbonate and water. After evaporation to dryness the crude product was purified by flash chromatography (silica gel, toluene/ ethylacetate 15:1) to yield 1.4 grams (67%) of Compound **500** as an amorphous solid.

### 6) Compound 600: (R)-3-(4-(4-Aminobutyloxy)phenyl-2-butylsulfonamino-propionic acid as illustrated in Figure 40 step v

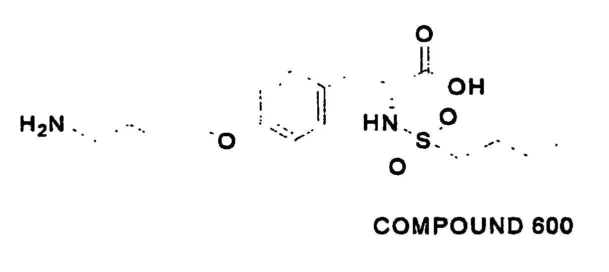

Compound **500** (1.3 g (2.6 mmol) was dissolved in 20 ml of ethyl acetate/methanol/water 5/3/1 and 0.2 ml trifluoroacetic acid (TFA) and hydrogenated under hydrogen (1 atmosphere; Parr Shaker apparatus) at 25C in the presence of 100 mg palladium (10% on charcoal). After 3 hours, the catalyst was filtered off and the solvent was evaporated to yield Compound 600 as an oily residue. After lyophilization from water 1.0 gram (quantitative) of Compound **600** was obtained as a white powder. FAB-MS: 373 (M⁺H⁺).

### 7) Compound 10: (R)-3-(4-(4-Guanidinobutyloxy)phenyl-2-butylsulfonamido-propionic acid as illustrated in Figure 40 step vi

Compound **600** (200 mg; 0.5 mmol), 3,5-dimethylpyrazol-1-carboxamidine nitrate (DPFN) (170 mg; 0.8 mmol; Aldrich Chemical Company) and triethylamine (0.15 ml, 1.0 mmol) in dimethylformamide (DMF; 5 ml) were heated at 60C for 12 hours. After cooling, the solvent was evaporated in vacuo, and the residue was purified by HPLC (Lichrocart RP-18, gradient acetonitrile/ water + 0.3% TFA 99:1 to 1:99) to yield 50 mg (25%) of Compound **10** as a white, amorphous powder, after lyophilization. FAB-MS: 415 (M⁺H⁺), m.p.: 70C.

### K. Compound 11: (S)-3-(4-(4-Azidobutyloxy)phenyl-2-(10-camphorsulfonamido)-propionic acid benzyl ester as illustrated in Figure 4

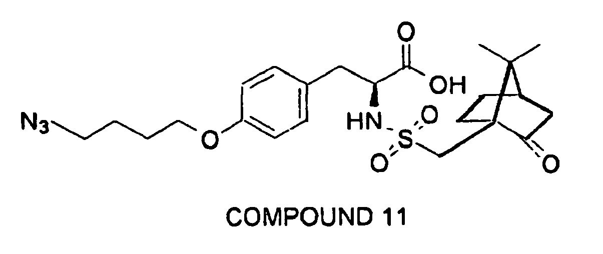

A mixture of compound 4 (1.0g; 2.7 mmol), 10-camphorsulfonic acid chloride (6.6 mmol; Aldrich Chemical Company) and triethyl amine (1.5 equivalents) were stirred in methylene chloride (20 mL) for 12 hours at room temperature. The reaction mixture was then evaporated and the residue was dissolved in ethylacetate, washed with dilute HCl, aqueous sodium bicarbonate and water. After evaporation to dryness the crude product was purified by flash chromatography (silica gel, toluene/ ethylacetate 15:1) to yield 1.4 grams (67%) of compound 11 as an amorphous solid.

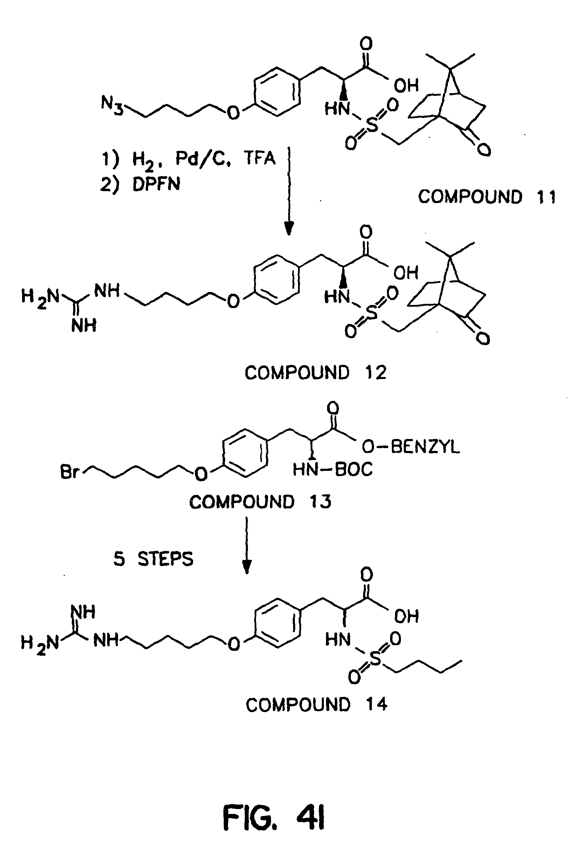

### L. Compound 12: (S)-3-(4-(4-Guanidinobutyloxy)phenyl-2-(10-camphorsulfonamido)-propionic acid as illustrated in Figure 41 steps i-ii

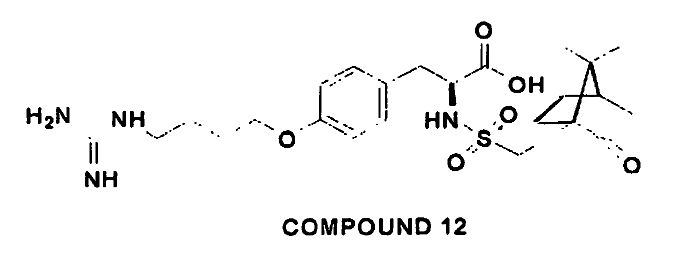

Compound **12** was obtained after hydrogenation and guanylation of Compound **11** according to the following conditions:
Step i: Compound **11** (1.3 g (2.6 mmol) was dissolved in 20 ml of ethyl acetate/ methanol/ water 5/3/1 and 0.2 ml trifluoroacetic acid (TFA) and hydrogenated under hydrogen (1 atmosphere; Parr Shaker apparatus) at 25C in the presence of 100 mg palladium (10% on charcoal). After 3 hours, the catalyst was filtered off and the solvent was evaporated to yield the intermediate amine as an oily residue. After lyophilization from water 1.0 gram (quantitative) of the intermediate amine was obtained as a white powder, which was carried on as follows:
Step ii: The above formed intermediate amine compound (200 mg; 0.5 mmol), 3,5-dimethylpyrazol-1-carboxamidine nitrate (DPFN) (170 mg; 0.8 mmol; Aldrich Chemical Company) and triethylamine (0.15 ml, 1.0 mmol) in dimethylformamide (DMF; 5 ml) were heated at 60C for 12 hours. After cooling, the solvent was evaporated in vacuo, and the residue was purified by HPLC (Lichrocart RP-18, gradient acetonitrile/ water + 0.3% TFA 99:1 to 1:99) to yield 50 mg (25%) of Compound 12 as a white, amorphous powder, after lyophilization. FAB-MS: 509.6 (M⁺H⁺).

### M. Compound 13: (S)-3-(4-(5-Bromopentyloxy)phenyl-2-N-tert.butyloxycarbonyl-propionic acid benzyl ester as illustrated in Figure 41

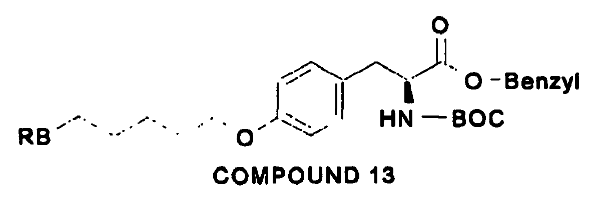

A mixture of t-Boc-L-tyrosine benzyl ester (4.5 grams, 12.1 mmol; Compound **1** synthesized as described above), 1,5-dibromopentane (5 ml, 36.7 mmol; Aldrich), potassium carbonate (10 g) and 18-crown-6 (0.25 g; Aldrich), was heated at 80C for 12 hours. After cooling, the precipate was filtered off and the reaction mixture was evaporated to dryness in vacuo. The crude product was then purified by crystallization using 100% hexane to yield 5.35 g (85%) of Compound **13.**

### N. Compound 14: (S)-3-(4-(5-Guanidinopentyloxy)phenyl-2-butylsulfonamido-propionic acid as illustrated in Figure 41 steps i-v

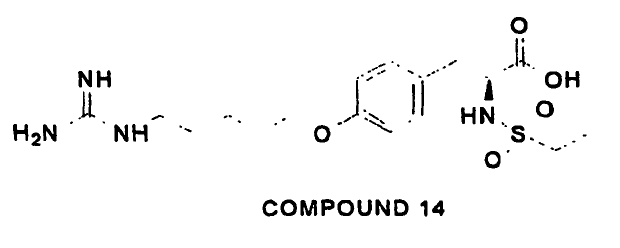

The 5 step reaction sequence of bromine-azide-exchange, Boc-cleavage, sulfonylation with butane sulfonic acid chloride, hydrogenation and guanylation with DPFN was carried out identically to the above procedures using intermediate Compounds **1-6** to form Compound **7** or the procedures using Compounds 100-600 to form Compound **10,** as disclosed above. Compound **14** was obtained as a white powder FAB-MS: 429 (M⁺H⁺).

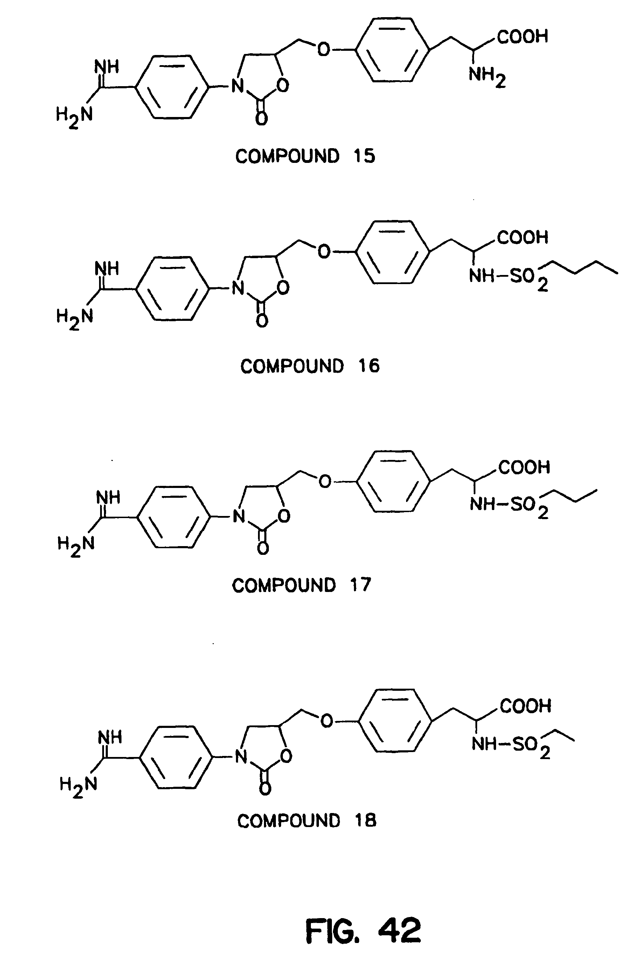

### O. Compound 15: 3-(4-amidinophenyl)-5-(4-(2-carboxy-2-amino-ethyl)phenoxy)methyl-2-oxzolidinoone, dihydrochloride as shown in Figure 42

### 1) Syntheses of starting material 2-N-BOC-amino-3-(4-hydroxy-phenyl)propionate for Compound 15

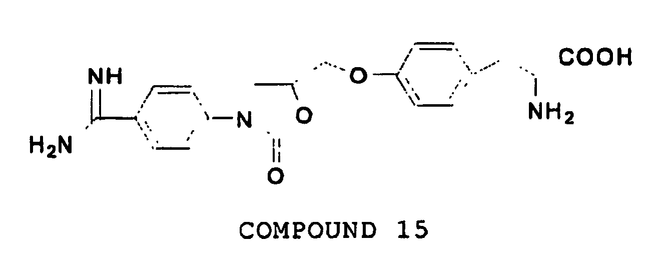

The starting material 2-N-BOC-amino-3-(4-hydroxyphenyl)propionate was obtained via esterification of (D or L), N-(tert-butoxycarbonyl)-L(D)-tyrosine (t-Boc-L(D)-tyrosine) (1.0 equivalents; Sigma) in 0.10 M methanol and dilute 1% HCl. The reaction mixture was stirred at 25C for 12 hours and then neutralized via potassium carbonate and then diluted with ethyl acetate (0.10 M) and washed 2X with water, 1X with brine and dried over magnesium sulfate. The solvent was then removed in vacuo and the crude product was then purified by silica gel column chromatography to obtain 2-N-BOC-amino-3-(4-hydroxyphenyl)propionate.

### 2) SYnthesis of starting material 3-p-N-BOC-amidinophenyl-5-methanesulfonyloxy-methyl-2-oxazolidinone for Compound 15: 3-step procedure as follows:

*p*-amino-benzonitrile (1.0 equivalents; Aldrich) in methylene chloride (0.10 M) was stirred with 2,3-epoxypropanol (1.0 equivalents; Aldrich) for 12 hours at 25C. The solvent was next removed in vacuo and the crude 4-(2,3-dihydroxypropylamino)benzonitrile was carried onto the next step as follows:
4-(2,3-dihydroxypropylamino)benzonitrile (1.0 equivalents; as described above), in dimethylformamide (0.10 M), at 25C, was stirred with diethyl carbonate (1.1 equivalents; Aldrich) and potassium tert-butylate (1.1 equivalents; Aldrich) at 110C for 6 hours. Next, the reaction mixture was diluted with ethyl acetate (0.10 M) and washed 2X with water, 1X with brine and dried over magnesium sulfate. The solvent was then removed in vacuo and the crude product was then purified by silica gel column chromatography to obtain 3-(4-cyanophenyl)-5-hydroxymethyl-2-oxazolidine and carried onto the next step as follows:
3-(4-cyanophenyl)-5-hydroxymethyl-2-oxazolidine (1.0 equivalents; as described above), in methylene chloride (0.10 M) at 25C was stirred with 1.1 equivalents hydrogen sulfide, 1.1 equivalents methyl iodide, and 1.1 equivalents ammonium acetate. The reaction mixture was stirred for 6 hours and then diluted with ethyl acetate (0.10 M) and washed 2X with water, 1X with brine and dried over magnesium sulfate. The solvent was then removed in vacuo and the crude product was then purified by silica gel column chromatography to obtain the amidine which was carried onto the next step as follows:
1.0 equivalents of the amidine, synthesized as described above, was protected with 1.1 equivalents of BOC-ON (2-(BOC-oxyimino)-2-phenylacetonitrile; Aldrich) in methylene chloride (0.10 M) at 25C and stirred for 6 hours. Next, the reaction mixture was diluted with ethyl acetate (0.10 M) and washed 2X with water, 1X with brine and dried over magnesium sulfate. The solvent was then removed in vacuo and the crude product was then esterified in 0.10 M methylene chloride and 1.1 equivalents methanesulfonyl chloride. The reaction mixture was stirred at 0C for 6 hours and then quenched with water (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain 3-p-N-BOC-amidino-phenyl-5-methanesulfonyloxy-methyl-2-oxazolidinone.

### 3) Coupling of intermediates 2-N-BOC-amino-3-(4-hydroxy-phenyl)propionate with 3-p-N-BOC-amidinophenyl-5-methanegulfonyloxy-methyl-2-oxazolidinone to form protected form of Compound 15, 3-(4-BOC-amidinophenyl)-5-(4-(2-methoxycarbonyl-2N-BOC-aminoethyl)phenyoxylmethyl-2-oxazolidinone

A mixture of 1.9 grams 2-N-BOC-amino-3-(4-hydroxy-phenyl)propionate (as described above), 20 ml dimethylformamide (DMF) and NaH (1.0 equivalent), was stirred for 30 minutes at room temperature. After stirring, 1.8 grams 3-p-N-BOC-amidino-phenyl-5-methanesulfonyloxy-methyl-2-oxazolidinone (as described above) in 10 ml dimethylformamide (DMF) was added and stirred again for 15 minutes at room temperature. The reaction mixture was then diluted with ethyl acetate (0.10 M) and washed 2X with water, 1X with brine and dried over magnesium sulfate. The solvent was then removed in vacuo and the crude product was then purified by silica gel column chromatography to obtain protected form of Compound 15, 3-(4-BOC-amidinophenyl)-5-(4-(2-methoxy-carbonyl-2N-BOC-aminoethyl)phenyoxylmethyl-2-oxazolidinone which was carried onto the next step.

### 4) Deprotection of protected form of Compound 15 to form Compound 15: 3-(4-amidinophenyl)-5-(4-(2-carboxsr-2-amino-ethyl)ohenoxy)methyl-2-oxazolidinone, dihydrochloride, Figure 42

Treatment of the protected form of Compound 15, 3-(4-BOC-amidinophenyl)-5-(4-(2-methoxy-carbonyl-2N-BOC-aminoethyl)phenyoxylmethyl-2-oxazolidinone (1.0 equivalents; synthesized as described above), with 4 ml 2N NaOH for 4 hours at room temperature. The mixture was then followed with 40 ml 2N HCl-solution in dioxane added dropwise at 0C to 25C for 3 hours. The reaction mixture was then quenched with sodium bicarbonate (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain Compound 15: 3-(4-amidinophenyl)-5-(4-(2-carboxy-2-amino-ethyl)phenoxy)methyl-2-oxazolidinone, dihydrochloride; m.p. 165C(d).

### P. Compound 16: 3-(4-amidinoshenyl)-5-(4-(2-carboxy-2-N-butylsulfonylaminoethyl)phenoxy)methyl-2-oxazolidinone as shown in Figure X (old 14)

### 1) Synthesis of starting material 2-N-butylsulfonylamino-3-(4-hydroxy-phenyl)propionate for Compound 16

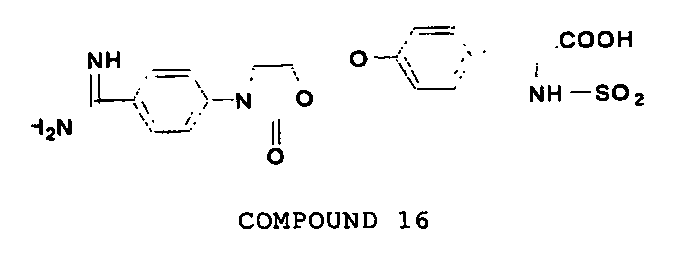

The starting material 2-N-butylsulfonylamino-3-(4-hydroxy-phenyl)propionate was obtained via esterification of ((D or L) tyrosine) (1.0 equivalents; Sigma) in 0.10 M methanol and dilute 1% HCl. The reaction mixture was stirred at 25C for 12 hours and then neutralized via potassium carbonate and then diluted with ethyl acetate (0.10 M) and washed 2X with water, 1X with brine and dried over magnesium sulfate. The solvent was then removed in vacuo and the crude product was then carried on as follows:
A mixture of the above compound (4.3 mmol), butane sulfonic acid chloride (6.6 mmol) and triethyl amine (1.5 equivalents) were stirred in methylene chloride (20 ml) for 12 hours at room temperature. The reaction mixture was then evaporated and the residue was dissolved in ethylacetate, washed with dilute HCl, aqueous sodium bicarbonate and water. After evaporation to dryness the crude product was purified by flash chromatography (silica gel, toluene/ ethylacetate 15:1) to yield the title compound.

### 2) Synthesis of starting material 3-p-N-BOC-amidinophenyl-5-methanesulfonyloxy-methyl-2-oxazolidinone for Compound 16: 3-step procedure as follows :

p-amino-benzonitrile (1.0 equivalents; Aldrich) in methylene chloride (0.10 M) was stirred with 2,3-epoxypropanol (1.0 equivalents; Aldrich) for 12 hours at 25C. The solvent was next removed in vacuo and the crude 4-(2,3-dihydroxypropylamino)benzonitrile was carried onto the next step as follows:
4-(2,3-dihydroxypropylamino)benzonitrile (1.0 equivalents; as described above), in dimethylformamide (0.10 M), at 25C, was stirred with diethyl carbonate (1.1 equivalents; Aldrich) and potassium tert-butylate (1.1 equivalents; Aldrich) at 110C for 6 hours. Next, the reaction mixture was diluted with ethyl acetate (0.10 M) and washed 2X with water, 1X with brine and dried over magnesium sulfate. The solvent was then removed in vacuo and the crude product was then purified by silica gel column chromatography to obtain 3-(4-cyanophenyl)-5-hydroxymethyl-2-oxazolidine and carried onto the next step as follows:
3-(4-cyanophenyl)-5-hydroxymethyl-2-oxazolidine (1.0 equivalents; as described above), in methylene chloride (0.10 M) at 25C was stirred with 1.1 equivalents hydrogen sulfide, 1.1 equivalents methyl iodide, and 1.1 equivalents ammonium acetate. The reaction mixture was stirred for 6 hours and then diluted with ethyl acetate (0.10 M) and washed 2X with water, 1X with brine and dried over magnesium sulfate. The solvent was then removed in vacuo and the crude product was then purified by silica gel column chromatography to obtain the amidine which was carried onto the next step as follows:
1.0 equivalents of the amidine, synthesized as described above, was protected with 1.1 equivalents of BOC-ON (2-(BOC-oxyimino)-2-phenylacetonitrile; Aldrich) in methylene chloride (0.10 M) at 25C and stirred for 6 hours. Next, the reaction mixture was diluted with ethyl acetate (0.10 M) and washed 2X with water, 1X with brine and dried over magnesium sulfate. The solvent was then removed in vacuo and the crude product was then esterified in 0.10 M methylene chloride and 1.1 equivalents methanesulfonyl chloride. The reaction mixture was stirred at 0C for 6 hours and then quenched with water (5 equivalents) and then diluted with ethyl acetate (0.10 M) and washed 2X with water, 1x with brine and dried over magnesium sulfate. The solvent was then removed in vacuo and the crude product was then purified by silica gel column chromatography to obtain 3-p-N-BOC-amidino-phenyl-5-methanesulfonyloxy-methyl-2-oxazolidinone.

### 3) Coupling of intermediates 2-N-butylsulfonylamino-3-(4-hydroxy-phenyl)propionate with 3-p-N-BOC-amidino-phenyl-5-methanesulfonyloxy-methyl-2-oxazolidinone to form protected form of Compound 16. 3-(4-BOC-amidinonhenyl)-5-(4-(2-methoxycarbonyl-2-N-butylsulfonylaminoethyl)phenyoxylmethyl-2-oxazolidinone

A mixture of 1.9 grams 2-N-butylsulfonylamino-3-(4-hydroxy-phenyl)propionate (as described above), 20 ml dimethylformamide (DMF) and NaH (1.0 equivalent), was stirred for 30 minutes at room temperature. After stirring, 1.8 grams 3-p-N-BOC-amidino-phenyl-5-methanesulfonyloxy-methyl-2-oxazolidinone (as described above) in 10 ml dimethylformamide (DMF) was added and stirred again for 15 minutes at room temperature. The reaction mixture was then diluted with ethyl acetate (0.10 M) and washed 2X with water, 1X with brine and dried over magnesium sulfate. The solvent was then removed in vacuo and the crude product was then purified by silica gel column chromatography to obtain protected form of Compound 16, 3-(4-BOC-amidinophenyl)-5-(4-(2-methoxy-carbonyl-2-N-butylsulfonylaminoethyl)-phenyoxylmethyl-2-oxazolidinone which was carried onto the next step.

### 4) Deprotection of protected form of Compound 16 to form Compound 16: 3-(4-amidinophenyl)-5-(4-(2-carboxy-2-N-butylsulfonylaminoethyl)phenoxy)methyl-2-oxazolidinone, Figure 42

Treatment of the protected form of Compound 16, 3-(4-BOC-amidinophenyl)-5-(4-(2-methoxy-carbonyl-2-N-butylsulfonylaminoethyl)phenyoxylmethyl-2-oxazolidinone (1.0 equivalents; synthesized as described above), with 4 ml 2N NaOH for 4 hours at room temperature. The mixture was then followed with 40 ml 2N HCl-solution in dioxane added dropwise at 0C to 25C for 3 hours. The reaction mixture was then quenched with sodium bicarbonate (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain Compound 16: 3-(4-amidinophenyl)-5-(4-(2-carboxy-2-N-butylsulfonylaminoethyl)phenoxy)methyl-2-oxazolidinone; m.p. 236-237C.

### Q. Compound 17: 3-(4-amidinophenyl)-5-(4-(2-carboxy-2-N-propyl-sulfonylaminoethyl)phenoxy)methyl-2-oxazolidinone as shown in Figure 42

### 1) Synthesis of starting material 2-N-propyl-sulfonylamino-3-(4-hydroxy-phenyl)propionate for Compound 17:

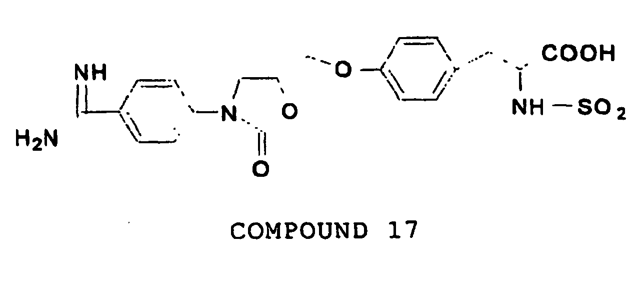

The starting material 2-N-propyl-sulfonylamino-3-(4-hydroxy-phenyl)propionate was obtained via esterification of ((D or L) tyrosine) (1.0 equivalents; Sigma) in 0.10 M methanol and dilute 1% HCl. The reaction mixture was stirred at 25C for 12 hours and then neutralized via potassium carbonate and then diluted with ethyl acetate (0.10 M) and washed 2X with water, 1X brine and dried over magnesium sulfate. The solvent was then removed in vacuo and the crude product was then carried on as follows:
A mixture of the above compound (4.3 mmol), propyl sulfonic acid chloride (6.6 mmol; Aldrich) and triethyl amine (1.5 equivalents) were stirred in methylene chloride (20 ml) for 12 hours at room temperature. The reaction mixture was then evaporated and the residue was dissolved in ethylacetate, washed with dilute HCl, aqueous sodium bicarbonate and water. After evaporation to dryness the crude product was purified by flash chromatography (silica gel, toluene/ ethylacetate 15:1) to yield the title compound.

### 2) synthesis of starting material 3-p-N-BOC-amidino-phanyl-5-methanesulfonyloxy-methyl-2-oxazolidinone for Compound 17: 3-step procedure as follows:

*p*-amino-benzonitrile (1.0 equivalents; Aldrich) in methylene chloride (0.10 M) was stirred with 2,3-epoxypropanol (1.0 equivalents; Aldrich) for 12 hours at 25C. The solvent was next removed in vacuo and the crude 4-(2,3-dihydroxypropylamino)benzonitrile was carried onto the next step as follows:
4-(2,3-dihydroxypropylamino)benzonitrile (1.0 equivalents; as described above), in dimethylformamide (0.10 M), at 25C, was stirred with diethyl carbonate (1.1 equivalents; Aldrich) and potassium tert-butylate (1.1 equivalents; Aldrich) at 110C for 6 hours. Next, the reaction mixture was diluted with ethyl acetate (0.10 M) and washed 2X with water, 1X with brine and dried over magnesium sulfate. The solvent was then removed in vacuo and the crude product was then purified by silica gel column chromatography to obtain 3-(4-cyanophenyl)-5-hydroxymethyl-2-oxazolidine and carried onto the next step as follows:
3-(4-cyanophenyl)-5-hydroxymethyl-2-oxazolidine (1.0 equivalents; as described above), in methylene chloride (0.10 M) at 25C was stirred with 1.1 equivalents hydrogen sulfide, 1.1 equivalents methyl iodide, and 1.1 equivalents ammonium acetate. The reaction mixture was stirred for 6 hours and then diluted with ethyl acetate (0.10 M) and washed 2X with water, 1X with brine and dried over magnesium sulfate. The solvent was then removed in vacuo and the crude product was then purified by silica gel column chromatography to obtain the amidine which was carried onto the next step as follows:
1.0 equivalents of the amidine, synthesized as described above, was protected with 1.1 equivalents of BOC-ON (2-(BOC-oxyimino)-2-phenylacetonitrile; Aldrich) in methylene chloride (0.10 M) at 25C and stirred for 6 hours. Next, the reaction mixture was diluted with ethyl acetate (0.10 M) and washed 2X with water, 1X with brine and dried over magnesium sulfate. The solvent was then removed in vacuo and the crude product was then esterified in 0.10 M methylene chloride and 1.1 equivalents methanesulfonyl chloride. The reaction mixture was stirred at 0C for 6 hours and then quenched with water (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain 3-p-N-BOC-amidino-phenyl-5-methanesulfonyloxy-methyl-2-oxazolidinone.

### 3) Coupling of intermediates 2-N-propyl-sulfonylamino-3-(4-hydroxy-phenyl)propionate with 3-p-N-BOC-amidino-phenyl-5-methanesulfonyloxy-methyl-2-oxazolidinone to form protected form of Compound 17 3-(4-BOC-amidinophenyl)-5-(4-(2 methoxy-carbonyl-2-N-propyl-sulfonylaminoethyl)-phenyoxylmethyl-2-oxazolidinone

A mixture of 1.9 grams 2-N-propyl-sulfonylamino-3-(4-hydroxy-phenyl)propionate (as described above), 20 ml dimethylformamide (DMF) and NaH (1.0 equivalent), was stirred for 30 minutes at room temperature. After stirring, 1.8 grams 3-p-N-BOC-amidino-phenyl-5-methanesulfonyloxy-methyl-2-oxazolidinone (as described above) in 10 ml dimethylformamide (DMF) was added and stirred again for 15 minutes at room temperature. The reaction mixture was then diluted with ethyl acetate (0.10 M) and washed 2X with water, 1X with brine and dried over magnesium sulfate. The solvent was then removed in vacuo and the crude product was then purified by silica gel column chromatography to obtain protected form of Compound 17, 3-(4-HOC-amidinophenyl)-5-(4-(2-methoxy-carbonyl-2-N-propyl-sulfonylaminoethyl)-phenyoxylmethyl-2-oxazolidinone which was carried onto the next step.

### 4) Deprotection of protected form of Compound 17 to form Compound 17: 3-(4-amidinophenyl)-5-(4-(2-carboxy-2-N-propylsulfonylaminoethyl)phenoxy)methyl-2-oxazolidinone, Figure 42

Treatment of the protected form of Compound 17, 3-(4-BOC-amidinophenyl)-5-(4-(2-methoxy-carbonyl-2-N-propylsulfonylaminoethyl)phenyoxylmethyl-2-oxazolidinone (1.0 equivalents; synthesized as described above), with 4 ml 2N NaOH for 4 hours at room temperature. The mixture was then followed with 40 ml 2N HCl-solution in dioxane added dropwise at 0C to 25C for 3 hours. The reaction mixture was then quenched with sodium bicarbonate (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain Compound 17: 3-(4-amidinophenyl)-5-(4-(2-carboxy-2-N-propylsulfonylaminoethyl)phenoxy)methyl-2-oxazolidinone; m.p. 200C (d).

### R. Compound 18 :3-(4-amidinophenyl)-5-(4-(2-carboxy-2-N-ethyl-sulfonylaminoethyl)phenoxy)methyl-2-oxazolidinone as shown in Figure 42

### 1) Synthesis of starting material 2-N-ethyl-sulfonylamino-3-(4-hydroxy-phenyl)propionatefor Compound 18:

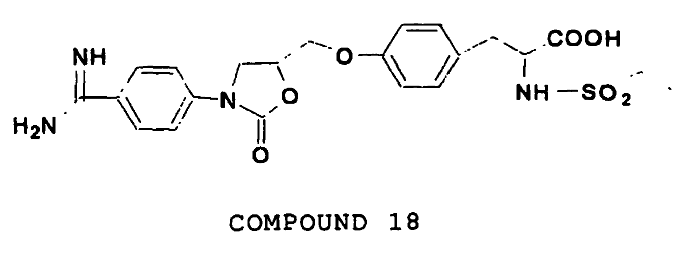

The starting material 2-N-ethyl-sulfonylamino-3-(4-hydroxy-phenyl)propionate was obtained via esterification of ((D or L) tyrosine) (1.0 equivalents; Sigma) in 0.10 M methanol and dilute 1% RC1. The reaction mixture was stirred at 25C for 12 hours and then neutralized via potassium carbonate and then diluted with ethyl acetate (0.10 M) and washed 2X with water, 1X with brine and dried over magnesium sulfate. The solvent was then removed in vacuo and the crude product was then carried on as follows:
A mixture of the above compound (4.3 mmol), ethyl sulfonic acid chloride (6.6 mmol; Aldrich) and triethyl amine (1.5 equivalents) were stirred in methylene chloride (20 ml) for 12 hours at room temperature. The reaction mixture was then evaporated and the residue was dissolved in ethylacetate, washed with dilute HCl, aqueous sodium bicarbonate and water. After evaporation to dryness the crude product was purified by flash chromatography (silica gel, toluene/ ethylacetate 15:1) to yield the title compound.

### 2) synthesis of starting material 3-p-N-BOC-amidinophenyl-5-methanesulfonyloxy-methyl-2-oxazolidinone for Compound 18: 3-step procedure as follows:

p-amino-benzonitrile (1.0 equivalents; Aldrich) in methylene chloride (0.10 M) was stirred with 2,3-epoxypropanol (1.0 equivalents; Aldrich) for 12 hours at 25C. The solvent was next removed in vacuo and the crude 4-(2,3-dihydroxypropylamino)benzonitrile was carried onto the next step as follows:
4-(2,3-dihydroxypropylamino)benzonitrile (1.0 equivalents; as described above), in dimethylformamide (0.10 M), at 25C, was stirred with diethyl carbonate (1.1 equivalents; Aldrich) and potassium tert-butylate (1.1 equivalents; Aldrich) at 110C for 6 hours. Next, the reaction mixture was diluted with ethyl acetate (0.10 M) and washed 2X with water, 1X with brine and dried over magnesium sulfate. The solvent was then removed in vacuo and the crude product was then purified by silica gel column chromatography to obtain 3-(4-cyanophenyl)-5-hydroxymethyl-2-oxazolidine and carried onto the next step as follows:
3-(4-cyanophenyl)-5-hydroxymethyl-2-oxazolidine (1.0 equivalents; as described above), in methylene chloride (0.10 M) at 25C was stirred with 1.1 equivalents hydrogen sulfide, 1.1 equivalents methyl iodide, and 1.1 equivalents ammonium acetate. The reaction mixture was stirred for 6 hours and then diluted with ethyl acetate (0.10 M) and washed 2X with water, 1X with brine and dried over magnesium sulfate. The solvent was then removed in vacuo and the crude product was then purified by silica gel column chromatography to obtain the amidine which was carried onto the next step as follows:
1.0 equivalents of the amidine, synthesized as described above, was protected with 1.1 equivalents of BOC-ON (2-(BOC-oxyimino)-2-phenylacetonitrile; Aldrich) in methylene chloride (0.10 M) at 25C and stirred for 6 hours. Next, the reaction mixture was diluted with ethyl acetate (0.10 M) and washed 2X with water, 1X with brine and dried over magnesium sulfate. The solvent was then removed in vacuo and the crude product was then esterified in 0.10 M methylene chloride and 1.1 equivalents methanesulfonyl chloride. The reaction mixture was stirred at 0C for 6 hours and then quenched with water (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain 3-p-N-BOC-amidino-phenyl-5-methanesulfonyloxy-methyl-2-oxazolidinone.

### 3) Coupling of intermediates 2-N-ethyl-sulfonylamino-3-(4-hydroxy-phenyl)propionate with 3-p-N-BOC-amidino-phenyl-5-methanesulfonyloxy-methyl-2-oxazolidinone to form protected form of Compound 18. 3-(4-BOC-amidinophenyl)-5-(4-(2-methoxy-carbonyl-2-N-ethyl-sulfonylaminoethyl)-phenyoxylmethyl-2-oxazolidinone

A mixture of 1.9 grams 2-N-ethyl-sulfonylamino-3-(4-hydroxy-phenyl)propionate (as described above), 20 ml dimethylformamide (DMF) and NaH (1.0 equivalent), was stirred for 30 minutes at room temperature. After stirring, 1.8 grams 3-p-N-BOC-amidino-phenyl-5-methanesulfonyloxy-methyl-2-oxazolidinone (as described above) in 10 ml dimethylformamide (DMF) was added and stirred again for 15 minutes at room temperature. The reaction mixture was then diluted with ethyl acetate (0.10 M) and washed 2X with water, 1X with brine and dried over magnesium sulfate. The solvent was then removed in vacuo and the crude product was then purified by silica gel column chromatography to obtain protected form of Compound 18, 3-(4-BOC-amidinophenyl)-5-(4-(2-methoxy-carbonyl-2-N-ethyl-sulfonylaminoethyl)-phenyoxylmethyl-2-oxazolidinone which was carried onto the next step.

### 4) Deprotection of protected form of Compound 18 to form Compound 18 : 3-(4-amidinophenyl)-5-(4-(2-carboxy-2-N-ethylsulfonylaminoethyl)phenoxy)methyl-2-oxazolidinone Figure 42

Treatment of the protected form of Compound 18, 3-(4-BOC-amidinophenyl)-5-(4-(2-methoxy-carbonyl-2-N-ethylsulfonylaminoethyl)phenyoxylmethyl-2-oxazolidinone (1.0 equivalents; synthesized as described above), with 4 ml 2N NaOH for 4 hours at room temperature. The mixture was then followed with 40 ml 2N HCl-solution in dioxane added dropwise at 0C to 25C for 3 hours. The reaction mixture was then quenched with sodium bicarbonate (5 equivalents) and then diluted with ethyl acetate (0.10 M) and washed 2X with water, 1X with brine and dried over magnesium sulfate. The solvent was then removed in vacuo and the crude product was then purified by silica gel column chromatography to obtain Compound 18: 3-(4-amidinophenyl)-5-(4-(2-carboxy-2-N-ethylsulfonylaminoethyl)phenoxy)methyl-2-oxazolidinone; m.p. 212C (d).

### 14. Inhibition of Growth Factor-Induced Angiogenesis as Measured in the CAM Assay with by Intravenous Application of αᵥβ₃ Ligand Organic Mimetics

The effect on growth factor-induced angiogenesis with organic mimetics of an αᵥβ₃ ligand intravenously injected into the CAM preparation was also evaluated for use in this invention.

The 10 day old CAM preparation was used as previously described in Example 5A. Twenty-four hours after bFGF-induced angiogenesis was initiated, the organic mimetics referred to as compounds 16 (81218), 17 (87292) and 18 (87293) were separately intravenously injected into the CAM preparation in a volume of 100 ul at a concentration of 1 mg/ml (100 ug/embryo) in 20% tetraglycol-PBS at pH 7.0. In parallel assays, compounds 7 (96112), 9 (99799), 10 (96229), 12 (112854) and 14 (96113) were similarly evaluated. The effects of the organic mimetics were analyzed 48 hours later where quantification was performed by counting the number of blood vessel branch points in the area of the filter disc in a double blind approach.

The results are respectively shown in Figures 43 and 44. In Figure 43, compounds 14 (96113), 10 (96229), 9 (99799) and 12 (112854), in decreasing order of inhibition, were effective at reducing the number of branch points of new blood vessels compared to control bFGF induction and compared to compound 7 (96112). In Figure 44, compounds 17 (87292) and 18 (87293) exhibited anti-angiogenic properties as compared to untreated bFGF control and treatment with compound 16 (81218).

In a third assay, organic compounds 7 (96112), 10 (96229) and 14 (96113) were assessed as inhibitors of bFGF-induced angiogenesis along with peptides 69601 and 66203. For this assay, 250 ug/ml of organic compounds were administered 18 hours after bFGF treatment as described in Example 7B. The results are shown in Figure 28 where as above, compounds 14 (96113) and 10 (96229) almost completely inhibited the formation of new blood vessels induced by bFGF.

Thus, the aforementioned Examples demonstrate that integrin αᵥβ₃ plays a key role in angiogenesis induced by a variety of stimuli and as such αᵥβ₃ is a valuable therapeutic target with the αᵥβ₃ antagonists of this invention for diseases characterized by neovascularization.

The foregoing written specification is considered to be sufficient to enable one skilled in the art to practice the invention. The present invention is not to be limited in scope by the cell line deposited, since the deposited embodiment is intended as a single illustration of one aspect of the invention and any cell line that is functionally equivalent is within the scope of this invention. The deposit of material does not constitute an admission that the written description herein contained is inadequate to enable the practice of any aspect of the invention, including the best mode thereof, nor is it to be construed as limiting the scope of the claims to the specific illustration that it represents.

## Claims

1. An αᵥβ₃ antagonist fo use in inhibiting αᵥβ₃-mediated angiogenesis in a tissue, wherein said antagonist is an organic mimetic compound selected from
(S)-3-(4-(4-guanidinobutyloxy)phenyl-2-butylsulfonamido-propionic acid,
(S)-3-(4-(4-guanidinobutyloxy)phenyl-2-N-t-butyloxycarbonyl-propionic acid,
(R)-3-(4-(4-guanidinobutyloxy)phenyl-2-butylsulfonamido-propionic acid,
(S)-3-(4-(4-guanidinobutyloxy)phenyl-2-(10-camphorsulfonamido)-propionic acid, and
(S)-3-(4-(5-guanidinopentyloxy)phenyl-2-butylsulfonamido-propionic acid.

2. An αᵥβ₃ antagonist as claimed in claim 1, wherein said tissue is a solid tumor, and said antagonist is for inducing solid tumor tissue regression.

3. An αᵥβ₃ antagonist as claimed in claim 1, wherein said tissue is a solid tumor undergoing neovascularization, and said antagonist is for use to inhibiting the growth of said tumor tissue.

4. An αᵥβ₃ antagonist as claimed in claim 1, wherein said tissue is inflamed tissue in which neovascularization is occurring.

5. An αᵥβ₃ antagonist as claimed in claim 1, wherein said tissue is retinal tissue in which neovascularization is occurring.

6. An αᵥβ₃ antagonist as claimed in claim 1, wherein said antagonist is for use in treating restenosis in a tissue wherein smooth muscle cell migration occurs following angioplasty.

7. An αᵥβ₃ antagonist as claimed in claim 1, wherein said antagonist is for use in reducing blood supply to a tissue required to support new growth of said tissue.

8. An αᵥβ₃ antagonist as claimed in any of claims 1-7, wherein said tissue is human tissue.

9. An αᵥβ₃ antagonist as claimed in claim 1, wherein said tissue is inflamed and said angiogenesis is inflamed tissue angiogenesis.

10. An αᵥβ₃ antagonist as claimed in claim 9 wherein said tissue is arthritic.

11. An αᵥβ₃ antagonist as claimed in claim 10, wherein said arthritic tissue is present in a mammal with rheumatoid arthritis.

12. An αᵥβ₃ antagonist as claimed in claim 1, wherein said tissue is the retinal tissue and said angiogenesis is retinal angiogenesis.

13. An αᵥβ₃ antagonist as claimed in claim 12, wherein said retinal tissue is in a patient with diabetic retinopathy or macular degeneration.

14. An αᵥβ₃ antagonist as claimed in claim 1, wherein said tissue is a solid tumor or a solid tumor metastasis and said angiogenesis is tumor angiogenesis.

15. An αᵥβ₃ antagonist as claimed in claim 14, wherein said tissue is a carcinoma.

16. An αᵥβ₃ antagonist as claimed in claim 15 wherein said solid tumor is a tumor of lung, pancreas, breast, colon, larynx or ovary.

17. An αᵥβ₃ antagonist as claimed in claim 14, wherein said antagonist is to be administered in conjunction with chemotherapy.

18. An αᵥβ₃ antagonist as claimed in any of claims 1-7, wherein said antagonist is to be administered by intravenous, transdermal, intrasynovial, intramuscular, or oral route.

19. An αᵥβ₃ antagonist as claimed in any of claims 1-7, wherein said antagonist is to be administered in an amount comprising from about 0.1 mg/kg to about 300 mg/kg.

20. An αᵥβ₃ antagonist as claimed in any of claims 1-7, wherein said antagonist is to be administered in a single dose intravenously.

21. An αᵥβ₃ antagonist as claimed in any of claims 1-7, wherein said antagonist is to be administered in one or more doses daily for one or more days.

22. An αᵥβ₃ antagonist as claimed in claim 1 or claim 6 wherein said antagonist is to be administered after angioplasty.

23. An αᵥβ₃ antagonist as claimed in claim 22 wherein said angioplasty is coronary angioplasty.

24. An αᵥβ₃ antagonist as claimed in claim 1 or claim 6 wherein said antagonist is to be administered to a patient at risk for restenosis following angioplasty.

## Patentansprüche

1. αᵥβ₃-Antagonist zur Verwendung bei der Hemmung von αᵥβ₃-vermittelter Angiogenese in einem Gewebe, wobei es sich bei dem Antagonisten um ein organisches Mimetikum handelt, ausgewählt aus:
(S)-3-(4-(4-Guanidinobutyloxy)phenyl-2-butylsulfonamido-propionsäure,
(S)-3-(4-(4-Guanidinobutyloxy)phenyl-2-N-*t*-butyloxycarbonyl-propionsäure,
(R)-3-(4-(4-Guanidinobutyloxy)phenyl-2-butylsulfonamido-propionsäure,
(S)-3-(4-(4-Guanidinobutyloxy)phenyl-2-(10-camphersulfonamido)-propionsäure und
(S)-3-(4-(5-Guanidinopentyloxy)phenyl-2-butylsulfonamido-propionsäure.

2. αᵥβ₃-Antagonist wie in Anspruch 1 beansprucht, wobei es sich bei dem Gewebe um einen soliden Tumor handelt und der Antagonist zum Induzieren der Regression des soliden Tumorgewebes vorgesehen ist.

3. αᵥβ₃-Antagonist wie in Anspruch 1 beansprucht, wobei es sich bei dem Gewebe um einen soliden Tumor handelt, bei dem Gefäßneubildung stattfindet, und der Antagonist zur Verwendung bei der Hemmung des Wachstums des Tumorgewebes vorgesehen ist.

4. αᵥβ₃-Antagonist wie in Anspruch 1 beansprucht, wobei es sich bei dem Gewebe um entzündetes Gewebe handelt, bei dem Gefäßneubildung stattfindet.

5. αᵥβ₃-Antagonist wie in Anspruch 1 beansprucht, wobei es sich bei dem Gewebe um Netzhautgewebe handelt, bei dem Gefäßneubildung stattfindet.

6. αᵥβ₃-Antagonist wie in Anspruch 1 beansprucht, wobei der Antagonist zur Verwendung bei der Behandlung von Restenose in einem Gewebe vorgesehen ist, bei dem nach einer Angioplastie eine Wanderung glatter Muskelzellen stattfindet.

7. αᵥβ₃-Antagonist wie in Anspruch 1 beansprucht, wobei der Antagonist zur Verwendung bei der Senkung der Blutversorgung für ein Gewebe vorgesehen ist, was zur Unterstützung des Neuwachstums des Gewebes erforderlich ist.

8. αᵥβ₃-Antagonist wie in einem der Ansprüche 1 - 7 beansprucht, wobei es sich bei dem Gewebe um menschliches Gewebe handelt.

9. αᵥβ₃-Antagonist wie in Anspruch 1 beansprucht, wobei das Gewebe entzündet ist und es sich bei der Angiogenese um Angiogenese in einem entzündeten Gewebe handelt.

10. αᵥβ₃-Antagonist wie in Anspruch 9 beansprucht, wobei das Gewebe arthritisch ist.

11. αᵥβ₃-Antagonist wie in Anspruch 10 beansprucht, wobei das arthritische Gewebe in einem Säuger mit rheumatoider Arthritis vorliegt.

12. αᵥβ₃-Antagonist wie in Anspruch 1 beansprucht, wobei es sich bei dem Gewebe um das Netzhautgewebe handelt und es sich bei der Angiogenese um Netzhaut-Angiogenese handelt.

13. αᵥβ₃-Antagonist wie in Anspruch 12 beansprucht, wobei das Netzhautgewebe in einem Patienten mit diabetischer Retinopathie oder Makuladegeneration vorliegt.

14. αᵥβ₃-Antagonist wie in Anspruch 1 beansprucht, wobei es sich bei dem Gewebe um einen soliden Tumor oder eine solide Tumor-Metastase handelt und es sich bei der Angiogenese um eine Tumor-Angiogenese handelt.

15. αᵥβ₃-Antagonist wie in Anspruch 14 beansprucht, wobei es sich bei dem Gewebe um ein Karzinom handelt.

16. αᵥβ₃-Antagonist wie in Anspruch 15 beansprucht, wobei es sich bei dem soliden Tumor um einen Lungen-, Pankreas-, Brust-, Colon-, Kehlkopf- oder Ovar-Tumor handelt.

17. αᵥβ₃-Antagonist wie in Anspruch 14 beansprucht, wobei der Antagonist in Verbindung mit Chemotherapie verabreicht werden soll.

18. αᵥβ₃-Antagonist wie in einem der Ansprüche 1 - 7 beansprucht, wobei der Antagonist auf intravenösem, transdermalem, intrasynovialem, intramuskulärem oder oralem Weg verabreicht werden soll.

19. αᵥβ₃-Antagonist wie in einem der Ansprüche 1 - 7 beansprucht, wobei der Antagonist in einer Menge, umfassend von etwa 0,1 mg/kg bis etwa 300 mg/kg, verabreicht werden soll.

20. αᵥβ₃-Antagonist wie in einem der Ansprüche 1 - 7 beansprucht, wobei der Antagonist in einer Einzeldosis intravenös verabreicht werden soll.

21. αᵥβ₃₃-Antagonist wie in einem der Ansprüche 1 - 7 beansprucht, wobei der Antagonist in einer oder mehreren Dosen täglich über einen oder mehrere Tage hinweg verabreicht werden soll.

22. αᵥβ₃-Antagonist wie in Anspruch 1 oder Anspruch 6 beansprucht, wobei der Antagonist nach einer Angioplastie verabreicht werden soll.

23. αᵥβ₃-Antagonist wie in Anspruch 22 beansprucht, wobei es sich bei der Angioplastie um eine Koronarangioplastie handelt.

24. αᵥβ₃-Antagonist wie in Anspruch 1 oder Anspruch 6 beansprucht, wobei der Antagonist einem Patienten mit Risiko der Restenose nach Angioplastie verabreicht werden soll.

## Revendications

1. Antagoniste de αᵥβ₃ à utiliser dans l'inhibition d'une angiogenèse véhiculée par αᵥβ₃ dans un tissu, où ledit antagoniste est un composé mimétique organique choisi parmi
l'acide (S)-3-(4-(4-guanidinobutyloxy)phényl-2-butylsulfonamido-propionique,
l'acide (S)-3-(4-(4-guanidinobutyloxy)phényl-2-N-t-butyloxycarbonyl-propionique,
l'acide (R)-3-(4-(4-guanidinobutyloxy)phényl-2-butylsulfonamido-propionique,
l'acide (S)-3-(4-(4-guanidinobutyloxy)phényl-2-(10-camphresulfonamido)-propionique, et
l'acide (S)-3-(4-(5-guanidinopentyloxy)phényl-2-butylsulfonamido-propionique.

2. Antagoniste de αᵥβ₃ selon la revendication 1, dans lequel ledit tissu est une tumeur solide et ledit antagoniste est destiné à induire une régression du tissu de tumeur solide.

3. Antagoniste de αᵥβ₃ selon la revendication 1, dans lequel ledit tissu est une tumeur solide subissant une néovascularisation, et ledit antagoniste est destiné à être utilisé dans l'inhibition de la croissance dudit tissu de tumeur.

4. Antagoniste de αᵥβ₃ selon la revendication 1, dans lequel ledit tissu est un tissu enflammé dans lequel une néovascularisation se produit.

5. Antagoniste de αᵥβ₃ selon la revendication 1, dans lequel ledit tissu est un tissu rétinien dans lequel une néovascularisation se produit.

6. Antagoniste de αᵥβ₃ selon la revendication 1, dans lequel ledit antagoniste est destiné à une utilisant dans le traitement d'une resténose dans un tissu dans lequel une migration de cellule de muscle lisse se produit après angioplastie.

7. Antagoniste de αᵥβ₃ selon la revendication 1, dans lequel ledit antagoniste est destiné à une utilisation dans la réduction de l'apport en sang à un tissu nécessaire pour soutenir une nouvelle croissance dudit tissu.

8. Antagoniste de αᵥβ₃ selon l'une quelconque des revendications 1 à 7, dans lequel ledit tissu est un tissu humain.

9. Antagoniste de αᵥβ₃ selon la revendication 1, dans lequel ledit tissu est enflammé et ladite angiogenèse est une angiogenèse de tissu enflammé.

10. Antagoniste de αᵥβ₃ selon la revendication 9, dans lequel ledit tissu est arthritique.

11. Antagoniste de αᵥβ₃ selon la revendication 10, dans lequel ledit tissu arthritique est présent chez un mammifère atteint de polyarthrite rhumatoïde.

12. Antagoniste de αᵥβ₃ selon la revendication 1, dans lequel ledit tissu est le tissu rétinien et ladite angiogenèse est une angiogenèse rétinienne.

13. Antagoniste de αᵥβ₃ selon la revendication 12, dans lequel ledit tissu rétinien se trouve chez un patient atteint d'une rétinopathie diabétique ou d'une dégénérescence maculaire.

14. Antagoniste de αᵥβ₃ selon la revendication 1, dans lequel ledit tissu est une tumeur solide ou une métastase de tumeur solide et ladite angiogenèse est une angiogenèse de tumeur.

15. Antagoniste de αᵥβ₃ selon la revendication 14, dans lequel ledit tissu est un carcinome.

16. Antagoniste de αᵥβ₃ selon la revendication 15, dans lequel ladite tumeur solide est une tumeur du poumon, du pancréas, du sein, du côlon, du larynx ou de l'ovaire.

17. Antagoniste de αᵥβ₃ selon la revendication 14, dans lequel ledit antagoniste doit être administré conjointement avec une chimiothérapie.

18. Antagoniste de αᵥβ₃ selon l'une quelconque des revendications 1 à 7, dans lequel ledit antagoniste doit être administré par voie intraveineuse, transdermique, intrasynoviale, intramusculaire ou orale.

19. Antagoniste de αᵥβ₃ selon l'une quelconque des revendications 1 à 7, dans lequel ledit antagoniste doit être administré dans une quantité comprenant d'environ 0,1 mg/kg à environ 300 mg/kg.

20. Antagoniste de αᵥβ₃ selon l'une quelconque des revendications 1 à 7, dans lequel ledit antagoniste doit être administré en une seule dose par voie intraveineuse.

21. Antagoniste de αᵥβ₃ selon l'une quelconque des revendications 1 à 7, dans lequel ledit antagoniste doit être administré en une ou plusieurs doses quotidiennes pendant un ou plusieurs jours.

22. Antagoniste de αᵥβ₃ selon la revendication 1 ou la revendication 6, dans lequel ledit antagoniste doit être administré après angioplastie.

23. Antagoniste de αᵥβ₃ selon la revendication 22, dans lequel ladite angioplastie est une angioplastie coronaire.

24. Antagoniste de αᵥβ₃ selon la revendication 1 ou la revendication 6, dans lequel ledit antagoniste doit être administré à un patient présentant un risque de resténose après angioplastie.


### SEQUENCE LISTING

(1) GENERAL INFORMATION:
   (i) APPLICANT:
      (A) NAME: THE SCRIPPS RESEARCH INSTITUTE
      (B) STREET: 10550 North Torrey Pines Road
      (C) CITY: La Jolla
      (D) STATE: California
      (E) COUNTRY: US
      (F) ZIP: 92037
      (G) TELEPHONE: (619) 784-2937
      (H) TELEFAX: (619) 784-9399
   (ii) TITLE OF INVENTION: METHODS AND COMPOSITIONS USEFUL FOR INHIBITION OF ANGIOGENESIS
   (iii) NUMBER OF SEQUENCES: 45
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER: PCT/US97/
      (B) FILING DATE: 30-MAY-1997
   (vi) PRIOR APPLICATION DATA:
      (A) APPLICATION NUMBER: US 08/210,715
      (B) FILING DATE: 18-MAR-1994
   (vi) PRIOR APPLICATION DATA:
      (A) APPLICATION NUMBER: US 08/366,665
      (B) FILING DATE: 30-DEC-1994
(2) INFORMATION FOR SEQ ID NO:1:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 1..6
      (D) OTHER INFORMATION: /label= BOC-GRGDFV-OMe /note- "BOC signifies the N-terminal protecting group butyloxycarbonyl; OMe signifies a C-terminal methyl ester; arginine in the second position."
   (ix) FEATURE:
      (A) NAME/KEY: Peptide
      (B) LOCATION: 1..6
      (D) OTHER INFORMATION: /label- OMe /note= "OMe signifies the C-terminal protecting group methyl ester."
   (ix) FEATURE:
      (A) NAME/KEY: Peptide
      (B) LOCATION: 1..6
      (D) OTHER INFORMATION: /label= D-Arg /note= "A prefix "D" in D-Arg signifies that the arginine in position 2 is a D-amino acid."
   (xi) SEQUENCE DESCRIPTION: SEQ ID NO:1:
   Gly Arg Gly Asp Phe Val
   1               5
(2) INFORMATION FOR SEQ ID NO:2:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 1..6
      (D) OTHER INFORMATION: /label= BOC /note= "BOC signifies the N-terminal blocking group tertbutyloxycarbonyl."
   (ix) FEATURE:
      (A) NAME/KEY: Peptide
      (B) LOCATION: 1..6
      (D) OTHER INFORMATION: /label= OH /note= "OH signifies a free C-terminal carboxylic acid."
   (ix) FEATURE:
      (A) NAME/KEY: Peptide
      (B) LOCATION: 1..6
      (D) OTHER INFORMATION: /label= D-Arg /note= "A prefix "D" in D-Arg signifies that the arginine in position 2 is a D-amino acid."
   (xi) SEQUENCE DESCRIPTION: SEQ ID NO:2:
   Gly Arg Gly Asp Phe Val
   1               5
(2) INFORMATION FOR SEQ ID NO:3:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 1..6
      (D) OTHER INFORMATION: /label= H /note= "H signifies a free N-terminal amine."
   (ix) FEATURE:
      (A) NAME/KEY: Peptide
      (B) LOCATION: 1..6
      (D) OTHER INFORMATION: /label= OH /note= "OH signifies a free C-terminal carboxylic acid."
   (ix) FEATURE:
      (A) NAME/KEY: Peptide
      (B) LOCATION: 1..6
      (D) OTHER INFORMATION: /label= D-Arg /note= "A prefix "D" in D-Arg at position 2, signifies that the arginine is a D-amino acid."
   (xi) SEQUENCE DESCRIPTION: SEQ ID NO:3:
   Gly Arg Gly Asp Phe Val
   1               5
(2) INFORMATION FOR SEQ ID NO:4:
   (i) SEQUENCE CHARACTERISTICS:
      (A) LENGTH: 6 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 2
      (D) OTHER INFORMATION: /note= "Arg is a D-amino acid."
   (xi) SEQUENCE DESCRIPTION: SEQ ID NO:4:
   Gly Arg Gly Asp Phe Val
   1               5
(2) INFORMATION FOR SEQ ID NO:5:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 4
      (D) OTHER INFORMATION: /note= "Arg is a D-amino acid."
   (xi) SEQUENCE DESCRIPTION: SEQ ID NO:5:
   Arg Gly Asp Phe Val
   1 5
(2) INFORMATION FOR SEQ ID NO:6:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 4
      (D) OTHER INFORMATION: /note- "Phe is a D-amino acid."
   (xi) SEQUENCE DESCRIPTION: SEQ ID NO:6:
   Arg Ala Asp Phe Val
   1 5
(2) INFORMATION FOR SEQ ID NO:7:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 5
      (D) OTHER INFORMATION: /note= "Val is a D-amino acid."
   (xi) SEQUENCE DESCRIPTION: SEQ ID NO:7:
   Arg Gly Asp Phe Val
   1 5
(2) INFORMATION FOR SEQ ID NO:8:
   (i) SEQUENCE CHARACTERISTICS:
      (A) LENGTH: 15 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:8:
   Tyr Thr Ala Glu Cys Lys Pro Gln Val Thr Arg Gly Asp Val Phe
   1               5                   10                  15
(2) INFORMATION FOR SEQ ID NO:9:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 2
      (D) OTHER INFORMATION: /note- "Ala is a D-amino acid."
   (xi) SEQUENCE DESCRIPTION: SEQ ID NO:9:
   Arg Ala Asp Phe Val
   1               5
(2) INFORMATION FOR SEQ ID NO:10:
   (i) SEQUENCE CHARACTERISTICS:
      (A) LENGTH: 6 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 5
      (D) OTHER INFORMATION: /note= "Phe is a D-amino acid."
   (xi) SEQUENCE DESCRIPTION: SEQ ID NO:10:
   Ala Arg Gly Asp Phe Leu
   1               5
(2) INFORMATION FOR SEQ ID NO:11:
   (i) SEQUENCE CHARACTERISTICS:
      (A) LENGTH: 6 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAKE/KEY: Peptide
      (B) LOCATION: 5
      (D) OTHER INFORMATION: /note= "Phe is a D-amino acid."
   (xi) SEQUENCE DESCRIPTION: SEQ ID NO:11:
   Gly Arg Gly Asp Phe Leu
   1               5
(2) INFORMATION FOR SEQ ID NO:12:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:12:
   Thr Arg Gln Val Val Cys Asp Leu Gly Asn Pro Met
   1               5                   10
(2) INFORMATION FOR SEQ ID NO:13:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:13:
   Gly Val Val Arg Asn Asn Glu Ala Leu Ala Arg Leu Ser
   1               5                   10
(2) INFORMATION FOR SEQ ID NO:14:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:14:
   Thr Asp Val Asn Gly Asp Gly Arg His Asp Leu
   1               5                   10
(2) INFORMATION FOR SEQ ID NO:15:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 5
      (D) OTHER INFORMATION: /note= "Methylation is at the amino (NH2) terminus of the valine residue."
   (xi) SEQUENCE DESCRIPTION: SEQ ID NO:15:
   Arg Gly Asp Phe Val
   1               5
(2) INFORMATION FOR SEQ ID NO:16:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 5
      (D) OTHER INFORMATION: /note= "Methylation is at the amino (NH2) terminus of the valine residue."
   (xi) SEQUENCE DESCRIPTION: SEQ ID NO:16:
   Arg Gly Glu Phe Val
   1               5
(2) INFORMATION FOR SEQ ID NO:17:
   (i) SEQUENCE CHARACTERISTICS:
      (A) LENGTH: 222 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:17:
   Lys Gly Ile Gln Glu Leu Tyr Gly Ala Ser Pro Asp Ile Asp Leu Gly
   1               5                   10                  15
   Thr Gly Pro Thr Pro Thr Leu Gly Pro Val Thr Pro Glu Ile Cys Lys
   20 25 30
   Gln Asp Ile Val Phe Asp Gly Ile Ala Gln Ile Arg Gly Glu Ile Phe
   35 40 45
   Phe Phe Lys Asp Arg Phe Ile Trp Arg Thr Val Thr Pro Arg Asp Lys
   50 55 60
   Pro Met Gly Pro Leu Leu Val Ala Thr Phe Trp Pro Glu Leu Pro Glu
   65 70 75 80
   Lys Ile Asp Ala Val Tyr Glu Ala Pro Gln Glu Glu Lys Ala Val Phe
   85 90 95
   Phe Ala Gly Asn Glu Tyr Trp Ile Tyr Ser Ala Ser Thr Leu Glu Arg
   100 105 110
   Gly Tyr Pro Lys Pro Leu Thr Ser Leu Gly Leu Pro Pro Asp Val Gln
   115 120 125
   Arg Val Asp Ala Ala Phe Asn Trp Ser Lys Asn Lys Lys Thr Tyr Ile
   130 135 140
   Phe Ala Gly Asp Lys Phe Trp Arg Tyr Asn Glu Val Lys Lys Lys Met
   145 150 155 160
   Asp Pro Gly Phe Pro Lys Leu Ile Ala Asp Ala Trp Asn Ala Ile Pro
   165 170 175
   Asp Asn Leu Asp Ala Val Val Asp Leu Gln Gly Gly Gly His Ser Tyr
   180 185 190
   Phe Phe Lys Gly Ala Tyr Tyr Leu Lys Leu Glu Asn Gln Ser Leu Lys
   195 200 205
   Ser Val Lys Phe Gly Ser Ile Lys Ser Asp Trp Leu Gly Cys
   210 215 220
(2) INFORMATION FOR SEQ ID NO:18:
   (i) SEQUENCE CHARACTERISTICS:
      (A) LENGTH: 193 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:18:
   Ile Cys Lys Gln Asp Ile Val Phe Asp Gly Ile Ala Gln Ile Arg Gly
   1               5                   10                  15
   Glu Ile Phe Phe Phe Lys Asp Arg Phe Ile Trp Arg Thr Val Thr Pro
               20                  25                  30
   Arg Asp Lys Pro Met Gly Pro Leu Leu Val Ala Thr Phe Trp Pro Glu
           35                  40                  45
   Leu Pro Glu Lys Ile Asp Ala Val Tyr Glu Ala Pro Gln Glu Glu Lys
       50                  55                  60
   Ala Val Phe Phe Ala Gly Asn Glu Tyr Trp Ile Tyr Ser Ala Ser Thr
   65                  70                  75                  80
   Leu Glu Arg Gly Tyr Pro Lys Pro Leu Thr Ser Leu Gly Leu Pro Pro
                   85                  90                  95
   Asp Val Gln Arg Val Asp Ala Ala Phe Asn Trp Ser Lys Asn Lys Lys
               100                 105                 110
   Thr Tyr Ile Phe Ala Gly Asp Lys Phe Trp Arg Tyr Asn Glu Val Lys
           115                 120                 125
   Lys Lys Met Asp Pro Gly Phe Pro Lys Leu Ile Ala Asp Ala Trp Asn
       130                 135                 140
   Ala Ile Pro Asp Asn Leu Asp Ala Val Val Asp Leu Gln Gly Gly Gly
   145 150 155 160
   His Ser Tyr Phe Phe Lys Gly Ala Tyr Tyr Leu Lys Leu Glu Asn Gln
   165 170 175
   Ser Leu Lys Ser Val Lys Phe Gly Ser Ile Lys Ser Asp Trp Leu Gly
   180 185 190
   Cys
(2) INFORMATION FOR SEQ ID NO:19:
   (i) SEQUENCE CHARACTERISTICS:
      (A) LENGTH: 74 amino acids
      (B) TYPE: amino acid
      (D) TOPOLOGY: linear
   (ii) MOLECULE TYPE: protein
   (v) FRAGMENT TYPE: internal
   (xi) SEQUENCE DESCRIPTION: SEQ ID NO:19:
   Ile Cys Lys Gln Asp Ile Val Phe Asp Gly Ile Ala Gln Ile Arg Gly
   1 5 10 15
   Glu Ile Phe Phe Phe Lys Asp Arg Phe Ile Trp Arg Thr Val Thr Pro
   20 25 30
   Arg Asp Lys Pro Met Gly Pro Leu Leu Val Ala Thr Phe Trp Pro Glu
   35 40 45
   Leu Pro Glu Lys Ile Asp Ala Val Tyr Glu Ala Pro Gln Glu Glu Lys
   50 55 60
   Ala Val Phe Phe Ala Gly Asn Glu Tyr Trp
   65 70
(2) INFORMATION FOR SEQ ID NO:20:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (v) FRAGMENT TYPE: internal
   (xi) SEQUENCE DESCRIPTION: SEQ ID NO:20:
   Ile Cys Lys Gln Asp Ile Val Phe Asp Gly Ile Ala Gln Ile Arg Gly
   1               5                   10                  15
   Glu Ile Phe Phe Phe Lys Asp Arg Phe Ile Trp Arg Thr Val Thr Pro
               20                  25                  30
   Arg Asp Lys Pro Met Gly Pro Leu Leu Val Ala Thr Phe Trp Pro Glu
           35                  40                  45
   Leu Pro Glu Lys Ile Asp Ala Val Tyr Glu Ala Pro Gln Glu Glu Lys
       50                  55                  60
   Ala Val Phe Phe Ala Gly Asn Glu Tyr Trp Ile Tyr Ser Ala Ser Thr
   65                  70                  75                  80
   Leu Glu Arg Gly Tyr Pro Lys Pro Leu Thr Ser Leu Gly Leu Pro Pro
                   85                  90                  95
   Asp Val Gln Arg Val Asp Ala Ala Phe Asn Trp Ser
               100                 105
(2) INFORMATION FOR SEQ ID NO:21:
   (i) SEQUENCE CHARACTERISTICS:
      (A) LENGTH: 122 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:21:
   Glu Tyr Trp Ile Tyr Ser Ala Ser Thr Leu Glu Arg Gly Tyr Pro Lys
   1               5                   10                  15
   Pro Leu Thr Ser Leu Gly Leu Pro Pro Asp Val Gln Arg Val Asp Ala
               20                  25                  30
   Ala Phe Asn Trp Ser Lys Asn Lys Lys Thr Tyr Ile Phe Ala Gly Asp
           35                  40                  45
   Lys Phe Trp Arg Tyr Asn Glu Val Lys Lys Lys Met Asp Pro Gly Phe
       50                  55                  60
   Pro Lys Leu Ile Ala Asp Ala Trp Asn Ala Ile Pro Asp Asn Leu Asp
   65                  70                  75                  80
   Ala Val Val Asp Leu Gln Gly Gly Gly His Ser Tyr Phe Phe Lys Gly
                   85                  90                  95
   Ala Tyr Tyr Leu Lys Leu Glu Asn Gln Ser Leu Lys Ser Val Lys Phe
               100                 105                 110
   Gly Ser Ile Lys Ser Asp Trp Leu Gly Cys
           115                 120
(2) INFORMATION FOR SEQ ID NO:22:
   (i) SEQUENCE CHARACTERISTICS:
      (A) LENGTH: 89 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:22:
   Phe Asn Trp Ser Lys Asn Lys Lys Thr Tyr Ile Phe Ala Gly Asp Lys
   1               5                   10                  15
   Phe Trp Arg Tyr Asn Glu Val Lys Lys Lys Met Asp Pro Gly Phe Pro
               20                  25                  30
   Lys Leu Ile Ala Asp Ala Trp Asn Ala Ile Pro Asp Asn Leu Asp Ala
           35                  40                  45
   Val Val Asp Leu Gln Gly Gly Gly His Ser Tyr Phe Phe Lys Gly Ala
       50                  55                  60
   Tyr Tyr Leu Lys Leu Glu Asn Gln Ser Leu Lys Ser Val Lys Phe Gly
   65                  70                  75                  80
   Ser Ile Lys Ser Asp Trp Leu Gly Cys
                   85
(2) INFORMATION FOR SEQ ID NO:23:
   (i) SEQUENCE CHARACTERISTICS:
      (A) LENGTH: 228 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:23:
   Lys Gly Ile Gln Glu Leu Tyr Glu Val Ser Pro Asp Val Glu Pro Gly
   1               5                   10                  15
   Pro Gly Pro Gly Pro Gly Pro Gly Pro Arg Pro Thr Leu Gly Pro Val
               20                  25                  30
   Thr Pro Glu Leu Cys Lys His Asp Ile Val Phe Asp Gly Val Ala Gln
           35                  40                  45
   Ile Arg Gly Glu Ile Phe Phe Phe Lys Asp Arg Phe Met Trp Arg Thr
       50                  55                  60
   Val Asn Pro Arg Gly Lys Pro Thr Gly Pro Leu Leu Val Ala Thr Phe
   65                  70                  75                  80
   Trp Pro Asp Leu Pro Glu Lys Ile Asp Ala Val Tyr Glu Ser Pro Gln
                   85                  90                  95
   Asp Glu Lys Ala Val Phe Phe Ala Gly Asn Glu Tyr Trp Val Tyr Thr
               100                 105                 110
   Ala Ser Asn Leu Asp Arg Gly Tyr Pro Lys Lys Leu Thr Ser Leu Gly
           115                 120                 125
   Leu Pro Pro Asp Val Gln Arg Ile Asp Ala Ala Phe Asn Trp Gly Arg
       130                 135                 140
   Asn Lys Lys Thr Tyr Ile Phe Ser Gly Asp Arg Tyr Trp Lys Tyr Asn
   145                 150                 155                 160
   Glu Glu Lys Lys Lys Met Glu Leu Ala Thr Pro Lys Phe Ile Ala Asp
                   165                 170                 175
   Ser Trp Asn Gly Val Pro Asp Asn Leu Asp Ala Val Leu Gly Leu Thr
               180                 185                 190
   Asp Ser Gly Tyr Thr Tyr Phe Phe Lys Asp Gln Tyr Tyr Leu Gln Met
           195                 200                 205
   Glu Asp Lys Ser Leu Lys Ile Val Lys Ile Gly Lys Ile Ser Ser Asp
       210                 215                 220
   Trp Leu Gly Cys
   225
(2) INFORMATION FOR SEQ ID NO:24:
   (i) SEQUENCE CHARACTERISTICS:
      (A) LENGTH: 193 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:24:
   Leu Cys Lys His Asp Ile Val Phe Asp Gly Val Ala Gln Ile Arg Gly
   1 5 10 15
   Glu Ile Phe Phe Phe Lys Asp Arg Phe Met Trp Arg Thr Val Asn Pro
   20 25 30
   Arg Gly Lys Pro Thr Gly Pro Leu Leu Val Ala Thr Phe Trp Pro Asp
   35 40 45
   Leu Pro Glu Lys Ile Asp Ala Val Tyr Glu Ser Pro Gln Asp Glu Lys
   50 55 60
   Ala Val Phe Phe Ala Gly Asn Glu Tyr Trp Val Tyr Thr Ala Ser Asn
   65 70 75 80
   Leu Asp Arg Gly Tyr Pro Lys Lys Leu Thr Ser Leu Gly Leu Pro Pro
   85 90 95
   Asp Val Gln Arg Ile Asp Ala Ala Phe Asn Trp Gly Arg Asn Lys Lys
   100 105 110
   Thr Tyr Ile Phe Ser Gly Asp Arg Tyr Trp Lys Tyr Asn Glu Glu Lys
   115 120 125
   Lys Lys Met Glu Leu Ala Thr Pro Lys Phe Ile Ala Asp Ser Trp Asn
       130                 135                 140
   Gly Val Pro Asp Asn Leu Asp Ala Val Leu Gly Leu Thr Asp Ser Gly
   145                 150                 155                 160
   Tyr Thr Tyr Phe Phe Lys Asp Gln Tyr Tyr Leu Gln Met Glu Asp Lys
                   165                 170                 175
   Ser Leu Lys Ile Val Lys Ile Gly Lys Ile Ser Ser Asp Trp Leu Gly
               180                 185                 190
   Cys
(2) INFORMATION FOR SEQ ID NO:25:
   (i) SEQUENCE CHARACTERISTICS:
      (A) LENGTH: 74 amino acids
      (B) TYPE: amino acid
      (D) TOPOLOGY: linear
   (ii) MOLECULE TYPE: protein
   (v) FRAGMENT TYPE: internal
   (xi) SEQUENCE DESCRIPTION: SEQ ID NO:25:
   Leu Cys Lys His Asp Ile Val Phe Asp Gly Val Ala Gln Ile Arg Gly
   1               5                   10                  15
   Glu Ile Phe Phe Phe Lys Asp Arg Phe Met Trp Arg Thr Val Asn Pro
               20                  25                  30
   Arg Gly Lys Pro Thr Gly Pro Leu Leu Val Ala Thr Phe Trp Pro Asp
           35                  40                  45
   Leu Pro Glu Lys Ile Asp Ala Val Tyr Glu Ser Pro Gln Asp Glu Lys
       50                  55                  60
   Ala Val Phe Phe Ala Gly Asn Glu Tyr Trp
   65                  70
(2) INFORMATION FOR SEQ ID NO:26:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (v) FRAGMENT TYPE: internal
   (xi) SEQUENCE DESCRIPTION: SEQ ID NO:26:
   Leu Cys Lys His Asp Ile Val Phe Asp Gly Val Ala Gln Ile Arg Gly
   1               5                   10                  15
   Glu Ile Phe Phe Phe Lys Asp Arg Phe Met Trp Arg Thr Val Asn Pro
               20                  25                  30
   Arg Gly Lys Pro Thr Gly Pro Leu Leu Val Ala Thr Phe Trp Pro Asp
           35                  40                  45
   Leu Pro Glu Lys Ile Asp Ala Val Tyr Glu Ser Pro Gln Asp Glu Lys
       50                  55                  60
   Ala Val Phe Phe Ala Gly Asn Glu Tyr Trp Val Tyr Thr Ala Ser Asn
   65                  70                  75                  80
   Leu Asp Arg Gly Tyr Pro Lys Lys Leu Thr Ser Leu Gly Leu Pro Pro
                   85                  90                  95
   Asp Val Gln Arg Ile Asp Ala Ala Phe Asn Trp Gly
               100                 105
   (i) SEQUENCE CHARACTERISTICS:
      (A) LENGTH: 122 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:27:
(2) INFORMATION FOR SEQ ID NO:28:
   (i) SEQUENCE CHARACTERISTICS:
      (A) LENGTH: 89 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:28:
   Phe Asn Trp Gly Arg Asn Lys Lys Thr Tyr Ile Phe Ser Gly Asp Arg
   1 5 10 15
   Tyr Trp Lys Tyr Asn Glu Glu Lys Lys Lys Met Glu Leu Ala Thr Pro
   20 25 30
   Lys Phe Ile Ala Asp Ser Trp Asn Gly Val Pro Asp Asn Leu Asp Ala
   35 40 45
   Val Leu Gly Leu Thr Asp Ser Gly Tyr Thr Tyr Phe Phe Lys Asp Gln
   50 55 60
   Tyr Tyr Leu Gln Met Glu Asp Lys Ser Leu Lys Ile Val Lys Ile Gly
   65 70 75 80
   Lys Ile Ser Ser Asp Trp Leu Gly Cys
   85
(2) INFORMATION FOR SEQ ID NO:29:
   (i) SEQUENCE CHARACTERISTICS:
      (A) LENGTH: 2123 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 132..2123
   (xi) SEQUENCE DESCRIPTION: SEQ ID NO:29:
(2) INFORMATION FOR SEQ ID NO:30:
   (i) SEQUENCE CHARACTERISTICS:
      (A) LENGTH: 663 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Met Lys Thr His Ser Val Phe Gly Phe Phe Phe Lys Val Leu Leu Ile
   1 5 10 15
   Gln Val Tyr Leu Phe Asn Lys Thr Leu Ala Ala Pro Ser Pro Ile Ile
   20 25 30
   Lys Phe Pro Gly Asp Ser Thr Pro Lys Thr Asp Lys Glu Leu Ala Val
   35 40 45
   Gln Tyr Leu Asn Lys Tyr Tyr Gly Cys Pro Lys Asp Asn Cys Asn Leu
   50 55 60
   Phe Val Leu Lys Asp Thr Leu Lys Lys Met Gln Lys Phe Phe Gly Leu
   65 70 75 80
   Pro Glu Thr Gly Asp Leu Asp Gln Asn Thr Ile Glu Thr Met Lys Lys
   85 90 95
   Pro Arg Cys Gly Asn Pro Asp Val Ala Asn Tyr Asn Phe Phe Pro Arg
   100 105 110
   Lys Pro Lys Trp Glu Lys Asn His Ile Thr Tyr Arg Ile Ile Gly Tyr
   115 120 125
   Thr Pro Asp Leu Asp Pro Glu Thr Val Asp Asp Ala Phe Ala Arg Ala
   130 135 140
   Phe Lys Val Trp Ser Asp Val Thr Pro Leu Arg Phe Asn Arg Ile Asn
   145 150 155 160
   Asp Gly Glu Ala Asp Ile Met Ile Asn Phe Gly Arg Trp Glu His Gly
   165 170 175
   Asp Gly Tyr Pro Phe Asp Gly Lys Asp Gly Leu Leu Ala His Ala Phe
   180 185 190
   Ala Pro Gly Pro Gly Ile Gly Gly Asp Ser His Phe Asp Asp Asp Glu
   195 200 205
   Leu Trp Thr Leu Gly Glu Gly Gln Val Val Arg Val Lys Tyr Gly Asn
   210 215 220
   Ala Asp Gly Glu Tyr Cys Lys Phe Pro Phe Trp Phe Asn Gly Lys Glu
   225 230 235 240
   Tyr Asn Ser Cys Thr Asp Ala Gly Arg Asn Asp Gly Phe Leu Trp Cys
   245 250 255
   Ser Thr Thr Lys Asp Phe Asp Ala Asp Gly Lys Tyr Gly Phe Cys Pro
   260 265 270
   His Glu Ser Leu Phe Thr Met Gly Gly Asn Gly Asp Gly Gln Pro Cys
   275 280 285
   Lys Phe Pro Phe Lys Phe Gln Gly Gln Ser Tyr Asp Gln Cys Thr Thr
   290 295 300
   Glu Gly Arg Thr Asp Gly Tyr Arg Trp Cys Gly Thr Thr Glu Asp Tyr
   305 310 315 320
   Asp Arg Asp Lys Lys Tyr Gly Phe Cys Pro Glu Thr Ala Met Ser Thr
   325 330 335
   Val Gly Gly Asn Ser Glu Gly Ala Pro Cys Val Phe Pro Phe Ile Phe
   340 345 350
   Leu Gly Asn Lys Tyr Asp Ser Cys Thr Ser Ala Gly Arg Asn Asp Gly
   355 360 365
   Lys Leu Trp Cys Ala Ser Thr Ser Ser Tyr Asp Asp Asp Arg Lys Trp
   370 375 380
   Gly Phe Cys Pro Asp Gln Gly Tyr Ser Leu Phe Leu Val Ala Ala His
   385 390 395 400
   Glu Phe Gly His Ala Met Gly Leu Glu His Ser Glu Asp Pro Gly Ala
   405 410 415
   Leu Met Ala Pro Ile Tyr Thr Tyr Thr Lys Asn Phe Arg Leu Ser Gln
   420 425 430
   Asp Asp Ile Lys Gly Ile Gln Glu Leu Tyr Glu Val Ser Pro Asp Val
   435 440 445
   Glu Pro Gly Pro Gly Pro Gly Pro Gly Pro Gly Pro Arg Pro Thr Leu
   450 455 460
   Gly Pro Val Thr Pro Glu Leu Cys Lys His Asp Ile Val Phe Asp Gly
   465 470 475 480
   Val Ala Gln Ile Arg Gly Glu Ile Phe Phe Phe Lys Asp Arg Phe Met
   485 490 495
   Trp Arg Thr Val Asn Pro Arg Gly Lys Pro Thr Gly Pro Leu Leu Val
   500 505 510
   Ala Thr Phe Trp Pro Asp Leu Pro Glu Lys Ile Asp Ala Val Tyr Glu
           515                 520                 525
   Ser Pro Gln Asp Glu Lys Ala Val Phe Phe Ala Gly Asn Glu Tyr Trp
       530                 535                 540
   Val Tyr Thr Ala Ser Asn Leu Asp Arg Gly Tyr Pro Lys Lys Leu Thr
   545                 550                 555                 560
   Ser Leu Gly Leu Pro Pro Asp Val Gln Arg Ile Asp Ala Ala Phe Asn
                   565                 570                 575
   Trp Gly Arg Asn Lys Lys Thr Tyr Ile Phe Ser Gly Asp Arg Tyr Trp
               580                 585                 590
   Lys Tyr Asn Glu Glu Lys Lys Lys Met Glu Leu Ala Thr Pro Lys Phe
           595                 600                 605
   Ile Ala Asp Ser Trp Asn Gly Val Pro Asp Asn Leu Asp Ala Val Leu
       610                 615                 620
   Gly Leu Thr Asp Ser Gly Tyr Thr Tyr Phe Phe Lys Asp Gln Tyr Tyr
   625                 630                 635                 640
   Leu Gln Met Glu Asp Lys Ser Leu Lys Ile Val Lys Ile Gly Lys Ile
                   645                 650                 655
   Ser Ser Asp Trp Leu Gly Cys
               660
(2) INFORMATION FOR SEQ ID NO:31:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:31:
      ATTGAATTCT TCTACAGTTC A 21
(2) INFORMATION FOR SEQ ID NO:32:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:32:
      ATGGGATCCA CTGCAAATTT C 21
(2) INFORMATION FOR SEQ ID NO:33:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:33:
      GCCGGATCCA TGACCAGTGT A 21
(2) INFORMATION FOR SEQ ID NO:34:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:34:
      GTGGGATCCC TGAAGACTAT G 21
(2) INFORMATION FOR SEQ ID NO:35:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:35:
      AGGGGATCCT TAAGGGGATT C 21
(2) INFORMATION FOR SEQ ID NO:36:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:36:
      CTCGGATCCT CTGCAAGCAC G 21
(2) INFORMATION FOR SEQ ID NO:37:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:37:
      CTCGGATCCT CTGCAAGCAC G 21
(2) INFORMATION FOR SEQ ID NO:38:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:38:
      GCAGGATCCG AGTGCTGGGT TTATAC 26
(2) INFORMATION FOR SEQ ID NO:39:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:39:
      GCAGAATTCA ACTGTGGCAG AAACAAG 27
(2) INFORMATION FOR SEQ ID NO:40:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:40:
      GTAGAATTCC AGCACTCATT TCCTGC 26
(2) INFORMATION FOR SEQ ID NO:41:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:41:
      TCTGAATTCT GCCACAGTTG AAGG 24
(2) INFORMATION FOR SEQ ID NO:42:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:42:
      ATTGAATTCT TCTACAGTTC A 21
(2) INFORMATION FOR SEQ ID NO:43:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:43:
      GATGAATTCT ACTGCAAGTT 20
(2) INFORMATION FOR SEQ ID NO:44:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:44:
      CACTGAATTC ATCTGCAAAC A 21
(2) INFORMATION FOR SEQ ID NO:45:
   (i) SEQUENCE CHARACTERISTICS:
      (A) LENGTH: 429 amino acids
      (B) TYPE: amino acid
      (D) TOPOLOGY: linear
   (ii) MOLECULE TYPE: protein
   (v) FRAGMENT TYPE: C-terminal
   (xi) SEQUENCE DESCRIPTION: SEQ ID NO:45:
   Tyr Cys Lys Phe Pro Phe Leu Phe Asn Gly Lys Glu Tyr Asn Ser Cys
   1               5                   10                  15
   Thr Asp Thr Gly Arg Ser Asp Gly Phe Leu Trp Cys Ser Thr Thr Tyr
               20                  25                  30
   Asn Phe Glu Lys Asp Gly Lys Tyr Gly Phe Cys Pro His Glu Ala Leu
           35                  40                  45
   Phe Thr Met Gly Gly Asn Ala Glu Gly Gln Pro Cys Lys Phe Pro Phe
       50                  55                  60
   Arg Phe Gln Gly Thr Ser Tyr Asp Ser Cys Thr Thr Glu Gly Arg Thr
   65                  70                  75                  80
   Asp Gly Tyr Arg Trp Cys Gly Thr Thr Glu Asp Tyr Asp Arg Asp Lys
                   85                  90                  95
   Lys Tyr Gly Phe Cys Pro Glu Thr Ala Met Ser Thr Val Gly Gly Asn
               100                 105                 110
   Ser Glu Gly Ala Pro Cys Val Phe Pro Phe Thr Phe Leu Gly Asn Lys
           115                 120                 125
   Tyr Glu Ser Cys Thr Ser Ala Gly Arg Ser Asp Gly Lys Met Trp Cys
       130                 135                 140
   Ala Thr Thr Ala Asn Tyr Asp Asp Asp Arg Lys Trp Gly Phe Cys Pro
   145                 150                 155                 160
   Asp Gln Gly Tyr Ser Leu Phe Leu Val Ala Ala His Glu Phe Gly His
                   165                 170                 175
   Ala Met Gly Leu Glu His Ser Gln Asp Pro Gly Ala Leu Met Ala Pro
               180                 185                 190
   Ile Tyr Thr Tyr Thr Lys Asn Phe Arg Leu Ser Gln Asp Asp Ile Lys
           195                 200                 205
   Gly Ile Gln Glu Leu Tyr Gly Ala Ser Pro Asp Ile Asp Leu Gly Thr
       210                 215                 220
   Gly Pro Thr Pro Thr Leu Gly Pro Val Thr Pro Glu Ile Cys Lys Gln
   225                 230                 235                 240
   Asp Ile Val Phe Asp Gly Ile Ala Gln Ile Arg Gly Glu Ile Phe Phe
                   245                 250                 255
   Phe Lys Asp Arg Phe Ile Trp Arg Thr Val Thr Pro Arg Asp Lys Pro
               260                 265                 270
   Met Gly Pro Leu Leu Val Ala Thr Phe Trp Pro Glu Leu Pro Glu Lys
   275 280 285
   Ile Asp Ala Val Tyr Glu Ala Pro Gln Glu Glu Lys Ala Val Phe Phe
   290 295 300
   Ala Gly Asn Glu Tyr Trp Ile Tyr Ser Ala Ser Thr Leu Glu Arg Gly
   305 310 315 320
   Tyr Pro Lys Pro Leu Thr Ser Leu Gly Leu Pro Pro Asp Val Gln Arg
   325 330 335
   Val Asp Ala Ala Phe Asn Trp Ser Lys Asn Lys Lys Thr Tyr Ile Phe
   340 345 350
   Ala Gly Asp Lys Phe Trp Arg Tyr Asn Glu Val Lys Lys Lys Met Asp
   355 360 365
   Pro Gly Phe Pro Lys Leu Ile Ala Asp Ala Trp Asn Ala Ile Pro Asp
   370 375 380
   Asn Leu Asp Ala Val Val Asp Leu Gln Gly Gly Gly His Ser Tyr Phe
   385 390 395 400
   Phe Lys Gly Ala Tyr Tyr Leu Lys Leu Glu Asn Gln Ser Leu Lys Ser
   405 410 415
   Val Lys Phe Gly Ser Ile Lys Ser Asp Trp Leu Gly Cys
   420 425